(19) Europäisches Patentamt
European Patent Office
Office européen des brevets

(11) Publication number: 0 234 944 B1

# (12) EUROPEAN PATENT SPECIFICATION

(45) Date of publication of patent specification: 10.04.91 Bulletin 91/15

(51) Int. Cl.[5]: C07C 243/38, C07D 307/54, A01N 37/28

(21) Application number: 87301730.5

(22) Date of filing: 27.02.87

(54) Insecticidal N'-substituted-N-acyl-N'-alkylcarbonylhydrazines.

The file contains technical information submitted after the application was filed and not included in this specification

(30) Priority: 28.02.86 US 835073
04.02.87 US 5824

(43) Date of publication of application: 02.09.87 Bulletin 87/36

(45) Publication of the grant of the patent: 10.04.91 Bulletin 91/15

(84) Designated Contracting States: AT BE CH DE ES FR GB GR IT LI LU NL SE

(56) References cited:
AT-B- 205 488
DD-A- 212 889
FR-A- 2 120 185
GB-A- 1 573 668
US-A- 3 773 830

(73) Proprietor: ROHM AND HAAS COMPANY
Independence Mall West
Philadelphia Pennsylvania 19105 (US)

(72) Inventor: Chi-Tung-Hsu, Adam
1686 Heebner Way
Lansdale Pennsylvania 19446 (US)
Inventor: Murphy, Raymond August
Forge Gate Apartments Apt. 38-B-1
Lansdale Pennsylvania 19446 (US)

(74) Representative: Angell, David Whilton et al
ROHM AND HAAS (UK) LTD. European Operations Patent Department Lennig House
2 Mason's Avenue
Croydon CR9 3NB (GB)

EP 0 234 944 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with certain N'-substituted-N-acyl-N'-alkylcarbonylhydrazines which are novel compounds useful as insecticides, with compositions containing those compounds and their use as insecticides.

The search for compounds which have a combination of excellent insecticidal activity and low undesirable toxicity continues because of factors such as the desire for compounds of high activity, good selectivity, low undesirable environmental impact, low production cost and effectiveness against insects resistant to many known insecticides.

We have now found certain compounds which are particularly suitable for controlling plant-destructive insects in crops of cultivated plants, ornamentals and forestry.

Certain hydrazine and/or hydrazide derivatives have been disclosed in the following literature which however except in one case as mentioned discloses no biological activity for any of the compounds.

In 25 Aust. J. Chem., 523-529 (1972), several N,N'-dibenzoylhydrazine derivatives are disclosed in which one or both nitrogen atoms are alkylated or phenylated.

In 61 Helv. Chim. Acta, 1477-1510 (1978), several N,N'-dibenzoylhydrazine and hydrazide derivatives are disclosed.

In 44 J.A.C.S., 2556-2567 (1922), isopropyl hydrazine $(CH_3)_2CH\text{-}NH\text{-}NH_2$, symmetrical diisopropyl hydrazine, dibenzoylisopropyl hydrazine and certain derivatives are disclosed.

In 44 J.A.C.S., 1557-1564 (1972), isopropyl, menthyl and bornyl semicarbazides are disclosed.

In 48 J.A.C.S., 1030-1035 (1926), symmetrical di-methylphenylmethyl hydrazine and certain related compounds including 1,2-bis-methylphenylmethyl-4-phenyl-semicarbazide are disclosed.

In 27 Bull. Chem. Soc. Japan, 624-627 (1954), certain hydrazine derivatives including alpha, beta-dibenzoylphenyl hydrazine are disclosed.

In J. Chem. Soc. (C), 1531-1536 (1966), N-N'-dibenzoylphenyl hydrazine and N-acetyl-N'-benzoyl-p-nitrophenyl hydrazine are disclosed.

In 56B Chem. Berichte, 954-962 (1923), symmetrical di-isopropyl hydrazines, symmetrical diisobutyl and certain derivatives including N,N'-diisobutyldibenzoyl hydrazine are disclosed.

In 590 Annalen der Chemie, 1-36 (1954), certain N-N'-dibenzoyl hydrazine derivatives are disclosed including N'-methyl- and N'-(2-phenyl)-isopropyl-N-N'-dibenzoyl hydrazine.

In J. Chem. Soc., 4191-4198 (1952), N, N'-di-n-propyl hydrazine and the bis-3,5-dinitrobenzoyl derivatives are disclosed.

In 32 Zhur. Obs. Khim., 2806-2809 (1962), N'-2,4,-methyl-2,4-pentadiene-N,N'-dibenzoyl hydrazine is disclosed.

In 17 Acta. Chim. Scand., 95-102 (1963), 2-benzoyl-thiobenzhydrazide ($C_6H_5\text{-}CS\text{-}NHNH\text{-}CO\text{-}C_6H_5$) and certain hydrazone and hydrazine derivatives are disclosed including 1,2-dibenzoyl-benzyl hydrazine.

In 25 Zhur. Obs. Khim., 1719-1723 (1955), N-N'-bis-cyclohexyl hydrazine and N,N'-dibenzoylcyclohexyl hydrazine are disclosed.

In J. Chem. Soc., 4793-4800 (1964), certain dibenzoyl hydrazine derivatives are disclosed including tribenzoyl hydrazine and N,N'-dibenzoylcyclohexyl hydrazine.

In 36 J. Prakt. Chem., 197-201 (1967), certain dibenzoyl hydrazine derivatives including N'-ethyl-; N'-n-propyl-; N'-isobutyl-; N'-neopentyl-; N'-n-heptyl-; and N'-cyclohexylmethyl-N,N'-dibenzoyl hydrazines are disclosed.

In 26 J.O.C., 4336-4340 (1961) N'-t-butyl-N,N'-di-(t-butoxycarbonyl) hydrazide is disclosed.

In 41 J.O.C., 3763-3765 (1976), N'-t-butyl-N-(phenylmethoxycarbonyl)-N'-(chlorocarbonyl)hydrazide is disclosed.

In 94 J.A.C.S., 7406-7416 (1972) N'-t-butyl-N,N'-dimethoxycarbonyl hydrazide is disclosed.

In 43 J.O.C., 808-815 (1978), N'-t-butyl-N-ethoxycarbonyl-N'-phenylaminocarbonyl-hydrazide and N'-t-butyl-N-ethoxycarbonyl-N'-methylaminocarbonyl hydrazide are disclosed.

The N'-substituted-N-acyl-N'-alkylcarbonyl hydrazines of the present invention differ from known compounds primarily by their N- and N'-substituents.

In 39 J. Econ. Ent., 416-417 (1946), certain N-phenyl-N'-acylhydrazines are disclosed and evaluated for their activity against codling moth larvae.

Compounds of the present invention are also distinguished by their excellent insecticidal activity against insects of the orders Lepidoptera and Coleoptera.

The compounds of the invention are those having the formula :

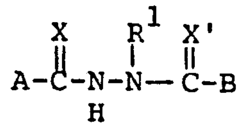

I

wherein

X and X' are the same or different O, S or NR ;

$R^1$ is unsubstituted branched ($C_3$-$C_{10}$) alkyl or straight chain ($C_1$-$C_4$) alkyl substituted with one or two of the same or different ($C_3$-$C_6$) cycloalkyl ;

A is unsubstituted or substituted naphthyl where the substituents can be from one to three of the same or different halo ; cyano ;nitro ; hydroxy ; ($C_1$-$C_4$) alkoxy ; ($C_1$-$C_4$) alkyl ; carboxy ; ($C_1$-$C_4$) alkoxy-carbonyl ; ($C_1$-$C_4$) alkanoyloxy ; $NH_2$ ; NHZ ; or NZZ' ;

unsubstituted or substituted phenyl where the substituents can be from one to five of the same or different halo ; nitro ; cyano ; hydroxy ; ($C_1$-$C_6$) alkyl ; halo-($C_1$-$C_6$) alkyl ; cyano-($C_1$-$C_6$) alkyl ; ($C_1$-$C_6$) alkoxy ; halo-($C_1$-$C_6$) alkoxy ; ($C_1$-$C_6$) alkoxy-($C_1$-$C_6$)alkyl having independently the stated number of carbon atoms in each alkyl group ; ($C_1$-$C_6$)-alkoxy-($C_1$-$C_6$) alkoxy having independently the stated number of carbon atoms in each alkyl group ; $-OCO_2R$ group ; ($C_2$-$C_6$) alkenyl optionally substituted with halo, cyano, ($C_1$-$C_4$) alkyl, ($C_1$-$C_4$) alkoxy, halo-($C_1$-$C_4$)-alkoxy or ($C_1$-$C_4$) alkylthio ; carboxy ; $-RCO_2R'$ group ; –COR group ; halo-($C_1$-$C_6$)-alkyl-carbonyl; cyano($C_1$-$C_6$) alkyl-carbonyl ; nitro-($C_1$-$C_6$) alkyl-carbonyl ; ($C_1$-$C_6$)-alkoxy-carbonyl ; halo-($C_1$-$C_6$)-alkoxy-carbonyl ; –OCOR group ; –NRR' ; amino substituted with hydroxy, ($C_1$-$C_4$) alkoxy or ($C_1$-$C_4$) alkylthio groups ; phenylamino ; diphenylamino ; –CONRR' group ; -OCONRR' group ; -NRCOR' group ; $-NRCO_2R'$ group ; –N(COR)COR' group ; –OCONRCOR' group ; sulfhydryl ; halothio ; ($C_1$-$C_6$) alkylthio ; halo-($C_1$-$C_6$) alkylthio ; –SOR group ; $-SO_2R$ group ; phenylsulfonyl ; $-OSO_2R$ group ; halo-($C_1$-$C_6$) alkylsulfonyloxy ; $-SO_2NRR'$ group; –NRSOR' group ; $-NRSO_2R'$ group ; –CSR group ; $-CS_2R$ group ; –NRCSR' group ; –SCOR group ; unsubstituted or substituted phenyl having one to three of the same or different halo, cyano, nitro, hydroxy ($C_1$-$C_4$) alkyl, ($C_1$-$C_4$) alkoxy, carboxy, ($C_1$-$C_4$) alkoxy-carbonyl, ($C_1$-$C_4$)-alkanoyloxy, $NH_2$, NHZ, NZZ' ; phenoxy where the phenyl ring is unsubstituted or substituted with one to three of the same or different halo, cyano, nitro, hydroxy, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or NZZ' ; phenylthio where the phenyl ring is unsubstituted or substituted with one to three of the same or different halo, cyano, nitro, hydroxy, ($C_1$-$C_4$) alkyl, ($C_1$-$C_4$) alkoxy, carboxy, ($C_1$-$C_4$)-alkoxy-carbonyl, ($C_1$-$C_4$) alkanoyloxy, $NH_2$, NHZ or NZZ' ; or when two adjacent positions on the phenyl ring are substituted with alkoxy groups, these groups may be joined to form, together with the carbon atoms to which they are both attached, a 5 or 6 membered dioxolano or dioxano heterocyclic ring ;

where R, R' and R" are hydrogen or ($C_1$-$C_6$)-alkyl, Z and Z' are ($C_1$-$C_4$) alkyl and "amino" means NRR' ;

B is insubstituted or substituted ($C_1$-$C_{10}$) alkyl having one to four of the same or different halo, cyano, nitro, hydroxy, ($C_1$-$C_4$) alkoxy, halo-($C_1$-$C_4$) alkoxy, carboxy, ($C_1$-$C_4$)-alkoxy-carbonyl, ($C_1$-$C_4$) alkanoyloxy, phenyl, $NH_2$, NHZ or NZZ' ;

unsubstituted or substituted ($C_3$-$C_8$) cycloalkyl or ($C_3$-$C_8$) cycloalkyl ($C_1$-$C_4$) alkyl, wherein the optional substituents are one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkanoyl, ($C_1$-$C_4$) alkoxy-carbonyl, ($C_1$-$C_4$) alkanoyloxy, phenyl, $NH_2$, NHZ or NZZ' ;

unsubstituted or substituted ($C_2$-$C_8$) alkenyl or unsubstituted or substituted ($C_3$-$C_8$)-alkadienyl having as substituent group(s) a furyl, thienyl or pyridyl group, or one to four of the same of different halo, cyano, nitro, hydroxy, ($C_1$-$C_4$) alkyl, ($C_3$-$C_6$)-cycloalkyl, halo-($C_1$-$C_4$) alkyl, ($C_1$-$C_4$)-alkoxy, halo-($C_1$-$C_4$) alkoxy, carboxy, ($C_1$-$C_4$) alkoxy-carbonyl, ($C_1$-$C_4$) alkanoyloxy, $NH_2$, NHZ or NZZ' ;

unsubstituted or substituted ($C_3$-$C_8$) cycloalkenyl or unsubstituted or substituted ($C_6$-$C_8$) cycloalkadienyl, having as substituent group(s) one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, phenyl, $NH_2$, NHZ or NZZ' ;

unsubstituted or substituted ($C_2$-$C_8$) alkynyl having as optional substituent group(s) one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$)-alkoxy, carboxy, ($C_1$-$C_4$) alkoxy-carbonyl, ($C_1$-$C_4$) alkanoyloxy, phenyl, $NH_2$, NHZ or NZZ' ; or

phenalkyl having one to four carbon atoms in the alkyl group and wherein the alkyl group is unsubstituted or substituted with one to three of the same or different halo, cyano, hydroxy, ($C_1$-$C_4$) alkyl, ($C_1$-$C_4$)-alkoxy, ($C_1$-$C_4$) alkoxy-carbonyl, $NH_2$, NHZ or NZZ' ; and the phenyl ring is unsubstituted or substituted with one to three of the same or different halo, cyano, nitro, hydroxy, ($C_1$-$C_4$) alkyl, halo-($C_1$-$C_4$) alkyl, cyano-($C_1$-$C_4$) alkyl, ($C_1$-$C_4$) alkoxy, halo-($C_1$-$C_4$) alkoxy, carboxy, ($C_1$-$C_4$)-alkoxy-carbonyl, ($C_1$-$C_4$) alkanoyloxy, ($C_2$-$C_6$) alkenyl, halo-($C_2$-

$C_6$) alkenyl, ($C_2$-$C_6$) alkynyl, $NH_2$, NHZ or NZZ' ; or

phenalkenyl having two to six carbon atoms in the alkenyl group and the alkenyl group is unsubstituted or substituted with one to three of the same or different halo, cyano, hydroxy, ($C_1$-$C_4$) alkyl, halo-($C_1$-$C_4$) alkyl, ($C_1$-$C_4$) alkoxy, halo-($C_1$-$C_4$) alkoxy, ($C_1$-$C_4$)-alkoxy-carbonyl, $NH_2$, NHZ or NZZ' ; and the phenyl ring is unsubstituted or substituted with one to three of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$)-alkoxy-carbonyl, ($C_1$-$C_4$) alkanoyloxy, $NH_2$, NHZ or NZZ' ; Z and Z' being as above defined ;

and agronomically acceptable salts thereof ; excluding those compounds wherein X and X' are O, $R^1$ is t-butyl, A is unsubstituted phenyl and B is unsubstituted methyl or unsubstituted ethyl, which we have found to have little or no insecticidal activity. The invention also provides insecticidal compositions comprising the above compounds together with agronomically acceptable diluent or carrier.

The term "halo" should be understood as including chloro, fluoro, bromo and iodo. The term "alkyl" by itself or as part of another substituent, unless otherwise stated, included straight or branched chain groups such as methyl, ethyl, n-propyl, isopropyl, n-butyl, t-butyl, isobutyl and neopentyl and, where indicated, higher homologues and isomers such as n-octyl and isooctyl. The term "haloalkyl" by itself or as part of another substituent is an alkyl group of the stated number of carbon atoms having one or more halo atoms bonded thereto such as chloromethyl, 1- or 2-bromoethyl and trifluoromethyl. Analogously, "cyanoalkyl" by itself or as part of another group is an alkyl group of the stated number of carbon atoms having one or more cyano groups bonded thereto ; "haloalkoxy" by itself or as part of another group is an alkoxy group of the stated number of carbon atoms having one or more halo atoms bonded thereto, such as difluoromethoxy, trifluoromethoxy, 2-fluoroethoxy and 2,2,2-trifluoroethoxy. "Alkenyl" and "alkynyl" by themselves or as part of another substituent comprise straight and branched chain groups of the stated number of carbon atoms. "Alkadienyl" is a straight or branched chain alkenyl group comprising two carbon-carbon double bonds that can be conjugated such as 1,3-butadienyl, cumulated such as 1,2-propadienyl or isolated such as 1,4-pentadienyl.

Typical compounds within the scope of the present invention which are worthy of mention include, but are not limited to :

N'-t-butyl-N-benzoyl-N'-acetylhydrazine
N'-t-butyl-N-benzoyl-N'-propionylhydrazine
N'-t-butyl-N-benzoyl-N'-butyrylhydrazine
N'-t-butyl-N-benzoyl-N'-valerylhydrazine
N'-t-butyl-N-benzoyl-N'-hexanoylhydrazine
N'-t-butyl-N-benzoyl-N'-heptanoylhydrazine
N'-t-butyl-N-benzoyl-N'-octanoylhydrazine
N'-t-butyl-N-benzoyl-N'-nonanoylhydrazine
N'-t-butyl-N-benzoyl-N'-decanoylhydrazine
N'-t-butyl-N-benzoyl-N'-undecanoylhydrazine
N'-t-butyl-N-benzoyl-N'-cyclopropylcarbonylhydrazine
N'-t-butyl-N-benzoyl-N'-cyclobutylcarbonylhydrazine
N'-t-butyl-N-benzoyl-N'-cyclohexylcarbonylhydrazine
N'-t-butyl-N-benzoyl-N'-cycloheptylcarbonylhydrazine
N'-t-butyl-N-benzoyl-N'-cyclooctylcarbonylhydrazine
N'-t-butyl-N-benzoyl-N'-acryloylhydrazine
N'-t-butyl-N-benzoyl-N'-allylcarbonylhydrazine
N'-t-butyl-N-benzoyl-N'-(1-propenylcarbonyl)hydrazine
N'-t-butyl-N-benzoyl-N'-crotonoylhydrazine
N'-t-butyl-N-benzoyl-N'-isocrotonoylhydrazine
N'-t-butyl-N-benzoyl-N'-cyclopropenylcarbonylhydrazine
N'-t-butyl-N-benzoyl-N'-cyclobutenylcarbonylhdyrazine
N'-t-butyl-N-benzoyl-N'-cyclopentylcarbonylhydrazine
N'-t-butyl-N-benzoyl-N'-cyclohexenylcarbonylhydrazine
N'-t-butyl-N-benzoyl-N'-cycloheptenylcarbonylhydrazine
N'-t-butyl-N-benzoyl-N'-cyclooctenylcarbonylhydrazine
N'-t-butyl-N-benzoyl-N'-propioloylhydrazine
N'-t-butyl-N-benzoyl-N'-(1-butynylcarbonyl)hydrazine
N'-t-butyl-N-benzoyl-N'-(1-pentynylcarbonyl)hydrazine
N'-t-butyl-N-benzoyl-N'-(1-hexynylcarbonyl)hydrazine
N'-t-butyl-N-benzoyl-N'-(1-heptynylcarbonyl)hydrazine
N'-t-butyl-N-benzoyl-N'-(1-octynylcarbonyl)hydrazine

N'-t-butyl-N-benzoyl-N'-(2-butynylcarbonyl)hydrazine
N'-t-butyl-N-benzoyl-N'-(2-pentynylcarbonyl)hydrazine
N'-t-butyl-N-benzoyl-N'-(3-methyl-1-butynylcarbonyl)hydrazine
N'-t-butyl-N-benzoyl-N'-(2-hexynylcarbonyl)hydrazine
N'-t-butyl-N-benzoyl-N'-(3-hexynylcarbonyl)hydrazine
N'-t-butyl-N-benzoyl-N'-(3,3-dimethyl-1-butynylcarbonyl)hydrazine
N'-t-butyl-N-benzoyl-N'-(4-octynylcarbonyl)hydrazine
N'-t-butyl-N-benzoyl-N'-benzylcarbonylhydrazine
N'-t-butyl-N-benzoyl-N'-phenethylcarbonylhydrazine
N'-t-butyl-N-benzoyl-N'-(3-phenylpropylcarbonyl)hydrazine
N'-t-butyl-N-benzoyl-N'-(4-phenylbutylcarbonyl)hydrazine
N'-t-butyl-N-benzoyl-N'-(4-(4-chlorophenyl)butylcarbonyl)hydrazine
N'-t-butyl-N-(4-chlorobenzoyl)-N'-acetylhydrazine
N'-t-butyl-N-(4-chlorobenzoyl)-N'-propionylhydrazine
N'-t-butyl-N-(4-chlorobenzoyl)-N'-butyrylhydrazine
N'-t-butyl-N-(4-chlorobenzoyl)-N'-valerylhydrazine
N'-t-butyl-N-(4-chlorobenzoyl)-N'-hexanoylhydrazine
N'-t-butyl-N-(4-chlorobenzoyl)-N'-heptanoylhydrazine
N'-t-butyl-N-(4-chlorobenzoyl)-N'-octanoylhydrazine
N'-t-butyl-N-(4-chlorobenzoyl)-N'-nonanoylhydrazine
N'-t-butyl-N-(4-chlorobenzoyl)-N'-decanoylhydrazine
N'-t-butyl-N-(4-chlorobenzoyl)-N'-undecanoylhydrazine
N'-t-butyl-N-(4-chlorobenzoyl)-N'-cyclopropylcarbonylhydrazine
N'-t-butyl-N-(4-chlorobenzoyl)-N'-cyclobutylcarbonylhydrazine
N'-t-butyl-N-(4-chlorobenzoyl)-N'-cyclohexylcarbonylhydrazine
N'-t-butyl-N-(4-chlorobenzoyl)-N'-cycloheptylcarbonylhydrazine
N'-t-butyl-N-(4-chlorobenzoyl)-N'-cyclooctylcarbonylhydrazine
N'-t-butyl-N-(4-chlorobenzoyl)-N'-acryloylhydrazine
N'-t-butyl-N-(4-chlorobenzoyl)-N'-allylcarbonylhydrazine
N'-t-butyl-N-(4-chlorobenzoyl)-N'-(1-propenylcarbonyl)hydrazine
N'-t-butyl-N-(4-chlorobenzoyl)-N'-crotonoylhydrazine
N'-t-butyl-N-(4-chlorobenzoyl)-N'-isocrotonoylhydrazine
N'-t-butyl-N-(4-chlorobenzoyl)-N'-cyclopropenylcarbonylhydrazine
N'-t-butyl-N-(4-chlorobenzoyl)-N'-cyclobutenylcarbonylhydrazine
N'-t-butyl-N-(4-chlorobenzoyl)-N'-cyclopentylcarbonylhydrazine
N'-t-butyl-N-(4-chlorobenzoyl)-N'-cyclohexenylcarbonylhydrazine
N'-t-butyl-N-(4-chlorobenzoyl)-N'-cycloheptenylcarbonylhydrazine
N'-t-butyl-N-(4-chlorobenzoyl)-N'-cyclooctenylcarbonylhydrazine
N'-t-butyl-N-(4-chlorobenzoyl)-N'-pripioloylhydrazine
N'-t-butyl-N-(4-chlorobenzoyl)-N'-(1-butynylcarbonyl)hydrazine
N'-t-butyl-N-(4-chlorobenzoyl)-N'-(1-pentynylcarbonyl)hydrazine
N'-t-butyl-N-(4-chlorobenzoyl)-N'-(1-hexynylcarbonyl)hydrazine
N'-t-butyl-N-(4-chlorobenzoyl)-N'-(1-heptynylcarbonyl)hydrazine
N'-t-butyl-N-(4-chlorobenzoyl)-N'-(1-octynylcarbonyl)hydrazine
N'-t-butyl-N-(4-chlorobenzoyl)-N'-(2-butynylcarbonyl)hydrazine
N'-t-butyl-N-(4-chlorobenzoyl)-N'-(2-pentynylcarbonyl)hydrazine
N'-t-butyl-N-(4-chlorobenzoyl)-N'-(3-methyl-1-butynylcarbonyl)hydrazine
N'-t-butyl-N-(4-chlorobenzoyl)-N'-(2-hexynylcarbonyl)hydrazine
N'-t-butyl-N-(4-chlorobenzoyl)-N'-(3-hexynylcarbonyl)hydrazine
N'-t-butyl-N-(4-chlorobenzoyl)-N'-(3,3-dimethyl-1-butynylcarbonyl)hydrazine
N'-t-butyl-N-(4-chlorobenzoyl)-N'-(4-octynylcarbonyl)hydrazine
N'-t-butyl-N-(4-chlorobenzoyl)-N'-benzylcarbonylhydrazine
N'-t-butyl-N-phenethylcarbonyl-N'-(4-chlorobenzoyl)hydrazine
N'-t-butyl-N-(4-chlorobenzoyl)-N'-(3-phenylpropylcarbonyl)hydrazine
N'-t-butyl-N-(4-chlorobenzoyl)-N'-(4-phenylbutylcarbonyl)hydrazine
N'-t-butyl-N-(4-chlorobenzoyl)-N'-(4-(4-chlorophenyl)butylcarbonyl)hydrazine
N'-t-butyl-N-(3-toluoyl)-N'-acetylhydrazine
N'-t-butyl-N-(3-toluoyl)-N'-propionylhydrazine

N'-t-butyl-N-(3-toluoyl)-N'-butyrylhydrazine
N'-t-butyl-N-(3-toluoyl)-N'-valerylhydrazine
N'-t-butyl-N-(3-toluoyl)-N'-hexanoylhydrazine
N'-t-butyl-N-(3-toluoyl)-N'-heptanoylhydrazine
N'-t-butyl-N-(3-toluoyl)-N'-octanoylhydrazine
N'-t-butyl-N-(3-toluoyl)-N'-nonanoylhydrazine
N'-t-butyl-N-(3-toluoyl)-N'-decanoylhydrazine
N'-t-butyl-N-(3-toluoyl)-N'-undecanoylhydrazine
N'-t-butyl-N-(3-toluoyl)-N'-cyclopropylcarbonylhydrazine
N'-t-butyl-N-(3-toluoyl)-N'-cyclobutylcarbonylhydrazine
N'-t-butyl-N-(3-toluoyl)-N'-cyclohexylcarbonylhydrazine
N'-t-butyl-N-(3-toluoyl)-N'-cycloheptylcarbonylhydrazine
N'-t-butyl-N-(3-toluoyl)-N'-cyclooctylcarbonylhydrazine
N'-t-butyl-N-(3-toluoyl)-N'-acrylylhydrazine
N'-t-butyl-N-(3-toluoyl)-N'-allylcarbonylhydrazine
N'-t-butyl-N-(3-toluoyl)-N'-(1-propenylcarbonyl)hydrazine
N'-t-butyl-N-(3-toluoyl)-N'-crotonoylhydrazine
N'-t-butyl-N-(3-toluoyl)-N'-isocrotonoylhydrazine
N'-t-butyl-N-(3-toluoyl)-N'-cyclopropenylcarbonylhydrazine
N'-t-butyl-N-(3-toluoyl)-N'-cyclobutenylcarbonylhydrazine
N'-t-butyl-N-(3-toluoyl)-N'-cyclopentylcarbonylhydrazine
N'-t-butyl-N-(3-toluoyl)-N'-cyclohexenylcarbonylhydrazine
N'-t-butyl-N-(3-toluoyl)-N'-cycloheptenylcarbonylhydrazine
N'-t-butyl-N-(3-toluoyl)-N'-cyclooctenylcarbonylhydrazine
N'-t-butyl-N-(3-toluoyl)-N'-propioloylhydrazine
N'-t-butyl-N-(3-toluoyl)-N'-(1-butynylcarbonyl)hydrazine
N'-t-butyl-N-(3-toluoyl)-N'-(1-pentynylcarbonyl)hydrazine
N'-t-butyl-N-(3-toluoyl)-N'-(1-hexynylcarbonyl)hydrazine
N'-t-butyl-N-(3-toluoyl)-N'-(1-heptynylcarbonyl)hydrazine
N'-t-butyl-N-(3-toluoyl)-N'-(1-octynylcarbonyl)hydrazine
N'-t-butyl-N-(3-toluoyl)-N'-(2-butynylcarbonyl)hydrazine
N'-t-butyl-N-(3-toluoyl)-N'-(2-pentynylcarbonyl)hydrazine
N'-t-butyl-N-(3-toluoyl)-N'-(3-methyl-1-butynylcarbonyl)hydrazine
N'-t-butyl-N-(3-toluoyl)-N'-(2-hexynylcarbonyl)hydrazine
N'-t-butyl-N-(3-toluoyl)-N'-(3-hexynylcarbonyl)hydrazine
N'-t-butyl-N-(3-toluoyl)-N'-(3,3-dimethyl-1-butynylcarbonyl)hydrazine
N'-t-butyl-N-(3-toluoyl)-N'-(4-octynylcarbonyl)hydrazine
N'-t-butyl-N-(3-toluoyl)-N'-benzoylcarbonylhydrazine
N'-t-butyl-N-(3-toluoyl)-N'-phenethylcarbonylhydrazine
N'-t-butyl-N-(3-toluoyl)-N'-(3-phenylpropylcarbonyl)hydrazine
N'-t-butyl-N-(3-toluoyl)-N'-(4-phenylbutylcarbonyl)hydrazine
N'-t-butyl-N-(3-toluoyl)-N'-(4-(4-chlorophenyl)butylcarbonyl)hydrazine
N'-t-butyl-N-(2-methoxybenzoyl)-N'-acetylhydrazine
N'-t-butyl-N-(2-methoxybenzoyl)-N'-propionylhydrazine
N'-t-butyl-N-(2-methoxybenzoyl)-N'-butyrylhydrazine
N'-t-butyl-N-(2-methoxybenzoyl)-N'-valerylhydrazine
N'-t-butyl-N-(2-methoxybenzoyl)-N'-hexanoylhydrazine
N'-t-butyl-N-(2-methoxybenzoyl)-N'-heptanoylhydrazine
N'-t-butyl-N-(2-methoxybenzoyl)-N'-octanoylhydrazine
N'-t-butyl-N-(2-methoxybenzoyl)-N'-nonanoylhydrazine
N'-t-butyl-N-(2-methoxybenzoyl)-N'-decanoylhydrazine
N'-t-butyl-N-(2-methoxybenzoyl)-N'-undecanoylhydrazine
N'-t-butyl-N-(2-methoxybenzoyl)-N'-cyclopropylcarbonylhydrazine
N'-t-butyl-N-(2-methoxybenzoyl)-N'-cyclobutylcarbonylhydrazine
N'-t-butyl-N-(2-methoxybenzoyl)-N'-cyclohexylcarbonylhydrazine
N'-t-butyl-N-(2-methoxybenzoyl)-N'-cycloheptylcarbonylhydrazine
N'-t-butyl-N-(2-methoxybenzoyl)-N'-cyclooctylcarbonylhydrazine
N'-t-butyl-N-(2-methoxybenzoyl)-N'-acryloylhydrazine

N'-t-butyl-N-(2-methoxybenzoyl)-N'-allylcarbonylhydrazine
N'-t-butyl-N-(2-methoxybenzoyl)-N'-(1-propenylcarbonyl)hydrazine
N'-t-butyl-N-(2-methoxybenzoyl)-N'-crotonoylhydrazine
N'-t-butyl-N-(2-methoxybenzoyl)-N'-isocrotonoylhydrazine
N'-t-butyl-N-(2-methoxybenzoyl)-N'-cyclopropenylcarbonylhydrazine
N'-t-butyl-N-(2-methoxybenzoyl)-N'-cyclobutenylcarbonylhydrazine
N'-t-butyl-N-(2-methoxybenzoyl)-N'-cyclopentylcarbonylhydrazine
N'-t-butyl-N-(2-methoxybenzoyl)-N'-cyclohexenylcarbonylhydrazine
N'-t-butyl-N-(2-methoxybenzoyl)-N'-cycloheptenylcarbonylhydrazine
N'-t-butyl-N-(2-methoxybenzoyl)-N'-cyclooctenylcarbonylhydrazine
N'-t-butyl-N-(2-methoxybenzoyl)-N'-propioloylhydrazine
N'-t-butyl-N-(2-methoxybenzoyl)-N'-(1-butynylcarbonyl)hydrazine
N'-t-butyl-N-(2-methoxybenzoyl)-N'-(1-pentynylcarbonyl)hydrazine
N'-t-butyl-N-(2-methoxybenzoyl)-N'-(1-hexynylcarbonyl)hydrazine
N'-t-butyl-N-(2-methoxybenzoyl)-N'-(1-heptynylcarbonyl)hydrazine
N'-t-butyl-N-(2-methoxybenzoyl)-N'-(1-octynylcarbonyl)hydrazine
N'-t-butyl-N-(2-methoxybenzoyl)-N'-(2-butynylcarbonyl)hydrazine
N'-t-butyl-N-(2-methoxybenzoyl)-N'-(2-pentynylcarbonyl)hydrazine
N'-t-butyl-N-(2-methoxybenzoyl)-N'-(3-methyl-1-butynylcarbonyl)hydrazine
N'-t-butyl-N-(2-methoxybenzoyl)-N'-(2-hexynylcarbonyl)hydrazine
N'-t-butyl-N-(2-methoxybenzoyl)-N'-(3-hexynylcarbonyl)hydrazine
N'-t-butyl-N-(2-methoxybenzoyl)-N'-(3,3-dimethyl-1-butynylcarbonyl)hydrazine
N'-t-butyl-N-(2-methoxybenzoyl)-N'-(4-octynylcarbonyl)hydrazine
N'-t-butyl-N-(2-methoxybenzoyl)-N'-benzylcarbonylhydrazine
N'-t-butyl-N-(2-methoxybenzoyl)-N'-phenethylcarbonylhydrazine
N'-t-butyl-N-(2-methoxybenzoyl)-N'-(3-phenylpropylcarbonyl)hydrazine
N'-t-butyl-N-(2-methoxybenzoyl)-N'-(4-phenylbutylcarbonyl)hydrazine
N'-t-butyl-N-(2-methoxybenzoyl)-N'-(4-(4-chlorophenyl)butylcarbonyl)hydrazine
N'-t-butyl-N-(3-fluorobenzoyl)-N'-acetylhydrazine
N'-t-butyl-N-(3-fluorobenzoyl)-N'-propionylhydrazine
N'-t-butyl-N-(3-fluorobenzoyl)-N'-butyrylhydrazine
N'-t-butyl-N-(3-fluorobenzoyl)-N'-valerylhydrazine
N'-t-butyl-N-(3-fluorobenzoyl)-N'-hexanoylhydrazine
N'-t-butyl-N-(3-fluorobenzoyl)-N'-heptanoylhydrazine
N'-t-butyl-N-(3-fluorobenzoyl)-N'-octanoylhydrazine
N'-t-butyl-N-(3-fluorobenzoyl)-N'-nonanoylhydrazine
N'-t-butyl-N-(3-fluorobenzoyl)-N'-decanoyl(3-fluorobenzoyl)hydrazine
N'-t-butyl-N-(3-fluorobenzoyl)-N'-undecanoylhydrazine
N'-t-butyl-N-(3-fluorobenzoyl)-N'-cyclopropylcarbonylhydrazine
N'-t-butyl-N-(3-fluorobenzoyl)-N'-cyclobutylcarbonylhydrazine
N'-t-butyl-N-(3-fluorobenzoyl)-N'-cyclohexylcarbonylhydrazine
N'-t-butyl-N-(3-fluorobenzoyl)-N'-cycloheptylcarbonylhydrazine
N'-t-butyl-N-(3-fluorobenzoyl)-N'-cyclooctylcarbonylhydrazine
N'-t-butyl-N-(3-fluorobenzoyl)-N'-acryloylhydrazine
N'-t-butyl-N-(3-fluorobenzoyl)-N'-allylcarbonylhydrazine
N'-t-butyl-N-(3-fluorobenzoyl)-N'-(1-propenylarbonyl)hydrazine
N'-t-butyl-N-(3-fluorobenzoyl)-N'-crotonoylhydrazine
N'-t-butyl-N-(3-fluorobenzoyl)-N'-isocrotonoylhydrazine
N'-t-butyl-N-(3-fluorobenzoyl)-N'-cyclopropenylcarbonylhydrazine
N'-t-butyl-N-(3-fluorobenzoyl)-N'-cyclobutenylcarbonylhydrazine
N'-t-butyl-N-(3-fluorobenzoyl)-N'-cyclopentylcarbonylhydrazine
N'-t-butyl-N-(3-fluorobenzoyl)-N'-cyclohexenylcarbonylhydrazine
N'-t-butyl-N-(3-fluorobenzoyl)-N'-cycloheptenylcarbonylhydrazine
N'-t-butyl-N-(3-fluorobenzoyl)-N'-cyclooctenylcarbonylhydrazine
N'-t-butyl-N-(3-fluorobenzoyl)-N'-propioloylhydrazine
N'-t-butyl-N-(3-fluorobenzoyl)-N'-(1-butynylcarbonyl)hydrazine
N'-t-butyl-N-(3-fluorobenzoyl)-N'-(1-pentynylcarbonyl)hydrazine
N'-t-butyl-N-(3-fluorobenzoyl)-N'-(1-hexynylcarbonyl)hydrazine

N'-t-butyl-N-(3-fluorobenzoyl)-N'-(1-heptynylcarbonyl)hydrazine
N'-t-butyl-N-(3-fluorobenzoyl)-N'-(1-octynylcarbonyl)hydrazine
N'-t-butyl-N-(3-fluorobenzoyl)-N'-(2-butynylcarbonyl)hydrazine
N'-t-butyl-N-(3-fluorobenzoyl)-N'-(2-pentynylcarbonyl)hydrazine
N'-t-butyl-N-(3-fluorobenzoyl)-N'-(3-methyl-1-butynylcarbonyl)hydrazine
N'-t-butyl-N-(3-fluorobenzoyl)-N'-(2-hexynylcarbonyl)hydrazine
N'-t-butyl-N-(3-fluorobenzoyl)-N'-(3-hexynylcarbonyl)hydrazine
N'-t-butyl-N-(3-fluorobenzoyl)-N'-(3,3-dimethyl-1-butynylcarbonyl)hydrazine
N'-t-butyl-N-(3-fluorobenzoyl)-N'-(4-octynylcarbonyl)hydrazine
N'-t-butyl-N-(3-fluorobenzoyl)-N'-benzylcarbonylhydrazine
N'-t-butyl-N-(3-fluorobenzoyl)-N'-phenethylcarbonylhydrazine
N'-t-butyl-N-(3-fluorobenzoyl)-N'-(3-phenylpropylcarbonyl)hydrazine
N'-t-butyl-N-(3-fluorobenzoyl)-N'-(4-phenylbutylcarbonyl)hydrazine
N'-t-butyl-N-(3-fluorobenzoyl)-N'-(4-(4-chlorophenyl)butylcarbonyl)hydrazine
N'-t-butyl-N-benzoyl-N'-(1-carboethoxy-2-hydroxypropionyl)hydrazine
N'-t-butyl-N-(2,3-dimethylbenzoyl)-N'-(3-cyclohexenylcarbonyl)hydrazine
N'-t-butyl-N-(2,3-dimethylbenzoyl)-N'-(2-(4-chlorophenyl)-3-methylbutanoyl)hydrazine
N'-t-butyl-N-benzoyl-N'-(2-methylcyclohexadi-2,5-enylcarbonyl)hydrazine
N'-t-butyl-N-benzoyl-N'-phenylpropynoylhydrazine
N'-t-butyl-N-benzoyl-N'-(2-methylbutanoyl)hydrazine
N'-t-butyl-N-benzoyl-N'-(2-phenyl-3-methylpentanoyl)hydrazine
N'-t-butyl-N-benzoyl-N'-(3-acetyl-2,2-dimethylcyclobutyl)-methylcarbonyl)hydrazine
N'-t-butyl-N-benzoyl-N'-(2-pentenoyl)hydrazine
N'-t-butyl-N-benzoyl-N'-(4-pentenoyl)hydrazine
N'-t-butyl-N-benzoyl-N'-(4-carbomethoxybutanoyl)hydrazine
N'-t-butyl-N-benzoyl-N'-methoxyacetylhydrazine
N'-t-butyl-N-(2,3-dimethylbenzoyl)-N'-methacryloylhydrazine
N'-t-butyl-N-(4-ethylbenzoyl)-N'-methacryloylhydrazine
N'-t-butyl-N-(2,3-dimethylbenzoyl)-N'-(2-methylpropionyl)hydrazine
N'-t-butyl-N-(2,3-dimethylbenzoyl)-N'-(2,2-dimethylpropionyl)hydrazine
N'-t-butyl-N-(2,3-dimethylbenzoyl)-N'-(2-ethylacryloyl)hydrazine
N'-t-butyl-N-(2,3-dimethylbenzoyl)-N'-(2-methyl-2-pentenoyl)hydrazine

Because of their good insecticidal activity, preferred compounds include those where, independently

X and X' are O or S ; and/or

$R^1$ is unsubstituted ($C_3$-$C_8$) branched alkyl or a ($C_1$-$C_4$) straight chain alkyl substituted with one or two of the same or different ($C_3$-$C_4$) cycloalkyl ; and/or

A is unsubstituted naphthyl ; or

unsubstituted or substituted phenyl where the substituents can be from one to three of the same or different halo ; nitro ; cyano ; ($C_1$-$C_4$) alkyl ; halo-($C_1$-$C_4$) alkyl ; ($C_1$-$C_4$) cyanoalkyl ; ($C_1$-$C_4$) alkoxy ; ($C_1$-$C_4$) alkoxy-($C_1$-$C_4$) alkyl ; –COD ; carboxy ; ($C_1$-$C_4$) alkoxy-carbonyl ; ($C_1$-$C_4$) alkanoyloxy ; $NH_2$, NHZ or NZZ' ; ($C_1$-$C_4$) alkylthio ; –CSD ; –$CS_2$D ; –SCOD ; unsubstituted or substituted phenyl having one to two of the same or different halo, nitro, ($C_1$-$C_4$) alkyl, ($C_1$-$C_4$) alkoxy, carboxy, ($C_1$-$C_4$) alkoxy-carbonyl, ($C_1$-$C_4$)- alkanoyloxy, $NH_2$, NHZ or NZZ' ; phenoxy where the phenyl ring is unsubstituted or substituted with one or two of the same or different halo, nitro, ($C_1$-$C_4$) alkyl, ($C_1$-$C_4$) alkoxy, carboxy, ($C_1$-$C_4$)- alkoxy-carbonyl, ($C_1$-$C_4$) alkanoyloxy, $NH_2$, NHZ or NZZ' ; or when two adjacent positions on the phenyl ring are substituted with alkoxy groups, these groups may be joined to form, together with the carbon atoms to which they are both attached, a 5- or 6-membered dioxolano or dioxano heterocyclic ring ; and/or

B is unsubstituted or substituted ($C_1$-$C_8$) alkyl having one to three of the same or different halo, cyano, ($C_1$-$C_4$) alkoxy, phenyl, ($C_1$-$C_4$) alkoxy-carbonyl or halo-($C_1$-$C_4$) alkoxy ;

unsubstituted or substituted ($C_3$-$C_6$) cycloalkyl or ($C_3$-$C_6$)cycloalkyl ($C_1$-$C_4$) alkyl, wherein the optional substituents are one or two of the same or different halo, ($C_1$-$C_4$) alkyl, halo-($C_1$-$C_4$) alkyl, ($C_1$-$C_4$) alkoxy ($C_1$-$C_4$) alkanoyl or halo-($C_1$-$C_4$) alkoxy ;

unsubstituted or substituted ($C_2$-$C_6$) alkenyl having a furyl or one to three of the same or different ($C_1$-$C_4$) alkyl, halo-($C_1$-$C_4$) alkyl, ($C_1$-$C_4$) alkoxy or halo-($C_1$-$C_4$) alkoxy ;

unsubstituted or substituted ($C_3$-$C_6$) cycloalkenyl having one or two of the same or different halo, ($C_1$-$C_4$) alkyl, halo-($C_1$-$C_4$) alkyl, ($C_1$-$C_4$) alkoxy or halo-($C_1$-$C_4$) alkoxy ;

unsubstituted or phenyl-substituted alkenyl ; phenalkyl having one or two carbon atoms in the alkyl group and the alkyl group is unsubstituted or substituted with one or two of the same or different halo, ($C_1$-$C_4$) alkyl,

halo-($C_1$-$C_4$) alkyl, ($C_1$-$C_4$) alkoxy or halo-($C_1$-$C_4$) alkoxy and the phenyl ring is unsubstituted or substituted with one or two of the same or different halo, ($C_1$-$C_4$) alkyl, halo-($C_1$-$C_4$) alkyl, ($C_1$-$C_4$) alkoxy or halo-($C_1$-$C_4$) alkoxy; or

phenalkenyl having two or three carbon atoms in the alkenyl group and the alkenyl group is unsubstituted or substituted with halo, ($C_1$-$C_4$) alkyl, halo-($C_1$-$C_4$) alkyl, ($C_1$-$C_4$) alkoxy or halo-($C_1$-$C_4$) alkoxy and the phenyl ring is unsubstituted or substituted with one or two of the same or different halo, cyano, ($C_1$-$C_4$) alkyl, halo-($C_1$-$C_4$) alkyl, ($C_1$-$C_4$) alkoxy or halo-($C_1$-$C_4$) alkoxy ;

where D is hydrogen or ($C_1$-$C_4$) alkyl and Z and Z' are ($C_1$-$C_4$) alkyl ; and agronomically acceptable salts thereof.

More preferred insecticidal compounds of the invention, include those where, independently,

X and X' are O or S ; and/or

$R^1$ is branched ($C_3$-$C_8$) alkyl ; and/or

A is unsubstituted naphthyl ;

unsubstituted or substituted phenyl having one to three of the same or different halo ; nitro ; cyano ; ($C_1$-$C_4$) alkyl ; halo-($C_1$-$C_4$) alkyl ; cyano-($C_1$-$C_4$) alkyl ; ($C_1$-$C_4$) alkoxy ; –COD ; ($C_1$-$C_4$) alkoxy-carbonyl ; ($C_1$-$C_4$) alkanoyloxy ; or unsubstituted or substituted phenyl having one or two of the same or different halo, nitro, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or NZZ' ; where D is hydrogen or ($C_1$-$C_4$) alkyl ; and/or

B is unsubstituted or substituted ($C_1$-$C_6$) alkyl having one to three of the same or different halo, cyano, ($C_1$-$C_4$) alkoxy, phenyl, ($C_1$-$C_4$) amkoxy-carbonyl or halo-($C_1$-$C_4$) alkoxy ;

unsubstituted or substituted ($C_3$-$C_6$) cycloalkyl or ($C_3$-$C_6$) cycloalkyl ($C_1$-$C_4$) alkyl, wherein the optional substituent is halo ($C_1$-$C_4$) alkyl, ($C_1$-$C_4$) alkanoyl, ($C_1$-$C_4$) alkoxy or halo-($C_1$-$C_4$) alkoxy ;

unsubstituted or substituted ($C_2$-$C_6$) alkenyl having one or two of the same or different ($C_1$-$C_4$) alkyl, ($C_1$-$C_4$) alkoxy or halo-($C_1$-$C_4$) alkoxy ;

unsubstituted or substituted ($C_4$-$C_6$) cycloalkenyl where the substituent is halo, ($C_1$-$C_4$) alkyl, ($C_1$-$C_4$) alkoxy or halo-($C_1$-$C_4$) alkoxy ; or

unsubstituted or phenyl-substituted alkynyl ;

and agronomically acceptable salts thereof.

Because of their excellent insecticidal activity, particularly preferred compounds of the present invention for use in the insecticidal compositions and formulations of the present invention include those where, independently,

X and X' are O ; and/or

$R^1$ is branched ($C_4$-$C_7$) alkyl ; and/or

A is unsubstituted, mono-substituted 2,3-disubstituted or 2,4-disubstituted phenyl where the substituents can be the same or different halo, ($C_1$-$C_4$) alkyl, ($C_1$-$C_4$) alkoxy, or halo-($C_1$-$C_4$) alkyl ; and/or

B is unsubstituted or substituted ($C_1$-$C_6$) alkyl having one to three of the same or different halo, phenyl or cyano ;

unsubstituted ($C_4$-$C_6$) cycloalkyl ;

unsubstituted or substituted ($C_2$-$C_5$) alkenyl having as the optional substituent(s) one or two of the same or different ($C_1$-$C_4$) alkyl ;

unsubstituted ($C_4$-$C_6$) cycloalkenyl ; or

phenalkyl having one or two carbon atoms in the alkyl group and the phenyl ring is unsubstituted or substituted with one or two of the same or different halo, ($C_1$-$C_4$) alkyl, or ($C_1$-$C_4$) alkoxy ; and

agronomically acceptable salts thereof.

Because of their outstanding insecticidal activity, most preferred compounds of the present invention for use in the insecticidal compositions and formulations of the present invention include those where, independently,

X and X' are O ; and/or

$R^1$ is t-butyl, neopentyl (2,2-dimethylpropyl) or 1,2,2-trimethylpropyl ; and/or

A is unsubstituted or mono-substituted phenyl where the substituent can be chloro, fluoro, bromo, iodo, methyl, ethyl, methoxy or trifluoromethyl ;.and/or

B is unsubstituted or substituted ($C_1$-$C_4$) alkyl having one to three of the same or different phenyl, chloro, fluoro or bromo ;

unsubstituted ($C_4$-$C_6$) cycloalkyl ;

unsubstituted ($C_4$-$C_6$)cycloalkenyl ; or

phenalkyl having one to two carbon atoms in the alkyl group and the phenyl ring is unsubstituted or substituted with one or two of the same or different chloro, fluoro, bromo, iodo, methyl, ethyl, methoxy or trifluoromethyl ; and

agronomically acceptable salts thereof.

Those N'-substituted-N-acyl-N'-alkylcarbonyl hydrazines of Formula I which possess acidic or basic functional groups may be further reacted to form novel salts with appropriate bases or acids. These salts also exhibit insecticidal activity. Typical salts are the agronomically acceptable metal salts, ammonium salts and acid addition salts. Among the metal salts are those in which the metal cation is an alkali metal cation such as sodium, potassium, lithium or the like; alkaline earth metal cation such as calcium, magnesium, barium, strontium or the like ; or heavy metal cation such as zinc, manganese, cupric, cuprous, ferric, ferrous, titanium, aluminum or the like. The ammonium salts include those in which the ammonium cation has the formula $NR^5R^6R^7R^8$ wherein each of $R^5$, $R^6$, $R^7$ and $R^8$ are independently hydrogen, hydroxy, $(C_1$-$C_4)$ alkoxy, $(C_1$-$C_{20})$ alkyl, $(C_3$-$C_8)$ alkenyl, $(C_3$-$C_8)$ alkynyl, $(C_2$-$C_8)$ hydroxyalkyl, $(C_2$-$C_8)$ alkoxyalkyl, $(C_2$-$C_6)$ aminoalkyl, $(C_2$-$C_6)$ haloalkyl, $NH_2$, $(C_1$-$C_4)$ alkyl- or di$(C_1$-C4) alkylamino, substituted or unsubstituted phenyl, substituted or unsubstituted phenylalkyl, having up to four carbon atoms in the alkyl moiety, or any two of $R^5$, $R^6$, $R^7$ or $R^8$ can be taken together to form with the nitrogen atom a 5- or 6- membered heterocyclic ring, optionally having up to one additional hetero atom (e.g., oxygen, nitrogen, or sulfur) in the ring, and preferably saturated, such as piperidino, morpholino, pyrrolidino or piperazino, or any three of $R^5$, $R^6$, $R^7$ or $R^8$ can be taken together to form with the nitrogen atom a 5- or 6-membered aromatic heterocyclic ring, such as piperazole or pyridine. When $R^5$, $R^6$, $R^7$ or $R^8$ substitutent in the ammonium group is a substituted phenyl or substituted phenylalkyl, the substituents on the phenyl and phenalkyl will generally be selected from halo, $(C_1$-$C_8)$ alkyl, $(C_1$-$C_4)$ alkoxy, hydroxy, nitro, trifluoromethyl, cyano, amino and $(C_1$-$C_4)$ alkylthio. Such substituted phenyl groups preferably have up to two such substituents. Representative ammonium cations include ammonium, dimethylammonium, 2-ethylhexylammonium, bis(2-hydroxyethyl)ammonium, tris(2-hydroxyethyl)ammonium, dicyclohexylammonium, t-octylammonium, 2-hydroxyethylammonium, morpholinium, piperidinium, 2-phenethylammonium, 2-methylbenzylammonium, n-hexylammonium, triethylammonium, trimethylammonium, tri(n-butyl)ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxyethyldimethyloctadecylammonium, 2-hydroxyethyldiethyloctylammonium, decyltrimethylammonium, hexyltriethylammonium and 4-methylbenzyltrimethylammonium. Among the acid addition salts are those in which the anion is an agronomically acceptable anion such as hydrochloride, hydrobromide, sulfate, nitrate, perchlorate, acetate and oxalate.

The compounds of this invention or their precursors can be prepared according to the following processes. Process A can be used when preparing compounds according to Formula I where X and X' are both oxygen.

Process A :

Step 1

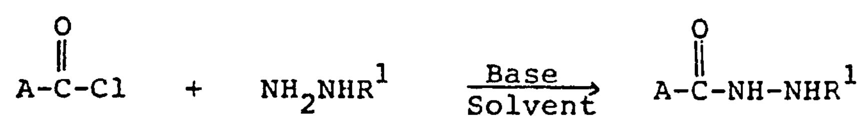


II III IV

Step 2

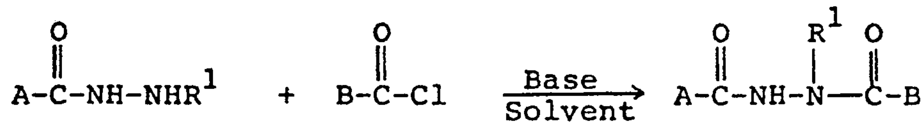


IV V I

where $R^1$, A and B are as defined above for Formula I and X and X' are oxygen.

Alternatively, process B can be used when preparing compounds according to Formula I where X and X' are oxygen, and $R^1$, A and B are as defined above for Formula I.

Process B :

Step 1

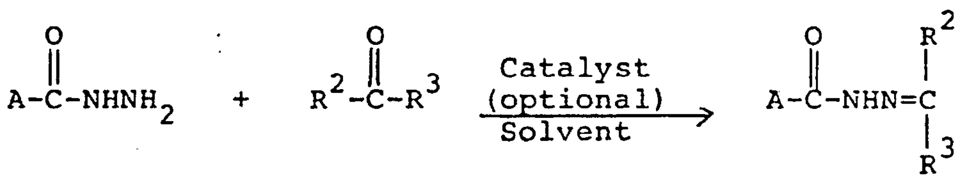


VI VII VIII

Step 2

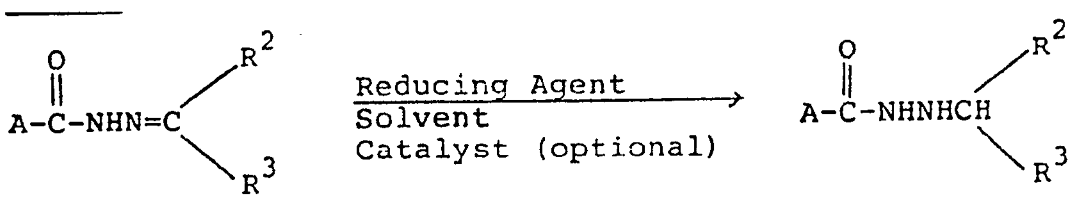


VIII IX (IV)

Step 3

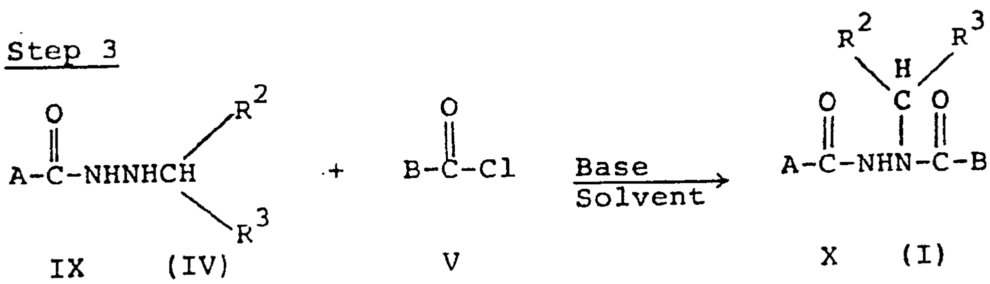


IX (IV) V X (I)

where X and X′ are oxygen, A and B are as defined above for Formula I, and $R^2$ and $R^3$ are the same or different hydrogen or ($C_2$-$C_9$) straight or branched chain alkyl provided that $R^2$ and $R^3$ are not both H or $R^2$ or $R^3$ is not a straight chain alkyl group when the other ($R^2$ or $R^3$) is hydrogen. As can be seen above, the intermediate product of Step 2, the compounds of Formula IX, corresponds to the compounds of Formula IV. In addition, the compound of Formula X corresponds to the compounds of Formula I where X and X′ are oxygen.

Process C can be used wren preparing compounds according to Formula I where A, B and $R^1$ are as defined for Formula I and one or both X and X′ are sulfur.

Process C :

Step 1

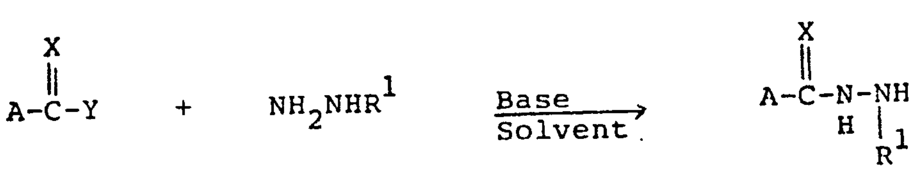


XI III XII

Step 2

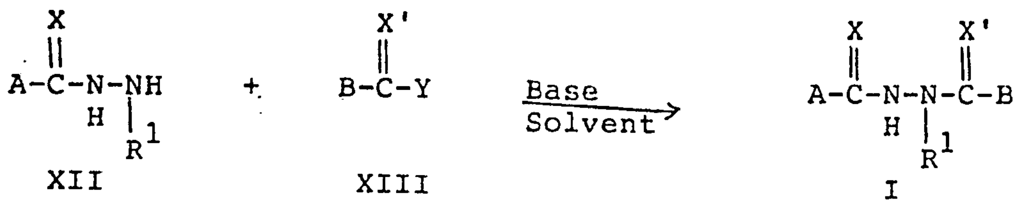


XII XIII I

wherein A, B and $R^1$ are as defined above for Formula I and one or both X and X′ are sulfur, and Y is a good leaving group such as carboxyalkylthio (for example, carboxymethylthio $-SCH_2CO_2H$) ; alkylthio (for example, methylthio) ; or halo (for example, chloro).

In process A, a compound of Formula II is reacted with a monosubstituted hydrazine of Formula III or a corresponding acid addition salt such as the hydrochloride salt or the like, in the presence of a base in an inert or substantially inert solvent or mixture of solvents to afford an intermediate product of Formula IV which can be isolated or further reacted with a compound of Formula V in the presence of a base in an inert or substantially inert solvent or mixture of solvents to afford the desired product of Formula 1.

Examples of the compounds of Formula II which can be used in the above processes include benzoyl chloride, 4-chlorobenzoyl chloride, 4-methylbenzoyl chloride, 3,5-dichlorobenzoyl chloride, 2-bromobenzoyl chloride and 3-cyanobenzoyl chloride.

Examples of the compounds of Formula V which can be used in the above processes include cyclohexylcarbonyl chloride, n-butanoyl chloride, n-pentanoyl chloride, phenylacetyl chloride, 1-cyclohexenecarbonyl chloride, pivaloyl chloride, trichloroacetyl chloride and methacryloyl chloride.

The compounds of Formula II and/or Formula V are generally commercially available or can be prepared by known procedures.

Examples of the compounds of Formula III which can be used in the above processes include isopropylhydrazine, t-butylhydrazine, neopentylhydrazine, alpha-methylneopentylhydrazine, isobutylhydrazine, isopentylhydrazine and isooctylhydrazine. The compounds of Formula III are generally commercially available or can be prepared by known procedures.

Suitable solvents for use in the above processes include water ; alcohols such as methanol, ethanol and isopropanol ; hydrocarbons such as toluene, xylene, hexane and heptane ; glyme ; tetrahydrofuran ; acetonitrile ; pyridine ; and/or haloalkanes such as methylene chloride. Mixtures of solvents can be used.

Preferred solvents are water, toluene and/or methylene chloride.

Examples of bases for use in the above processes include tertiary amines such as triethylamine ; pyridine; potassium carbonate ; sodium carbonate ; sodium bicarbonate ; sodium hydroxide ; and potassium hydroxide. Preferred bases are sodium hydroxide, potassium hydroxide and triethylamine.

In Process B, a compound of Formula VI is reacted with a ketone or aldehyde of Formula VII in an inert or substantially inert solvent or mixture of solvents and optionally in the presence of a catalyst to afford an intermediate product of Formula VIII. The intermediate product of Formula VIII is then further reacted with a reducing agent in an inert or substantially inert solvent or mixture of solvents to afford a second intermediate product of Formula IX (IV) which can be isolated or further reacted with a compound of Formula V in the presence of a base in an inert or substantially inert solvent or mixture of solvents to afford the desired product of Formula X (I).

Examples of the compounds of Formula VI which can be used in the above Process B include benzoylhydrazine, 4-chlorobenzoylhydrazine, 2-methylbenzoylhydrazine, 4-methylbenzoylhydrazine, 3,5-dichlorobenzoylhydrazine and the like. The compounds of Formula VI are generally commercially available or can be prepared by known procedures.

Examples of the compounds of Formula VII which can be used in the above Process B include 1,1,1-trimethylacetaldehyde, methylethylketone and diethylketone. The compounds of Formula VII are generally commercially available or can be prepared by known procedures.

Optionally, a catalyst may be used in Step 1 of Process B. Suitable catalysts generally include organic acids such as acetic acid, trifluoroacetic acid and oxalic acid ; mineral acids such as hydrochloric acid, sulfuric acid and nitric acid ; arylsulfonic acids such as toluenesulfonic acid ; or pyridinium toluenesulfonate. Preferred catalysts are organic acids or arylsulfonic acids. Most preferred catalysts are acetic acid and trifluoroacetic acid.

Suitable solvents for use in the above Process B, Step 1, include alcohols such as methanol, ethanol and isopropanol ; hydrocarbons such as toluene and benzene ; ethers such as tetrahydrofuran (THF) and glyme ; and dimethylformamide. Preferred solvents are alcohols and hydrocarbons. Most preferred solvents are

alcohols such as methanol and ethanol.

Examples of suitable reducing agents for use in the above Process B, Step 2, include hydrides such as sodium borohydride and derivatives thereof such as sodium cyanoborohydride, lithium aluminum hydride and derivatives thereof ; or diborane. Preferred reducing agents are sodium borohydride and derivatives thereof or lithium aluminum hydride and derivatives thereof. Most preferred as a reducing agent is sodium cyanoborohydride.

Optionally, in Process B, Step 2, a catalyst may be included. Examples of suitable catalysts include organic acids such as acetic acid, trifluoroacetic acid ; or mineral acids such as hydrochloride acid and sulfuric acid. Preferred catalysts are organic acids and hydrochloric acid. Most preferred catalysts are acetic acid, trifluoroacetic acid and hydrochloric acid.

Suitable solvents for use in the above Process B, Step 2, include alcohols such as methanol, ethanol and isopropanol ; ethers such as tetrahydrofuran (THF), diethylether and glyme ; or halohydrocarbons such as methylene chloride and chloroform. Preferred solvents are alcohols and most preferred are methanol and ethanol.

Step 3 of Process B corresponds to Step 2 of Process A. Consequently, those bases and solvents suitable for use in Step 2 of Process A are suitable for use in Step 3 of Process B including the preferred bases and solvents described above.

In Process C, a compound of Formula XI is reacted with a monosubstituted hydrazine of Formula III or a corresponding acid addition salt such as the hydrochloride salt in the presence of a base in an inert or substantially inert solvent or mixture of solvents to afford an intermediate compound of Formula XII which can be isolated or further reacted with a compound of Formula XIII in the presence of a base in an inert or substantially inert solvent or mixture of solvents to afford the desired product of Formula I.

Examples of the compounds of Formula XI which can be used in the above Process C include 3-methyl-methylthio-thiobenzoate, 4-chloromethylthio-thiobenzoate, 4-methyl-methylthio-thiobenzoate and carboxymethylthio-thiobenzoate.

Examples of the compounds of Formula XIII which can be used in the above Process C include methylthio-thiobutyrate, methylthio-thiopentanoate, methylthio-thiocyclopentanecarboxylate and methylthio-alphaphenylthioacetate.

The compounds of Formula XI and/or Formula XII are generally commercially available or can be prepared by known procedures.

Suitable solvents for use in Process C are generally polar high-boiling solvents such as dimethylformamide (DMF) ; glyme ; tetrahydrofuran (THF) ; and pyridine. The preferred solvent is pyridine.

Suitable bases for use in Process C include tertiary amines such as triethylamine ; and pyridine. The preferred base is pyridine.

The above processes A and B can be carried out at temperatures between about –20°C and about 100°C, preferably between about –5°C and about 50°C.

Process C can be carried out at temperatures between about 10°C and 200°C, preferably between about 70°C and about 100°C.

Preparation of the compounds of the present invention by processes A, B and C is generally carried out at about atmospheric pressure although higher or lower pressures can be used if desired.

Substantially equimolar amounts of reactants are preferably used in processes A, B and C although higher or lower amounts can be used if desired.

Generally, about one equivalent of base is used per equivalent of starting material of Formula II, V, XI and/or XIII. Where the acid addition salt of the monosubstituted hydrazine of Formula III is used, one additional equivalent of base is used. For example, in Process A, when substituents A and B are the same and a monosubstituted hydrazine is used, about two equivalents of base are used since about two equivalents of a suitably substituted benzoyl chloride of Formula II or V are employed. In Process A, when substituents A and B are different and an acid addition salt of the monosubstituted hydrazine of Formula III is used, about two equivalents of base are used in step 1 and about one equivalent of base is used in step 2.

Modifications to the above processes may be necessary to accommodate reactive functionalities of particular A and/or B substituents. Such modifications would be apparent to those skilled in the art.

The compounds of the invention which are agronomically acceptable salts of compounds of Formula I can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide or nitrate with a metal salt of a compound of Formula I in a suitable solvent. When metal hydroxides are used as reagents, useful solvents include water ; ethers such as glyme ; dioxane ; tetrahydrofuran ; alcohols such as methanol, ethanol and isopropanol. When metal hydrides are used as reagents, useful solvents include nonhydroxylic solvents, for example, ethers such as

dioxane, glyme and diethylether ; tetrahydrofuran ; hydrocarbons such as toluene, xylene, hexane, pentane, heptane and octane ; and dimethylformamide. When amines are used as reagents, useful solvents include alcohols, such as methanol or ethanol ; hydrocarbons, such as toluene, xylene and hexane ; tetrahydrofuran; glyme ; dioxane ; or water. When ammonium salts are used as reagents, useful solvents include water ; alcohols, such as methanol or ethanol ; glyme ; or tetrahydrofuran. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0°C to about 100°C, preferably at about room temperature.

The acid addition salts of the invention can be prepared by reacting hydrochloric, hydrobromic, sulfuric, nitric, phosphoric, acetic, propionic, benzoic or other suitable acid with a compound of Formula I having a basic functional group in a suitable solvent. Useful solvents include water, alcohols, ethers, esters, ketones and haloalkanes. The particular choice of solvent will depend on the relative solubilities of the starting materials and the resulting salts and slurries rather than solutions of certain reagents may be used to obtain the salts. Generally, equivalent molar amounts of starting materials are used and the salt-forming reaction is carried out at from about −10°C to about 100°C, preferably at about room temperature.

The following examples are given solely to further illustrate this invention. In Table I, some N′-substituted-N-acyl-N′-alkylcarbonylhdyrazines of the present invention that have been made are listed. Structures were confirmed by NMR and in some cases by IR and/or elemental analysis. Specific illustrative preparation of the compounds of Examples 1, 2, 7, 8, 10, 18 and 21 are described after Table I.

TABLE I

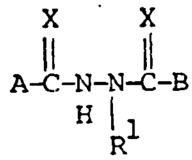

| Ex. No. | X | X' | $R^1$ | A | B | m.p. (°C) |
|---|---|---|---|---|---|---|
| 1 | O | O | $-C(CH_3)_3$ | $-C_6H_5$ | $-CH_2CH_2CH_2CH_2CH_3$ | 117-118 |
| 2 | O | O | $-C(CH_3)_3$ | $-C_6H_5$ | 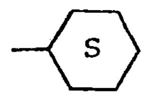 | 202-204 |
| 3 | O | O | $-C(CH_3)_3$ | $-C_6H_3Cl_2-3,5$ | $-CH_2CH_2Br$ | Solid |
| 4 | O | O | $-C(CH_3)_3$ | $-C_6H_3Cl_2-3,5$ | $-CH_2Cl$ | Solid |
| 5 | O | O | $-C(CH_3)_3$ | $-C_6H_3Cl_2-3,5$ | $-CH(CH_3)Br$ | Solid |
| 6 | O | O | $-C(CH_3)_3$ | $-C_6H_3Cl_2-3,5$ | $-CH=CH_2$ | Solid |
| 7 | O | O | $-C(CH_3)_3$ | $-C_6H_5$ | $-C(CH_3)_3$ | 217-220 |
| 8 | O | O | $-C(CH_3)_3$ | $-C_6H_5$ | $-CH_2C_6H_5$ | 167-169 |
| 9 | O | O | $-C(CH_3)_3$ | $-C_6H_3Cl_2-3,4$ | $-CH_3$ | 119-130 |
| 10 | O | O | $-C(CH_3)_3$ | $-C_6H_5$ | 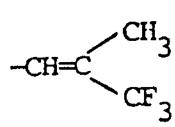 | Oil |
| 11 | O | O | $-C(CH_3)_3$ | $-C_6H_4Cl-4$ | $-C(CH_3)_3$ | 212-213 |
| 12 | O | O | $-C(CH_3)_3$ | $-C_6H_4Cl-4$ | $-CH=\underset{H}{C}C_6H_5$ | 210 |
| 13 | O | O | $-C(CH_3)_3$ | $-C_6H_4Cl-4$ | $-CH_2C_6H_5$ | 215 |

TABLE I (Cont'd)

| Ex. No. | X | X' | $R^1$ | A | B | m.p. (°C) |
|---|---|---|---|---|---|---|
| 14 | O | O | $-C(CH_3)_3$ | $-C_6H_3NO_2$-2-Cl-4 | $-CH{=}\underset{H}{C}C_6H_4CF_3$-3 | 180 |
| 15 | O | O | $-C(CH_3)_3$ | $-C_6H_3NO_2$-2-Cl-4 | $-CH{=}\underset{H}{C}C_6H_4CN$-4 | 248 |
| 16 | O | O | $-C(CH_3)_3$ | $-C_6H_3NO_2$-2-Cl-4 | $-CH{=}\underset{H}{C}C_6H_4Cl$-4 | 245 |
| 17 | O | O | $-C(CH_3)_3$ | $-C_6H_5$ | 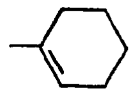 | 192-193 |
| 18 | O | O | $-C(CH_3)_3$ | $-C_6H_5$ | $-C(CH_3)=CH_2$ | 148-152 |
| 19 | O | O | $-C(CH_3)_3$ | $-C_6H_5$ | 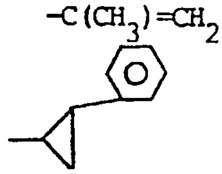 | 175-177 |
| 20 | O | O | $-C(CH_3)_3$ | $-C_6H_3Cl_2$-3,4 | 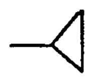 | 157-158 |
| 21 | O | O | $-C(CH_3)_3$ | $-C_6H_4Cl$-4 | 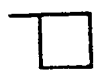 | 188-192 |
| 22 | O | O | $-C(CH_3)_3$ | $-C_6H_5$ | $-CH{=}\underset{H}{C}-$ 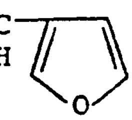 | Solid |
| 23 | O | O | $-C(CH_3)_3$ | $-C_6H_5$ | $CH_2OH$<br>$-CHCO_2CH_2CH_3$ | Low melting solid |
| 24 | O | O | $-C(CH_3)_3$ | $-C_6H_3(CH_3)_2$-2,3 | 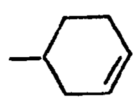 | 188 |
| 25 | O | O | $-C(CH_3)_3$ | $-C_6H_3(CH_3)_2$-2,3 | $CH(CH_3)_2$<br>$-CH$<br>$C_6H_4Cl$-4 | 160-163 |

TABLE I (Cont'd)

| Ex. No. | X | X' | $R^1$ | A | B | m.p. (°C) |
|---|---|---|---|---|---|---|
| 26 | O | O | $-C(CH_3)_3$ | $-C_6H_5$ | 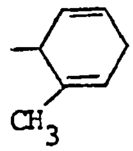 | |
| 27 | O | O | $-C(CH_3)_3$ | $-C_6H_5$ | $-C{\equiv}C-C_6H_5$ | 146-147 |
| 28 | O | O | $-C(CH_3)_3$ | $-C_6H_5$ | 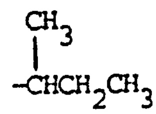 | 148-150 |
| 29 | O | O | $-C(CH_3)_3$ | $-C_6H_5$ | 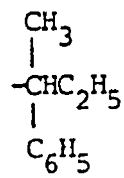 | 165-166 |
| 30 | O | O | $-C(CH_3)_3$ | $-C_6H_5$ | 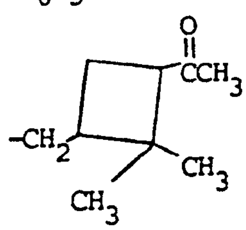 | low melting solid |
| 31 | O | O | $-C(CH_3)_3$ | $-C_6H_5$ | $-CH{=}CHCH_2CH_3$ | |
| 32 | O | O | $-C(CH_3)_3$ | $-C_6H_5$ | $-CH_2CH_2CH{=}CH_2$ | |
| 33 | O | O | $-C(CH_3)_3$ | $-C_6H_5$ | $-CH_2CH_2CH_2CO_2CH_3$ | Oil |
| 34 | O | O | $-C(CH_3)_3$ | $-C_6H_5$ | $-CH_2OCH_3$ | 117-118 |
| 35 | O | O | $-C(CH_3)_3$ | $-C_6H_3(CH_3)_2$-2,3 | 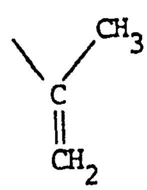 | 115-118 |
| 36 | O | O | $-C(CH_3)_3$ | $-C_6H_4CH_2CH_3$-4 | 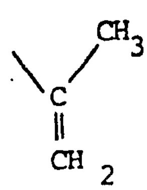 | 108-118 |

TABLE I (Cont'd)

| Ex. No. | X | X' | $R^1$ | A | B | m.p. (°C) |
|---|---|---|---|---|---|---|
| 37 | O | O | $-C(CH_3)_3$ | $-C_6H_3(CH_3)_2$-2,3 | $-CH(CH_3)_2$ | 143-144 |
| 38 | O | O | $-C(CH_3)_3$ | $-C_6H_3(CH_3)_2$-2,3 | $-C(CH_3)_3$ | 175-181 |
| 39 | O | O | $-C(CH_3)_3$ | $-C_6H_3(CH_3)_2$-2,3 | 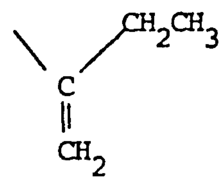 | 118-122 |
| 40 | O | O | $-C(CH_3)_3$ | $-C_6H_3(CH_3)_2$-2,3 | 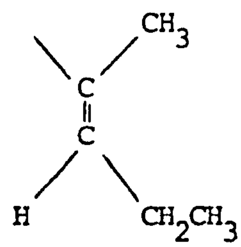 | 140-143 |

EXAMPLE NO. 1 – Preparation of N'-t-butyl-N-benzoyl-N'-n-pentanecarbonylhydrazine

To a stirred suspension of t-butylhydrazine hydrochloride (1 g, 0.008 M) in toluene (30 ml) at room temperature was added dropwise a 50% aqueous solution of sodium hydroxide (0.64 g, 0.008 M). After 15 min., the reaction mixture was cooled to 5°C and a solution of benzoyl chloride (1.12 g, 0.008 M) in toluene (5 ml) and a solution of aqueous 50% sodium hydroxide (0.64 g, 0.008 M) were added dropwise simultaneously from separate addition funnels while maintaining the temperature at or below 10°C. Following the addition, the reaction mixture was warmed to room temperature and stirred for 1 hr. The reaction was diluted with hexane and the solid N'-t-butyl-N-benzoylhydrazine (IV) was isolated by filtration.

To a stirred solution of the monobenzoylated compound (1.4 g, 0.0073 M) in toluene (30 ml) at 5°C, were added dropwise simultaneously from separate addition funnels, solutions of hexanoyl chloride (1 g, 0.0073 M) in toluene (5 ml) and aqueous 50% sodium hydroxide solution (0.58 g, 0.0073 M) while maintaining the temperature below 10°C. Following the addition, the reaction mixture was warmed to room temperature and stirred for 1 hr. The mixture was then diluted with hexane and the solid product isolated by filtration. The product was washed with water and hexane and dried. The crude product was recrystallized from ethermethanol to afford N'-t-butyl-N-benzoyl-N'-n-pentanecarbonylhydrazine as a white powder. m.p. 117-118°C.

EXAMPLE NO. 2 – Preparation of N'-t-butyl-N-benzoyl-N'-cyclohexanecarbonylhydrazine

To a stirred suspension of t-butylhydrazine hydrochloride (1 g, 0.008 M) in toluene (30 ml) at room temperature was added dropwise a 50% aqueous solution of sodium hydroxide (0.64 g, 0.008 M). After 15 min., the reaction mixture was cooled to 5°C and a solution of benzoyl chloride (1.12 g, 0.008 M) in toluene (5 ml) and a solution of aqueous 50% sodium hydroxide (0.64 g, 0.008 M) were added dropwise simultaneously from separate addition funnels while maintaining the temperature at or below 10°C. Following the addition, the reaction mixture was warmed to room temperature and stirred for 1 hr. The reaction was diluted with hexane and the solid N'-t-butyl-N-benzoylhydrazine was isolated by filtration.

To a stirred solution of the monobenzoylated compound (1.4 g, 0.0073 M) in toluene (30 ml) at 5°C, were added dropwise simultaneously from separate addition funnels, solutions of cyclohexanecarbonyl chloride (1.1 g, 0.0073 M) in toluene (5 ml) and aqueous 50% sodium hydroxide solution (0.58 g, 0.0073 M) while maintaining the temperature below 10°C. Following the addition, the reaction mixture was warmed to room temperature and

stirred for 1 hr. The mixture was then diluted with hexane and the solid product isolated by filtration. The product was washed with water and hexane and dried. The crude product was recrystallized from ether-methanol to afford N'-t-butyl-N-benzoyl-N'-cyclohexanecarbonylhydrazine as a white powder. m.p. 202-204°C.

EXAMPLE NO. 7 – Preparation of N'-t-butyl-N-benzoyl-N'-pivaloylhydrazine

To a stirred suspension of t-butylhydrazine hydrochloride (2 g, 0.016 M) in toluene (50 ml) at room temperature was added dropwise a 50% aqueous solution of sodium hydroxide (1.28 gm, 0.016 M). After 15 min., the reaction mixture was cooled to 5°C and a solution of benzoyl chloride (2.3 g, 0.017 M) in toluene (5 ml) and a solution of aqueous 50% sodium hydroxide (1.36 g, 0.017 M) were added dropwise simultaneously from separate addition funnels while maintaining the temperature at or below 10°C. Following the addition, the reaction mixture was warmed to room temperature and stirred for 1 hr. The reaction mixture was diluted with hexane and the solid N'-t-butyl-N-benzoylhydrazine was isolated by filtration.

To a stirred solution of the monobenzoylated compound (2 g, 0.010 M) in pyridine (15 ml) was added pivaloyl chloride (1.8 g, 0.015 M) and a catalytic amount of 4-dimethylamino pyridine. The mixture was heated to 60°C and stirred for approximately 1 hr., cooled and diluted with methylene chloride. The organic layer was washed with 10% HCl (3 x 25 ml) and water (50 ml), dried over magnesium sulfate and the solvent removed under vacuum. The solid product was recrystallized from methanol-ether to afford N'-t-butyl-N-benzoyl-N'-pivalovlhydrazine in 60% yield as a white solid. m.p. 217-220°C.

EXAMPLE No. 8 – Preparation of N'-t-butyl-N-benzoyl-N'-phenylacetylhydrazine

To a stirred suspension of t-butylhydrazine hydrochloride (1 g, 0.008 M) in toluene (30 ml) at room temperature was added dropwise a 50% aqueous solution of sodium hydroxide (0.64 g, 0.008 M). After 15 min., the reaction mixture was cooled to 5°C and a solution of benzoyl chloride (1.12 g, 0.008 M) in toluene (5 ml) and a solution of aqueous 50% sodium hydroxide (0.64 g, 0.008 M) were added dropwise simultaneously from separate addition funnels while maintaining the temperature at or below 10°C. Following the addition, the reaction mixture was warmed to room temperature and stirred for 1 hr. The reaction was diluted with hexane and the solid N'-t-butyl-N-benzoylhydrazine was isolated by filtration.

To a stirred solution of the monobenzoylated compound (1.5 g, 0.0078 M) in toluene (30 ml) at 5°C, were added dropwise simultaneously from separate addition funnels, solutions of phenylacetyl chloride (1.2 g, 0.008 M) in toluene (5 ml) and aqueous 50% sodium hydroxide solution (0.63 g, 0.0078 M) while maintaining the temperature below 10°C. Following the addition, the reaction mixture was warmed to room temperature and stirred for 1 hr. The mixture was then diluted with hexane and the solid product isolated by filtration. The product was washed with water and hexane and dried. The crude product was recrystallized from ether-methanol to afford N'-t-butyl-N-benzoyl-N'-phenylacetylhydrazine as a white powder. m.p. 167-169°C.

EXAMPLE NO. 10 – Preparation of N'-t-butyl-N-benzoyl-N'-(beta-trifluoromethylcrotonyl)hydrazine

N'-t-butyl-N-benzoylhydrazine (1.0 g) in 2 ml toluene was added dropwise to a solution of beta-trifluoromethyl-crotonyl chloride (prepared by addition of oxalyl chloride (1.3 g) to a solution of 1.5 g beta-trifluoromethyl-crotonic acid in 5 ml toluene at 23°C). After 30 min. at 23°C, the reaction mixture was partitioned between saturated aqueous sodium bicarbonate (20 ml) and ether (20 ml). The ether layer was evaporated under reduced pressure to about 10 ml and the excess hydrazine was removed by filtration. The filtrate was evaporated to give N'-t-butyl-N-benzoyl-N'-(beta-trifluoromethyl-crotonyl)hydrazine as a colorless oil.

EXAMPLE NO. 18 – Preparation of N'-t-butyl-N-benzoyl-N'-methacryloylhydrazine

N'-t-butyl-N-benzoylhydrazine (0.9 g) suspended in 10 ml toluene and 1 ml $H_2O$ containing 0.3 g 50% sodium hydroxide was treated with 1.2 g of methacryloyl chloride at 23°C. After 18 hours, 5 ml of hexane was added and N'-t-butyl-N-benzoyl-N'-methacryloylhydrazine was collected by filtration. m.p. 148-152°C.

EXAMPLE NO. 21 – Preparation of N'-t-butyl-N'-cyclobutanecarbonyl-N-(4-chlorobenzoyl)hydrazine

To a stirred solution of N'-t-butyl-N-(4-chlorobenzoyl)hydrazine (2.0 g, 8.8 mmole) in toluene (35 ml) at 5°C was added cyclobutanecarboxylic acid chloride (1.25 g, 10.5 mmole) in one portion. To the above mixture was dropwise added 50% NaOH solution (0.85 g, 10.6 mmole). After addition, the ice water bath was removed and the reaction mixture was stirred at room temperature overnight.

The mixture was diluted with hexane (30 ml) and $H_2O$ (30 ml) and stirred for another 30 min. The solid product was collected by suction-filtration and washed with $H_2O$ (100 ml) and hexane (100 ml) to yield 1.5 g of **N'-t-butyl-N'-cyclobutanecarbonyl-N-(4-chlorobenzoyl)hydrazine.**

By following substantially the procedures in Processes A, B and C as exemplified above by the preparation of the compounds of Examples 1, 2, 7, 8, 10, 18 and 21, the compounds of Formula I can be prepared and the compounds of the remaining Examples were prepared.

As previously noted, the compounds of the present invention exhibit excellent pesticidal activity and are selective against insects of the orders Lepidoptera and Coleoptera.

In general, for the control of insects in agriculture, horticulture and forestry, a dosage corresponding to from about 10 grams to about 10 kilograms of the active substance per hectare may be used and from about 100 grams to about 5 kilograms per hectare of the active substance is preferred. The exact amount of dosage for a given situation can be determined by routine testing and depends on a variety of factors, for example, the substance used, the kind of pest, the formulation used, the state of the crop infested with the pest and the prevailing weather conditions. The term "insecticidal" as employed in the specification and claims of this application is to be construed as any- means which adversely affects the existence or growth of the target insects. Such means can comprise a complete killing action, eradication, arresting in growth, inhibition, reducing in number or any combination thereof. The terms "control" and "combat" as employed in the specification and claims of this application is to be construed as including an "insecticidal" action and the protection of plants from insect damage. By "insecticidally effective amount" is meant that dosage of active substance sufficient to exert insect "control".

The compounds of the present invention, for practical applications, can be utilized in the form of compositions or formulations. Examples of the preparation of compositions and formulations can be found in the American Chemical Society publication "Pesticidal Formulation Research," (1969), Advances in Chemistry Series No. 86, written by Wade Van Valkenburg ; and the Marcel Dekker, Inc. publication "Pesticide Formulations," (1973), edited by Wade Van Valkenburg. In these compositions and formulations, the active substance is mixed with conventional inert agronomically acceptable (i.e., plant compatible and/or pesticidally inert) diluents or extenders such as solid carrier material or liquid carrier material, of the type usable in conventional compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, adjuvants such as surfactants, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait with non-lethal toxicants change the behavior, feeding habits and physiology of the pest for the purpose of control.

The invert emulsions are mainly used for aerial application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations can be prepared in a known manner, for instance by extending the active compounds with conventional dispersible liquid diluent carriers and/or dispersible solid carriers optionally with the use of carrier vehicle assistants, e.g., conventional surface-active agents, including emulsifying agents and/or dispersing agents, whereby, for example, in the case where water is used as diluent, organic solvents may be added as auxiliary solvents. The following may be chiefly considered for use as conventional carrier vehicles for this purpose : aerosol propellants which are gaseous at normal temperatures and pressures, such as halogenated hydrocarbons, e.g., dichlorodifluoromethane and trifluorochloromethane, as well as butane, propane, nitrogen and carbon dioxide ; inert dispersible liquid diluent carriers, including inert organic solvents, such as aromatic hydrocarbons (e.g., benzene, toluene, xylene, alkyl naphthalenes, etc.), halogenated, especially chlorinated, aromatic hydrocarbons (e.g., chlorobenzenes, etc.), cycloalkanes (e.g., cyclohexane, etc.), paraffins (e.g., petroleum or mineral oil fractions), chlorinated aliphatic hydrocarbons (e.g., methylene chloride, chloroethylenes, etc.), vegetable oils (e.g., soybean oil, cottonseed oil, corn oil, etc.), alcohols (e.g., methanol, ethanol, propanol, butanol, glycol, etc.) as well as ethers and esters thereof (e.g., glycol monomethyl ether), amines (e.g., ethanolamine), amides (e.g., dimethyl formamide), sulfoxides (e.g., dimethyl sulfoxide), acetonitrile, ketones (e.g., acetone, methyl ethyl ketone, methyl isobutyl ketone, cyclohexanone, isophorone), and/or water ; solid carriers including ground natural minerals, such as kaolins, clays, talc, chalk, quartz, attapulgite, montmorillonite or diatomaceous earth, and ground synthetic minerals, such as highly-dispersed silicic acid, alumina and silicates ; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks. The following may be chiefly considered for use as conventional carrier vehicle assistants : emulsifying agents, such as cationic and/or nonionic and/or anionic emulsifying agents (e.g., polyethylene oxide esters of fatty acids, polyethylene oxide ethers of fatty alcohols, alkyl sulfates, alkyl sulfonates, aryl sulfonates, albumin hydrolysates and especially alkyl arylpolyglycol ethers, magnesium stearate, sodium oleate) ; and/or dispersing agents, such as lignin, sulfite waste liquors, methyl cellulose.

Adhesives such as carboxymethylcellulose and natural and synthetic polymers in the form of powders, granules or latices, such as gum arabic, polyvinyl alcohol and polyvinyl acetate, can be used in the formulations.

If desired, it is possible to use colorants in compositions and formulations containing compounds of the present invention such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid and/or liquid dispersible carrier vehicles and/or with,other known compatible active agents, especially plant protection agents, such as other insecticides, arthropodicides, nematicides, fungicides, bactericides, rodenticides, herbicides, fertilizers, growth-regulating agents and synergists if desired, or in the form of particular dosage preparations for specific application made therefrom, such as solutions, emulsions, suspensions, powders, pastes, and granules which are thus ready for use.

As concerns commercially marketed preparations, these generally contemplate carrier composition mixtures in which the active compound is present in an amount substantially between about 0.1% and 99% by weight, and preferably between about 1% and 75% by weight, of the mixture. Carrier composition mixtures suitable for direct application or field application generally contemplate those in which the active compound is used in an amount substantially between about 0.0001% and 5%, preferably between about 0.001% and 3%, by weight of the mixture. Thus the present invention contemplates overall formulations and compositions which comprise mixtures of a conventional dispersible carrier such as (1) a dispersible inert finely divided carrier solid, and/or (2) a dispersible carrier liquid such as an inert organic solvent and/or water, preferably including a surface-active effective amount of a carrier vehicle assistant (e.g., a surface-active agent, such as an emulsifying agent and/or a dispersing agent), and an amount of the active compound generally between about 0.0001% and about 99% by weight of the composition, preferably between about 0.001% and about 90% by weight of the composition and more preferably between about 0.01% and about 75% by weight of the composition which is effective for the purpose in question.

The active compounds can be applied as sprays by conventional methods, such as conventional high-gallonage hydraulic sprays, low gallonage sprays, ultra-low-volume sprays, airblast spray, aerial sprays and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 microns or less) using airplane crop spraying techniques. Typically only a few liters per hectare are needed and often amounts up to about 15 to 1000 g/hectare, preferably about 40 to 600 g/hectare are sufficient. With ultra-low-volume, it is possible to use highly concentrated liquid compositions with said liquid carrier vehicles containing from about 20 to about 95% by weight of the active compound.

Furthermore, the present invention contemplates methods of selectively killing, combating or controlling pests, which comprises contacting insects with a correspondingly combative of at least one active compound of the invention alone or together with a carrier vehicle (composition or formulation) as noted above. The term "contacting" as employed in the specification and claims of this application is to be construed as applying to at least one of (a) such insects and (b) the corresponding habitat thereof (i.e., the locus to be protected, for example, to a growing crop or an area where a crop is to be grown) the active compound of this invention alone or as a constituent of a composition or formulation. The formulations or compositions are applied in the usual manner, for instance by spraying, atomizing, vaporizing, scattering, dusting, watering, squirting, sprinkling, pouring, fumigating, dry dressing, moist dressing, wet dressing, slurry dressing, encrusting or otherwise disseminating.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is

possible to go above or below the aforementioned concentration ranges.

Granular preparations are produced for example, by taking up the active substance in a solvent and by using the resulting solution, as the case may be in the presence of a binder, to impregnate a granular carrier material, such as porous granules (for example, pumice and attaclay) or chopped tobacco stems or the like.

A granular preparation (frequently termed a "pellet") may alternatively be produced by compressing the active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are conveniently produced by mixing from about 10 to about 99 parts by weight of a solid inert carrier such, for example, as the aforementioned carrier materials with from about 1 to about 80 parts by weight of active substance optionally dissolved in a volatile solvent such as acetone, from about 1 to about 5 parts by weight of dispersing agent such, for example, as the ligno-sulfonates or alkylnaphthalene sulfonates known for this purpose and preferably also from about 0.5 to about 5 parts by weight of wetting agent, such as fatty alcohol sulfates, or alkylarylsulfonates of fatty acid condensation products. In the case of flowables, a liquid inert carrier such as water also included.

To produce emulsifiable concentrates the active compound is dissolved or finely divided in a suitable solvent which preferably is poorly miscible with water, emulsifier being added to the resulting solution. Examples of suitable solvents are xylene, toluene, high-boiling aromatic petroleum distillates, for example solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e., preparation which when burning are capable of emitting a pesticidal smoke, are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

Representative preparation of compositions and formulations including the compounds of the present invention are set forth below as Examples A through I by way of illustration but not limitation.

Example A

Granular

| Ingredient | %/wt. |
| --- | --- |
| Toxicant and toxicant impurities | 0.25 |
| Triton X-305 (binder) (Octylphenyl-3-ethylene oxide ethanol) | 0.25 |
| Agsorb 24/48 (diluent) | 99.50 |

Preparation : The toxicant and Triton X-305 are dissolved into methylene chloride and the mixture is added to the Agsorb with continuous mixing. The methylene chloride is then allowed to evaporate.

Example B

Dust

| Ingredient | %/wt. |
| --- | --- |
| Toxicant and toxicant impurities | 1.0 |
| Talc 99.0 | |

Preparation : Toxicant is dissolved in excess acetone and the mixture is impregnated onto the talc. The acetone is then permitted to evaporate.

Example C

Wettable Powder

| Ingredient | %/wt. |
| --- | --- |
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| Barden clay (diluent) | 31.7 |
| HiSil 233 (diluent) (Sodium silica) | 30.0 |

Preparation : The toxicant, optionally dissolved in a volatile solvent, is absorbed onto the Barden clay and HiSil carriers. The Duponal and Reax are then added and the entire dry mixture blended until homogenous. The composition is then micronized to a fine particle size.

Example D

Emulsifiable Concentrate

| Ingredient | %/wt. |
|---|---|
| Toxicant and toxicant impurities | 15.0 |
| Sponto 232T (emulsifier) (Anionic and nonionic blend of the following surfactants: calcium dodecyl benzene sulfonate; and ethoxylated alkylphenol) | 6.0 |
| Sponto 234T (emulsifier) (Anionic and nonionic blend of the following surfactants: calcium dodecyl benzene sulfonate; and ethoxylated alkylphenol) | 4.0 |
| Cyclohexanone (solvent) | 22.5 |
| Tenneco 500-100 (solvent) (Aromatic solvent mixture principally comprising xylene, cumene and ethyl benzene having a boiling point range of 290-345°F) | 52.5 |

Preparation : All ingredients are mixed together with continuous agitation until a homogeneous clear solution is obtained.

Example E

Aerosol

| Ingredient | %/wt. |
|---|---|
| Toxicant and toxicant impurities | 0.5 |
| Freon 12 | 99.5 |

Preparation : The components are mixed and packaged under pressure in a suitable container equipped with a release spray valve.

Example F

Fumigating Candle or Fumigating Powder

| Ingredient | %/wt. |
|---|---|
| Toxicant and toxicant impurities | 1.0 |
| Wood dust | 96.0 |
| Starch | 3.0 |

Preparation : Toxicant, wood dust, and starch are blended together and then molded into a candle using a small amount of water to activate the starch.

Example G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon 4200 ((3-isothiazolone) preservative) | 0.05 |

Preparation : The corn oil and corn syrup are added to the wheat bran with adequate mixing. The toxicant and Kathon are premixed with excess acetone and this solution is added to the wheat bran base with continued mixing. The acetone is then permitted to evaporate.

Method B

| Ingredient | %/wt. |
|---|---|
| Toxicant and toxicant impurities | 0.06 |
| Granulated Sugar (carrier and attractant) | 99.94 |

Example M

Pellet

Same as Example G, Method A, with this addition : the bait composition is formed into 1/4" diameter by 3/8" long pellets using a suitable die and press apparatus.

Example 1

Flowable

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| HiSil 233 (diluent) (Sodium silica) | 30.0 |
| Kelzan (thickener) (Xanthan gum) | 0.5 |
| Water | 31.2 |

Preparation : The toxicant is absorbed onto the HiSil carrier. The Duponal and Reax are then added and the entire dry mixture blended until homogeneous. The composition is then micronized to a fine particle size. The resulting powder is suspended in water and the Kelzan added.

Compositions and formulations according to the present invention may also include known pesticidal compounds. This expands the spectrum of activity of the preparations and may give rise to synergism.

The following known insecticidal, fungicidal and acaricidal compound are suitable for use in such a combined preparation.

Insecticides such as :

Chlorinated hydrocarbons, for example, 2,2-bis(p-chlorophenyl)-1,1,1-trichloroethane and hexachloroepoxyoctahydrodimethanonaphthalene ;

Carbamates, for example, N-methyl-1-naphthylcarbamates ;

Dinitrophenols, for example, 2-methyl-4,6-dinitrophenol and 2-(2-butyl)-4,6-dinitrophenyl-3,3-dimethylacrylate ;

Organic phosphorus compounds, such as dimethyl-2-methoxy-3-carbonyl-1-methylvinyl phosphate, O,O-diethyl-O-p-nitrophenylphosphorothioate ; N-monomethylamide of O,O-dimethyldithiophosphorylacetic acid ;

Diphenylsulfides, for example, p-chlorobenzyl or p-chlorophenyl sulfide and 2,4,4′,5-tetrachlorodiphenylsulfide ;

Diphenylsulfonates, for example, p-chlorophenylbenzenesulfonate ;

Methylcarbinols, for example, 4,4-dichloro-1-trichloromethylbenzhydrol ;

Quinoxaline compounds, such as methylquinoxaline dithiocarbonate ;

Amidines such as N′-(4-chloro-2-methylphenyl) N,N-dimethylformamidine ;

Pyrethroids such as Allethrin ;

Biologicals such as Baccilus thuringiensis preparations ; Organic tin compounds such as tricyclohexyltin hydroxide ;

Synergists such as piperonyl butoxide.

Fungicides such as :

Organic mercury compounds, for example, phenylmercury-acetate and methylmercurycyanoguanide ;

Organic tin compounds, for example, triphenyltin hydroxide and triphenyltin acetate ;

Alkylenebisdithiocarbamates, for example, zinc ethylene-bisthiocarbamate and manganese ethylenebisdithiocarbamate ; and

2,4-dinitro-6-(2-octyl-phenylcrotonate), 1-bis(di-methylamino)phosporyl-3-phenyl-5-amino-1,2,4-triazole, 6-methylquinoxaline-2,3-dithiocarbonate, 1,4-dithioanthraquinone-2,3-dicarbonitrile, N-trichloromethylthiophthalimide, N-trichloromethylthiotetrahydrophthalimide, N-(1,1,2,2-tetrachloroethylthio)tetrahydrophthalimide, N-dichlorofluoromethylthio-N-phenyl-N′-dimethylsulfonyldiamide and tetrachloroisophthalonitrile.

Biological Activity

It has been found by biological evaluation that compounds of the invention have pesticidal activity and are capable of controlling larvae and adult forms of pests, especially insects from the orders Lepidoptera and Coleoptera. One skilled in the art will know how to determine the activity of a given compound against a given insect and the dosage required to obtain general or selective insecticidal effects.

As previously noted, the compounds of the invention are particularly suitable for controlling plant-destructive insects in crops of cultivated plants, such as, but not limited to, cotton, vegetables, corn and other cereals; forestry, such as, but not limited to, birch, spruce, pine and fir ; and ornamental plants, flowers and trees. Compounds of the invention are also particularly suitable for controlling insects destructive to stored commodities such as seeds ; fruit crops, such as, but not limited to, fruit and citrus trees and raspberry bushes ; and turf, such as, but not limited to, lawns and sod.

In evaluating the pesticidal activity of the compounds of this invention, the following test procedures were employed.

A test solution containing 600 parts per million (ppm) was made by dissolving the test compound in a solvent (acetone : methanol, 1 : 1), adding water to give an acetone :methanol :water system of 5 : 5 : 90 and then a surfactant. A 1 : 1 mixture of an alkylarylpolyetheralcohol (sold as Triton X-155) and a modified phthalic glycerol alkyl resin (sold as Triton B-1956) was utilized at the equivalent of 1 ounce per 100 gal. of test solution as a surfactant.

Initial evaluations were made on one or more of the following pests.

| Code Symbol | Common Name | Latin Name |
|---|---|---|
| SAW | Southern Armyworm | Spodoptera eridania |
| MBB | Mexican Bean Beetle | Epilachna varivestsis |

For the foliar bean beetle and armyworm tests, individual bean (Phaseolus limensis var. Woods' Prolific) leaves are placed on moistened pieces of filter paper in Petri dishes. The leaves are then sprayed with the test solution using a rotating turntable and allowed to dry. The dishes are infested with 10 third instar larvae of Southern armyworm or Mexican bean beetle. The dishes are then covered.

The percent mortaility for the bean beetle and armyworm evaluations are determined 96 hours after treatment. Evaluations are based on a scale of 0-100% in which 0 equals no activity and 100 equals total kill.

The rotating turntable consists of a fixed, continuously operating spray nozzle under which targets are rotated at a fixed speed and distance. When the target is a Petri dish (such as for the armywomn), the distance from the nozzle is 15 inches. The nozzle is located 8 inches from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At the 10 psig air pressure used and with liquid siphon feed 0.5 GPH (gallons per hour) are delivered in a round spray pattern with a 21° spray angle. Targets are misted with spray droplets to the point that the droplets coalesce to form a uniform thin film insufficient to drown test organisms.

All treatments are maintained at 75-80°F under continuous fluorescent light in a well-ventilated room.

For soil treatment (systemic) trials, a portion of the 600 ppm test solution is diluted to 150 ppm. Ten (10) ml of the 150 ppm test solution is pipetted into soil (approximately 200 g of standard greenhouse soil) in a 3-inch pot containing a lima bean seedling. This results in a soil concentration of approximately 8 ppm. Treated plants are maintained under existing greenhouse conditions for one week. Two bean leaves are removed and placed individually on moist filter paper in Petri dishes. One leaf is infested with 10 third instar larvae of Mexican bean beetle. The other leaf is infested with 10 third instar larvae of Southern armyworm. The dishes are then covered and held for 3 days at which time the percent control preclude insect starvation.

The results of the initial insecticidal evaluations are given in Table II.

Armyworm and bean beetle spray (foliar) results are 96 hour observations. Soil treatment results are 72 hour observations. At the discretion of the experimenter, particular evaluations were held for 144 hour observations. If, after 144 hours, there was a change in the percent control, it is shown in parentheses.

TABLE II

Biological Evaluations

| Example No. | Foliar Application Test Species SAW | Foliar Application Test Species MBB | Soil Application Test Species MBB | Soil Application Test Species SAW |
|---|---|---|---|---|
| 1 | 10 | 40 | --[a] | -- |
| 2 | 100 | 0 | 0 | 20 |
| 3 | 20[b] | 100[b] | -- | -- |
| 4 | 0[b] | 0[b] | -- | -- |
| 5 | 0[b] | 0[b] | -- | -- |
| 6 | 0[b] | 0[b] | -- | -- |
| 7 | 100 | 100 | 60(100) | 100 |
| 8 | 90 | 50 | 40(100) | 0(20) |
| 9 | 0 | 0 | 0 | 0 |
| 10 | 0 | 10 | 0 | 0 |
| 11 | 80 | 0[b] | -- | -- |
| 12 | 0 | 20 | 0 | 0 |
| 13 | 80 | 80 | 0 | 0 |
| 14 | 0 | 30 | 0 | 0 |
| 15 | 0 | 40 | 20 | 0 |
| 16 | 0 | 0 | 0 | 0 |
| 17 | 100 | 0 | 0 | 100 |
| 18 | 100 | 100 | 60(100) | 90 |
| 19 | 0 | 0 | 0 | 0 |
| 20 | 0 | 10 | 40(20) | 0 |
| 21 | 0 | 100 | 0 | 0 |
| 22 | 10[b] | 0[b] | -- | -- |

[a] Not tested

[b] 48 hour results

TABLE II (Cont'd)

Biological Evaluations

| Example No. | Foliar Application Test Species SAW | MBB | Soil Application Test Species MBB | SAW |
|---|---|---|---|---|
| 23 | 0 | 30 | - | - |
| 24 | 80 | 0 | - | - |
| 25 | 30 | 30 | - | - |
| 26 | 100 | 40 | - | - |
| 27 | 100 | 10 | - | - |
| 28 | 0 | 100 | - | - |
| 29 | 100 | 30 | - | - |
| 30 | 100 | 30 | - | - |
| 31 | 100 | 20 | - | - |
| 32 | 100 | 60 | - | - |
| 33 | 100 | 40 | - | - |
| 34 | 100 | 20 | - | - |
| 35 | 100 | 0 | - | - |
| 36 | 100 | 40 | - | - |
| 37 | 0 | 70 | - | - |
| 38 | 0 | 20 | - | - |
| 39 | 100 | 0 | - | - |
| 40 | | | - | - |

In its mechanical aspects therefore a process of the invention for improving the commercial value and/or profitability of vendible crops from plants whose growth is affected or likely to be affected by insects comprises (1) charging to a container, fumigation device or mechanical dissemination device an insecticidal composition of the invention as hereinbefore described (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

The following words are trademarks which may or may not be registered in some or all of the designated states : Triton, Agsorb, Duponal, Reax, Hisil, Sponto, Tenneco, Kathon and Kelzan.

## Claims

### Claims for the Contracting States : BE, CH, DE, FR, GB, GR, IT, LI, LU, NL, SE

1. An insecticidally active compound which is a compound of the formula

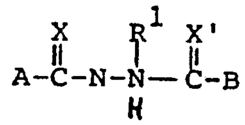

(I)

wherein

X and X′ are the same or different O, S or NR ;

$R^1$ is unsubstituted branched ($C_3$-$C_{10}$) alkyl or straight chain ($C_1$-$C_4$) alkyl substituted with one or two of the same or different ($C_3$-$C_6$) cycloalkyl ;

A is unsubstituted or substituted naphthyl where the substituents can be from one to three of the same or different halo ; cyano ; nitro ; hydroxy ; ($C_1$-$C_4$) alkoxy ; ($C_1$-$C_4$) alkyl ; carboxy ; ($C_1$-$C_4$) alkoxy-carbonyl ; ($C_1$-$C_4$) alkanoyloxy or $NH_2$, NHZ or –NZZ′ ;

unsubstituted or substituted phenyl where the substituents can be from one to five of the same or different halo ; nitro ; cyano ; hydroxy ; ($C_1$-$C_6$) alkyl ; halo-($C_1$-$C_6$) alkyl ; cyano-($C_1$-$C_6$) alkyl ; ($C_1$-$C_6$) alkoxy ; halo-($C_1$-$C_6$) alkoxy ; ($C_1$-$C_6$) alkoxy-($C_1$-$C_6$) alkyl having independently the stated number of carbon atoms in each alkyl group ; ($C_1$-$C_6$) alkoxy-($C_1$-$C_6$) alkoxy having independently the stated number of carbon atoms in each alkyl group ; $-OCO_2R$ group ; ($C_2$-$C_6$) alkenyl optionally substituted with halo, cyano, ($C_1$-$C_4$) alkyl, ($C_1$-$C_4$) alkoxy, halo-($C_1$-$C_4$) alkoxy or ($C_1$-$C_4$) alkylthio ; carboxy ; $-RCO_2R'$ group ; –COR ; halo-($C_1$-$C_6$) alkyl-carbonyl ; cyano-($C_1$-$C_6$) alkyl-carbonyl ; nitro-($C_1$-$C_6$) alkyl-carbonyl ; ($C_1$-$C_6$) alkoxy-carbonyl ; halo-($C_1$-$C_6$) alkoxy-carbonyl ; –OCOR group ; –NRR′ group ; amino substituted with hydroxy, ($C_1$-$C_4$) alkoxy or ($C_1$-$C_4$) alkylthio groups ; phenylamino ; diphenylamino ; –CONRR′ group ; –OCONRR′ group ; –NRCOR′ group ; $-NRCO_2R'$ group ; –N(COR)COR′ group ; –OCONRCOR′ group ; sulfhydryl ; halothio ; ($C_1$-$C_6$) alkylthio ; halo-($C_1$-$C_6$) alkylthio ; –SOR group ; $-SO_2R$ group ; phenylsulfonyl ; $-OSO_2R$ group ; halo-($C_1$-$C_6$)alkylsulfonyloxy ; $-SO_2NRR'$ group ; –NRSOR′ group ; $-NRSO_2R'$ group ; –CSR group ; $CS_2R$ group ; –NRCSR′ group ; –SCOR group ; unsubstituted or substituted phenyl having one to three of the same or different halo, cyano, nitro, hydroxy ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′; phenoxy where the phenyl ring is unsubstituted or substituted with one to three of the same or different halo, cyano, nitro, hydroxy, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; phenylthio where the phenyl ring is unsubstituted or substituted with one to three of the same or different halo, cyano, nitro, hydroxy, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; or when two adjacent positions on the phenyl ring are substituted with alkoxy groups, these groups may be joined to form, together with the carbon atoms to which they are both attached, a 5- or 6-membered dioxolano or dioxano heterocyclic ring ;

B is unsubstituted or substituted ($C_1$-$C_{10}$) alkyl having as the optional substituent(s) one to four of the same or different halo, cyano, nitro, hydroxy, ($C_1$-$C_4$) alkoxy, halo-($C_1$-$C_4$) alkoxy, carboxy, ($C_1$-$C_4$) alkoxycarbonyl, ($C_1$-$C_4$) alkanoyloxy, phenyl, $NH_2$, NHZ or –NZZ′.

unsubstituted or substituted ($C_3$-$C_8$) cycloalkyl or unsubstituted or substituted ($C_3$-$C_8$) cycloalkyl ($C_1$-$C_4$) alkyl, having as the optional substituent(s) one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkanoyl, ($C_1$-$C_4$) alkoxy-carbonyl, ($C_1$-$C_4$) alkanoyloxy, phenyl, $NH_2$, NHZ or –NZZ′ ;

unsubstituted or substituted ($C_2$-$C_8$) alkenyl or unsubstituted or substituted ($C_3$-$C_8$) alkadienyl having, as the optional substituent(s), a furyl, thienyl or pyridyl group or one to four of the same or different halo, cyano, nitro, hydroxy, ($C_1$-$C_4$) alkyl, ($C_3$-$C_6$) cycloalkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ;

unsubstituted or substituted ($C_3$-$C_8$)cycloalkenyl or unsubstituted or substituted ($C_6$-$C_8$) cycloalkadienyl having as the optional substituent(s), one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ;

unsubstituted or substituted ($C_2$-$C_8$) alkynyl having, as optional substituent(s), one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, phenyl, $NH_2$, NHZ or –NZZ′ ;

phenalkyl having one to four carbon atoms in the alkyl group and wherein the alkyl group is unsubstituted or substituted with one to three of the same or different halo, cyano, hydroxy, ($C_1$-$C_4$) alkyl, ($C_1$-$C_4$) alkoxy, ($C_1$-$C_4$) alkoxy-carbonyl, $NH_2$, NHZ or –NZZ′, and the phenyl ring is unsubstituted or substituted with one to three of the same or different halo, cyano, nitro, hydroxy, ($C_1$-$C_4$) alkyl, halo-($C_1$-$C_4$) alkyl, cyano-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, ($C_2$-$C_6$) alkenyl, halo-($C_2$-$C_6$) alkenyl, ($C_2$-$C_6$) alkynyl, $NH_2$, NHZ or –NZZ′ ; or

phenalkenyl having two to six carbon atoms in the alkenyl group and the alkenyl group is unsubstituted or substituted with one to three of the same or different halo, cyano, hydroxy, $(C_1-C_4)$ alkyl, halo-$(C_1-C_4)$ alkyl, $(C_1-C_4)$ alkoxy, halo-$(C_1-C_4)$ alkoxy, $(C_1-C_4)$ alkoxy-carbonyl, $NH_2$, NHZ or –NZZ', and the phenyl ring is unsubstituted or substituted with one to three of the same or different halo, cyano, nitro, hydroxy, $(C_1-C_4)$ alkyl, halo-$(C_1-C_4)$ alkyl, $(C_1-C_4)$ alkoxy, halo-$(C_1-C_4)$ alkoxy, carboxy, $(C_1-C_4)$ alkoxy-carbonyl, $(C_1-C_4)$ alkanoyloxy or $NH_2$, NHZ or –NZZ' ;

where R and R' are hydrogen or $(C_1-C_6)$ alkyl ; Z and Z' are $(C_1-C_4)$ alkyl ; "amino" means NRR' ; or an agronomically acceptable salt thereof ; except for such a compound in which X and X' are O, $R^1$ is t-butyl, A is unsubstituted phenyl and B is unsubstituted methyl or unsubstituted ethyl.

2. A compound according to claim 1 which is a compound wherein

X and X' are O or S ; and/or

$R^1$ is unsubstituted branched $(C_3-C_8)$ alkyl or a straight chain $(C_1-C_4)$ alkyl substituted with one or two of the same or different $(C_3-C_4)$ cycloalkyl ; and/or

A is unsubstituted naphthyl ; or

unsubstituted or substituted phenyl where the substituents can be from one to three of the same or different halo ; nitro, cyano ; $(C_1-C_4)$ alkyl ; halo-$(C_1-C_4)$ alkyl ; cyano-$(C_1-C_4)$ alkyl, $(C_1-C_4)$ alkoxy ; $(C_1-C_4)$ alkoxy-$(C_1-C_4)$ alkyl ; –COD ; carboxy ; $(C_1-C_4)$ alkoxy-carbonyl ; $(C_1-C_4)$ alkanoyloxy ; $NH_2$, NHZ or –NZZ' ; $(C_1-C_4)$ alkylthio ; –CSD ; $-CS_2D$ ; –SCOD ; unsubstituted or substituted phenyl having one to two of the same or different halo, nitro,$(C_1-C_4)$ alkyl, $(C_1-C_4)$ alkoxy, carboxy, $(C_1-C_4)$ alkoxy-carbonyl, $(C_1-C_4)$ alkanoyloxy, $NH_2$, NHZ or –NZZ' ; or when two adjacent positions on the phenyl ring are substituted with alkoxy groups, these groups are joined to form, together with the carbon atoms to which they are attached, a 5- or 6-membered dioxolano or dioxano heterocyclic ring ; and/or

B is unsubstituted or substituted $(C_1-C_8)$ alkyl having as the optional substituent(s) one to three of the same or different halo, cyano, $(C_1-C_4)$alkoxy, phenyl, $(C_1-C_4)$ alkoxy-carbonyl or halo-$(C_1-C_4)$ alkoxy ;

unsubstituted or substituted $(C_3-C_6)$ cycloalkyl or unsubstituted or substituted $(C_3-C_5)$ cycloalkyl-$(C_1-C_4)$ alkyl, having as the optional substituent(s) one or two of the same or different halo, $(C_1-C_4)$ alkyl, halo-$(C_1-C_4)$ alkyl, $(C_1-C_4)$ alkoxy, $(C_1-C_4)$ alkanoyl or $(C_1-C_4)$ haloalkoxy ;

unsubstituted or substituted $(C_2-C_6)$alkenyl having as the optional substituent(s) a furyl or one to three of the same or different $(C_1-C_4)$ alkyl, halo-$(C_1-C_4)$ alkyl, $(C_1-C_4)$ alkoxy or halo-$(C_1-C_4)$alkoxy ;

unsubstituted or substituted $(C_1-C_6)$ cycloalkenyl having as the optional substituent(s) one or two of the same or different halo, $(C_1-C_4)$ alkyl, halo-$(C_1-C_4)$alkyl, $(C_1-C_4)$ alkoxy or halo-$(C_1-C_4)$ alkoxy ;

unsubstituted or phenyl substituted alkynyl ; phenalkyl having one or two carbon atoms in the alkyl group and the alkyl group is unsubstituted or substituted with one or two of the same or different halo, $(C_1-C_4)$ alkyl, halo-$(C_1-C_4)$ alkyl, $(C_1-C_4)$ alkoxy or halo-$(C_1-C_4)$ alkoxy and the phenyl ring is unsubstituted or substituted with one or two of the same or different halo, $(C_1-C_4)$ alkyl, halo-$(C_1-C_4)$ alkyl, $(C_1-C_4)$ alkoxy or halo-$(C_1-C_4)$ alkoxy; or

phenalkenyl having two or three carbon atoms in the alkenyl group and the alkenyl group is unsubstituted or substituted with halo, $(C_1-C_4)$ alkyl, halo-$(C_1-C_4)$ alkyl, $(C_1-C_4)$ alkoxy or halo-$(C_1-C_4)$ alkoxy and the phenyl ring is unsubstituted or substituted with one or two of the same or different halo, $(C_1-C_4)$ alkyl, halo-$(C_1-C_4)$ alkyl, $(C_1-C_4)$ alkoxy or halo-$(C_1-C_4)$ alkoxy ; or an

agronomically acceptable salt thereof ; wherein D is hydrogen or $(C_1-C_4)$ alkyl and Z and Z' are $(C_1-C_4)$ alkyl.

3. A compound according to claim 2 which is a compound wherein

X and X' are O or S ; and/or

$R^1$ is branched $(C_3-C_8)$ alkyl ; and/or

A is unsubstituted naphthyl ; or unsubstituted or substituted phenyl having one to three of the same or different halo, nitro, cyano, $(C_1-C_4)$ alkyl, halo-$(C_1-C_4)$ alkyl, cyano-$(C_1-C_4)$ alkyl, $(C_1-C_4)$ alkoxy, –COD, $(C_1-C_4)$ alkoxy-carbonyl, $(C_1-C_4)$ alkanoyloxy, or

unsubstituted or substituted phenyl having one or two of the same or different halo, nitro, $(C_1-C_4)$ alkyl, $(C_1-C_4)$ alkoxy, carboxy, $(C_1-C_4)$ alkoxy-carbonyl, $(C_1-C_4)$ alkanoyloxy, $NH_2$, NHZ or –NZZ' ; and/or

B is unsubstituted or substituted $(C_1-C_6)$ alkyl having one to three of the same or different halo, cyano, $(C_1-C_4)$ alkoxy, phenyl, $(C_1-C_3)$ alkoxy-carbonyl or halo-$(C_1-C_4)$ alkoxy ;

unsubstituted or substituted $(C_3-C_6)$ cycloalkyl or unsubstituted or substituted $(C_3-C_6)$ cycloalkyl-$(C_1-C_4)$ alkyl where the optional substituent is halo, $(C_1-C_4)$ alkyl, $(C_1-C_4)$ alkanoyl, $(C_1-C_4)$ alkoxy or halo-$(C_1-C_4)$ alkoxy ;

unsubstituted or substituted $(C_2-C_6)$ alkenyl having one or two of the same or different $(C_1-C_4)$ alkyl, $(C_1-C_4)$ alkoxy or halo-$(C_1-C_4)$ alkoxy ;

unsubstituted or substituted $(C_4-C_6)$ cycloalkenyl where the substituent is halo, $(C_1-C_4)$ alkyl, $(C_1-C_4)$ alkoxy

or halo-($C_1$-$C_4$) alkoxy ; or
unsubstituted or phenyl-substituted alkynyl ; or an agronomically acceptable salt thereof.

4. A compound according to claim 3 which is a compound wherein
X and X′ are O ; and/or
$R^1$ is branched ($C_4$-$C_7$) alkyl ; and/or
A is unsubstituted, monosubstituted, 2,3-disubstituted or 2,4-disubstituted phenyl where the substituents can be the same or different halo, ($C_1$-$C_4$) alkyl, ($C_1$-$C_4$) alkoxy, or halo-($C_1$-$C_4$) alkyl ; and/or
B is unsubstituted or substituted ($C_1$-$C_6$) alkyl having one to three of the same or different halo, phenyl or cyano ;
unsubstitute4 ($C_4$-$C_6$) cycloalkyl ;
unsubstituted or substituted ($C_2$-$C_5$) alkenyl having as the optional substituent(s) one or two of the same or different ($C_1$-$C_4$) alkyl ;
unsubstituted ($C_4$-$C_6$) cycloalkenyl ; or
phenalkyl having one or two carbon atoms in alkyl group and the phenyl ring is unsubstituted or substituted with one or two of the same or different halo, ($C_1$-$C_4$) alkyl, or ($C_1$-$C_4$) alkoxy ;
or an agronomically acceptable salt thereof.

5. A compound according to claim 4 which is a compound wherein
X and X′ are O ; and/or
$R^1$ is t-butyl, neopentyl (2,2-dimethylpropyl) or 1,2,2-trimethylpropyl ; and/or
A is unsubstituted or monosubstituted phenyl where the substituent can be chloro, fluoro, bromo, iodo, methyl, ethyl, methoxy or trifluoromethyl ; and/or
B is unsubstituted or substituted ($C_1$-$C_4$) alkyl having as the optional substituent(s) one to three of the same or different, phenyl, chloro, fluoro or bromo ;
unsubstituted ($C_4$-$C_6$) cycloalkyl ;
unsubstituted ($C_4$-$C_6$) cycloalkenyl ; or
phenalkyl having one to two carbon atoms in the alkyl group and the phenyl ring is unsubstituted or substituted with one or two of the same or different chloro, fluoro, bromo, iodo, methyl, ethyl, methoxy or trifluoromethyl ; or an
agronomically acceptable salt thereof.

6. A compound according to claim 3 which is a compound wherein
X and X′ are O ;
$R^1$ is t-butyl ; and
A is phenyl and B is cyclohexyl, benzyl, 1-cyclohexenyl, 2-methylcyclohexadi-2,5-enyl,phenylethynyl, 1-methylpropyl, 2-methyl-1-phenylbutyl, 3-acetyl-2,2-dimethylcyclobutylmethyl, 1-butenyl, 3-butenyl, 3-carbomethoxypropyl or methoxymethyl ; or
A is 3,5-dichlorophenyl and B is 2-bromoethyl ; or
A is 4-chlorophenyl and B is cyclobutyl ; or
A is 2,3-dimethylphenyl and B is 1-methylethenyl or 1-ethylethenyl ; or
A is 4-ethylphenyl and B is 1-methylethenyl.

7. An insecticidal composition comprising an agronomically acceptable diluent or carrier and an insecticidal compound as claimed in any of claims 1 to 6.

8. A composition according to claim 7 containing the insecticidal compound in an amount of from 0.0001, preferably 0.001, to 99% by weight.

9. A composition according to claim 7 containing the insecticidal compound in an amount of from 0.01 to 99% by weight.

10. A composition according to any of claims 7 to 9 in the form of a wettable powder, a flowable, a dust, a granule, a bait or an emulsifiable concentrate.

11. A method of controlling insects which comprises contacting said insects with insecticidal compound according to any of claims 1 to 6, optionally in a composition according to any of claims 7 to 9.

12. A method according to claim 11 which comprises applying the compound at a rate of from about 10 grams to about 10 kilograms per hectare, to growing plants or to an area where plants are to be grown.

13. A method according to claim 12 wherein the rate of application is 100 grams to 5 kilograms of the compound per hectare.

14. A method according to claim 13 of controlling insects from the order Lepidoptera or Coleoptera.

**Claims for the Contracting State : AT**

1. An insecticidally active composition which comprises a compound of the formula

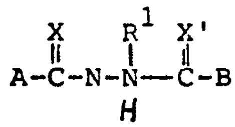

(I)

wherein

X and X′ are the same or different O, S or NR ;

$R^1$ is unsubstituted branched ($C_3$-$C_{10}$) alkyl or straight chain ($C_1$-$C_4$) alkyl substituted with one or two of the same or different ($C_3$-$C_6$) cycloalkyl ;

A is unsubstituted or substituted naphthyl where the substituents can be from one to three of the same or different halo ; cyano ; nitro ; hydroxy ; ($C_1$-$C_4$) alkoxy ; ($C_1$-$C_4$) alkyl ; carboxy ; ($C_1$-$C_4$) alkoxy-carbonyl ; ($C_1$-$C_4$) alkanoyloxy or $NH_2$, NHZ or –NZZ′ ;

unsubstituted or substituted phenyl where the substituents can be from one to five of the same or different halo ; nitro ; cyano ; hydroxy ; ($C_1$-$C_6$) alkyl ; halo-($C_1$-$C_6$) alkyl ; cyano-(C1-$C_6$) alkyl ; ($C_1$-$C_6$) alkoxy ; halo-($C_1$-$C_6$) alkoxy ; ($C_1$-$C_6$) alkoxy-($C_1$-$C_6$) alkyl having independently the stated number of carbon atoms in each alkyl group ; ($C_1$-$C_6$) alkoxy-($C_1$-$C_6$) alkoxy having independently the stated number of carbon atoms in each alkyl group ; –$OCO_2R$ group ; ($C_2$-$C_6$) alkenyl optionally substituted with halo, cyano, ($C_1$-$C_4$) alkyl, ($C_1$-$C_4$) alkoxy, halo-($C_1$-$C_4$) alkoxy or ($C_1$-$C_4$) alkylthio ; carboxy ; –$RCO_2R'$ group ; –COR ; halo-($C_1$-$C_6$) alkyl-carbonyl ; cyano-($C_1$-$C_6$) alkyl-carbonyl ; nitro-($C_1$-$C_6$) alkyl-carbonyl ; ($C_1$-$C_6$) alkyl-carbonyl ; halo-($C_1$-$C_6$) alkoxy-carbonyl ; –OCOR group ; –NRR′ group ; amino substituted with hydroxy, ($C_1$-$C_4$) alkoxy or ($C_1$-$C_4$) alkylthio groups ; phenylamino ; diphenylamino ; –CONRR′ group ; –OCONRR′ group ; -NRCOR′ group ; –$NRCO_2R'$ group ; –N(COR)COR′ group ; –OCONRCOR′ group ; sulfhydryl ; halothio ; ($C_1$-$C_6$) alkylthio ; halo-($C_1$-$C_6$) alkylthio ; –SOR group ; –$SO_2R$ group ; phenylsulfonyl ; –$OSO_2R$ group ; halo-($C_1$-$C_6$) alkylsulfonyloxy ; –$SO_2NRR'$ group; –NRSOR′ group ; –$NRSO_2R'$ group ; –SR group ; $CS_2R$ group ; –NRCSR′ group ; –SCOR group ; unsubstituted or substituted phenyl having one to three of the same or different halo, cyano, nitro, hydroxy ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; phenoxy where the phenyl ring is unsubstituted or substituted with one to three of the same or different halo, cyano, nitro, hydroxy, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; phenylthio where the phenyl ring is unsubstituted or substituted with one to three of the same or different halo, cyano, nitro, hydroxy, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; or when two adjacent positions on the phenyl ring are substituted with alkoxy groups, these groups may be joined to form, together with the carbon atoms to which they are both attached, a 5- or 6-membered dioxolano or dioxano heterocyclic ring ;

B is unsubstituted or substituted ($C_1$-$C_{10}$) alkyl having as the optional substituent(s) one to four of the same or different halo, cyano, nitro, hydroxy, ($C_1$-$C_4$) alkoxy, halo-($C_1$-$C_4$) alkoxy, carboxy, ($C_1$-$C_4$) alkoxycarbonyl, ($C_1$-$C_4$) alkanoyloxy, phenyl, $NH_2$, NHZ or –NZZ′

unsubstituted or substituted ($C_3$-$C_8$) cycloalkyl or unsubstituted or substituted ($C_3$-$C_8$) cycloalkyl ($C_1$-$C_4$) alkyl, having as the optional substituent(s) one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkanoyl, ($C_1$-$C_4$) alkoxy-carbonyl, ($C_1$-$C_4$) alkanoyloxy, phenyl, $NH_2$, NHZ or –NZZ′ ;

unsubstituted or substituted ($C_2$-$C_8$) alkenyl or unsubstituted or substituted ($C_3$-$C_8$) alkadienyl having, as the optional substituent(s), a furyl, thienyl or pyridyl group or one to four of the same or different halo, cyano, nitro, hydroxy, ($C_1$-$C_4$) alkyl, ($C_3$-$C_6$) cycloalkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ;

unsubstituted or substituted ($C_3$-$C_8$) cycloalkenyl or unsubstituted or substituted ($C_6$-$C_8$) cycloalkadienyl having as the optional substituent(s), one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ;

unsubstituted or substituted ($C_2$-$C_8$) alkynyl having, as optional substituent(s), one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, phenyl, $NH_2$, NHZ or –NZZ′ ;

phenalkyl having one to four carbon atoms in the alkyl group and wherein the alkyl group is unsubstituted or substituted with one to three of the same or different halo, cyano, hydroxy, ($C_1$-$C_4$) alkyl, ($C_1$-$C_4$) alkoxy, ($C_1$-$C_4$) alkoxy-carbonyl, $NH_2$, NHZ or –NZZ′, and the phenyl ring is unsubstituted or substituted with one to three of the same or different halo, cyano, nitro, hydroxy, ($C_1$-$C_4$) alkyl, halo-($C_1$-$C_4$) alkyl, cyano-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, ($C_2$-$C_6$) alkenyl, halo-($C_2$-$C_6$) alkenyl, ($C_2$-$C_6$) alkynyl, $NH_2$, NHZ or –NZZ′ ; or

phenalkenyl having two to six carbon atoms in the alkenyl group and the alkenyl group is unsubstituted or substituted with one to three of the same or different halo, cyano, hydroxy, $(C_1-C_4)$ alkyl, halo-$(C_1-C_4)$ alkyl, $(C_1-C_4)$ alkoxy, halo-$(C_1-C_4)$ alkoxy, $(C_1-C_4)$ alkoxy-carbonyl, $NH_2$, NHZ or –NZZ', and the phenyl ring is unsubstituted or substituted with one to three of the same or different halo, cyano, nitro, hydroxy, $(C_1-C_4)$ alkyl, halo-$(C_1-C_4)$ alkyl, $(C_1-C_4)$ alkoxy, halo$(C_1-C_4)$ alkoxy, carboxy, $(C_1-C_4)$ alkoxy-carbonyl, $(C_1-C_4)$ alkanoyloxy or $NH_2$, NHZ or –NZZ' ;

where R and R' are hydrogen or $(C_1-C_6)$ alkyl ; Z and Z' are $(C_1-C_4)$ alkyl ; "amino" means NRR' ; or an agronomically acceptable salt thereof ; except for such a compound in which X and X' are O, $R^1$ is t-butyl, A is unsubstituted phenyl and B is unsubstituted methyl or unsubstituted ethyl ; together with agronomically acceptable diluent or carrier.

2. A composition according to claim 1 wherein

X and X' are O or S ; and/or

$R^1$ is unsubstituted branched $(C_3-C_8)$ alkyl or a straight chain $(C_1-C_4)$ alkyl substituted with one or two of the same or different $(C_3-C_4)$ cycloalkyl ; and/or

A is unsubstituted naphthyl ; or

unsubstituted or substituted phenyl where the substituents can be from one to three of the same or different halo ; nitro, cyano ; $(C_1-C_4)$ alkyl ; halo-$(C_1-C_4)$ alkyl ; cyano-$(C_1-C_4)$ alkyl, $(C_1-C_4)$ alkoxy ; $(C_1-C_4)$ alkoxy-$(C_1-C_4)$ alkyl ; –COD ; carboxy ; $(C_1-C_4)$ alkoxy-carbonyl ; $(C_1-C_4)$ alkanoyloxy ; $NH_2$, NHZ or –NZZ' ;$(C_1-C_4)$ alkylthio ; –CSD ; $-CS_2D$ ; –SCOD ; unsubstituted or substituted phenyl having one to two of the same or different halo, nitro, $(C_1-C_4)$ alkyl, $(C_1-C_4)$ alkoxy, carboxy, $(C_1-C_4)$ alkoxy-carbonyl, $(C_1-C_4)$ alkanoyloxy, $NH_2$, NHZ or –NZZ' ; or when two adjacent positions on the phenyl ring are substituted with alkoxy groups, these groups are joined to form, together with the carbon atoms to which they are attached, a 5- or 6-membered dioxolano or dioxano heterocyclic ring ; and/or

B is unsubstituted or substituted $(C_1-C_6)$ alkyl having as the optional substituent(s) one to three of the same or different halo, cyano, $(C_1-C_4)$ alkoxy, phenyl, $(C_1-C_4)$ alkoxy-carbonyl or halo-$(C_1-C_4)$ alkoxy ;

unsubstituted or substituted $(C_3-C_6)$ cycloalkyl or unsubstituted or substituted $(C_3-C_5)$ cycloalkyl-$(C_1-C_4)$ alkyl, having as the optional substituent(s) one or two of the same or different halo, $(C_1-C_4)$ alkyl, halo-$(C_1-C_4)$ alkyl, $(C_1-C_4)$ alkoxy, $(C_1-C_4)$ alkanoyl or $(C_1-C_4)$ haloalkoxy ;

unsubstituted or substituted $(C_2-C_6)$ alkenyl having as the optional substituent(s) a furyl or one to three of the same or different $(C_1-C_4)$ alkyl, halo-$(C_1-C_4)$ alkyl, $(C_1-C_4)$ alkoxy or halo-$(C_1-C_4)$ alkoxy ;

unsubstituted or substituted $(C_3-C_6)$ cycloalkenyl having as the optional substituent(s) one or two of the same or different halo, $(C_1-C_4)$ alkyl, hale-$(C_1-C_4)$ alkyl, $(C_1-C_4)$ alkoxy or halo-$(C_1-C_4)$ alkoxy ;

unsubstituted or phenyl substituted alkynyl ; phenalkyl having one or two carbon atoms in the alkyl group and the alkyl group is unsubstituted or substituted with one or two of the same or different halo, $(C_1-C_4)$ alkyl, halo-$(C_1-C_4)$ alkyl, $(C_1-C_4)$ alkoxy or halo-$(C_1-C_4)$ alkoxy and the phenyl ring is unsubstituted or substituted with one or two of the same or different halo, $(C_1-C_4)$ alkyl, halo-$(C_1-C_4)$ alkyl, $(C_1-C_4)$ alkoxy or halo-$(C_1-C_4)$ alkoxy; or

phenalkenyl having two or three carbon atoms in the alkenyl group and the alkenyl group is unsubstituted or substituted with halo, $(C_1-C_4)$ alkyl, halo-$(C_1-C_4)$ alkyl, $(C_1-C_4)$ alkoxy or halo-$(C_1-C_4)$ alkoxy and the phenyl ring is unsubstituted or substituted with one or two of the same or different halo, $(C_1-C_4)$ alkyl, halo-$(C_1-C_4)$ alkyl, $(C_1-C_4)$ alkoxy or halo-$(C_1-C_4)$ alkoxy ;

wherein D is hydrogen or $(C_1-C_4)$ alkyl and Z and Z' are $(C_1-C_4)$ alkyl.

3. A composition according to claim 2 wherein

X and X' are O or S ; and/or

$R^1$ is branched $(C_3-C_8)$ alkyl ; and/or

A is unsubstituted naphthyl ; or unsubstituted or substituted phenyl having one to three of the same or different halo, nitro, cyano, $(C_1-C_4)$ alkyl, halo-$(C_1-C_4)$ alkyl, cyano-$(C_1-C_4)$ alkyl, $(C_1-C_4)$ alkoxy, –COD, $(C_1-C_4)$ alkoxy-carbonyl, $(C_1-C_4)$ alkanoyloxy, or unsubstituted or substituted phenyl having one or two of the same or different halo, nitro, $(C_1-C_4)$ alkyl, $(C_1-C_4)$ alkoxy, carboxy, $(C_1-C_4)$ alkoxy-carbonyl, $(C_1-C_4)$ alkanoyloxy, $NH_2$, NHZ or –NZZ' ; and/or

B is unsubstituted or substituted $(C_1-C_6)$ alkyl having one to three of the same or different halo, cyano, $(C_1-C_4)$ alkoxy, phenyl, $(C_1-C_3)$ alkoxy-carbonyl or halo-$(C_1-C_4)$ alkoxy ;

unsubstituted or substituted $(C_3-C_6)$ cycloalkyl or unsubstituted or substituted $(C_3-C_6)$ cycloalkyl-$(C_1-C_4)$ alkyl where the optional substituent is halo, $(C_1-C_4)$ alkyl, $(C_1-C_4)$ alkanoyl, $(C_1-C_4)$ alkoxy or halo-$(C_1-C_4)$ alkoxy ;

unsubstituted or substituted $(C_2-C_6)$ alkenyl having one or two of the same or different $(C_1-C_4)$ alkyl, $(C_1-C_4)$ alkoxy or halo-$(C_1-C_4)$ alkoxy ;

unsubstituted or substituted $(C_4-C_6)$ cycloalkenyl where the substituent is halo, $(C_1-C_4)$ alkyl, $(C_1-C_4)$ alkoxy

or halo-($C_1$-$C_4$) alkoxy ; or

unsubstituted or phenyl-substituted alkynyl.

4. A composition according to claim 3 wherein

X and X' are O ; and/or

$R^1$ is branched ($C_4$-$C_7$) alkyl ; and/or

A is unsubstituted, monosubstituted, 2,3-disubstituted or 2,4-disubstituted phenyl where the substituents can be the same or different halo, ($C_1$-$C_4$) alkyl, ($C_1$-$C_4$) alkoxy, or halo-($C_1$-$C_4$) alkyl ; and/or

B is unsubstituted or substituted ($C_1$-$C_6$) alkyl having one to three of the same or different halo, phenyl or cyano ;

unsubstituted ($C_4$-$C_6$) cycloalkyl ;

unsubstituted or substituted ($C_2$-$C_5$) alkenyl having as the optional substituent(s) one or two of the same or different ($C_1$-$C_4$) alkyl ;

unsubstituted ($C_4$-$C_6$) cycloalkenyl ; or

phenalkyl having one or two carbon atoms in alkyl group and the phenyl ring is unsubstituted or substituted with one or two of the same or different halo, ($C_1$-$C_4$) alkyl, or ($C_1$-$C_4$) alkoxy,

5. A composition according to claim 4 wherein

X and X' are O ; and/or

$R^1$ is t-butyl, neopentyl (2,2-dimethylpropyl) or 1,2,2-trimethylpropyl ; and/or

A is unsubstituted or monosubstituted phenyl where the substituent can be chloro, fluoro, bromo, iodo, methyl, ethyl, methoxy or trifluoromethyl ; and/or

B is unsubstituted or substituted ($C_1$-$C_4$) alkyl having as the optional substituent(s) one to three of the same or different, phenyl, chloro, fluoro or bromo ;

unsubstituted ($C_4$-$C_6$) cycloalkyl ;

unsubstituted ($C_4$-$C_6$) cycloalkenyl ; or

phenalkyl having one to two carbon atoms in the alkyl group and the phenyl ring is unsubstituted or substituted with one or two of the same or different chloro, fluoro, bromo, iodo, methyl, ethyl, methoxy or trifluoromethyl.

6. A composition according to claim 3 wherein

X and X' are O ;

$R^1$ is t-butyl ; and

A is phenyl and B is cyclohexyl, benzyl, 1-cyclohexenyl, 2-methylcyclohexadi-2,5-enyl, phenylethynyl, 1-methylpropyl, 2-methyl-1-phenylbutyl, 3-acetyl-2,2-dimethylcyclobutylmethyl, 1-butenyl, 3-butenyl, 3-carbomethoxypropyl or methoxymethyl ; or

A is 3,5-dichlorophenyl and B is 2-bromoethyl ; or

A is 4-chlorophenyl and B is cyclobutyl ; or

A is 2,3-dimethylphenyl and B is 1-methylethenyl or 1-ethylethenyl ; or

A is 4-ethylphenyl and B is 1-methylethenyl.

7. A composition according to any preceding claim containing the insecticidal compound in an amount of from 0,0001, preferably 0.001, to 99% by weight.

8. A composition according to claim 7 containing the insecticidal compound in an amount of from 0.01 to 99% by weight.

9. A composition according to claims 7 or 8 in the form of a wettable powder, a flowable, a dust, a granule, a bait or an emulsifiable concentrate.

10. A method of controlling insects which comprises contacting said insects with insecticidal compound as defined in any of claims 1 to 6, optionally in a composition according to any of claims 1 to 9.

11. A method according to claim 10 which comprises applying the compound at a rate of from about 10 grams to about 10 kilograms per hectare, to growing plants or to an area where plants are to be grown.

12. A method according to claim 11 wherein the rate of application is 100 grams to 5 kilograms of the compound per hectare.

13. A method according to claim 12 of controlling insects from the order Lepidoptera or Coleoptera.

**Claims for the Contracting State : ES**

1. The use of an insecticidally active compound which is a compound of the formula

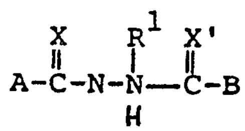

(I)

wherein

X and X′ are the same or different O, S or NR ;

$R^1$ is unsubstituted branched ($C_3$-$C_{10}$) alkyl or straight chain ($C_1$-$C_4$) alkyl substituted with one or two of the same or different ($C_3$-$C_6$) cycloalkyl ;

A is unsubstituted or substituted naphthyl where the substituents can be from one to three of the same or different halo ; cyano ; nitro ; hydroxy ; ($C_1$-$C_4$) alkoxy ; ($C_1$-$C_4$) alkyl ; carboxy ; ($C_1$-$C_4$) alkoxy-carbonyl ; ($C_1$-$C_4$) alkanoyloxy or $NH_2$, NHZ or –NZZ′ ;

unsubstituted or substituted phenyl where the substituents can be from one to five of the same or different halo ; nitro ; cyano ; hydroxy ; ($C_1$-$C_6$) alkyl ; halo-($C_1$-$C_6$) alkyl ; cyano-($C_1$-$C_6$) alkyl ; ($C_1$-$C_6$) alkoxy ; halo-($C_1$-$C_6$) alkoxy ; ($C_1$-$C_6$) alkoxy-($C_1$-$C_6$) alkyl having independently the stated number of carbon atoms in each alkyl group ; ($C_1$-$C_6$) alkoxy-($C_1$-$C_6$) alkoxy having independently the stated number of carbon atoms in each alkyl group ; $-OCO_2R$ group ; ($C_2$-$C_6$) alkenyl optionally substituted with halo, cyano, ($C_1$-$C_4$) alkyl, ($C_1$-$C_4$) alkoxy, halo-($C_1$-$C_4$) alkoxy or ($C_1$-$C_4$) alkylthio ; carboxy ; $-RCO_2R'$ group ; –COR ; halo-($C_1$-$C_6$) alkyl-carbonyl ; cyano-($C_1$-$C_6$) alkyl-carbonyl ; nitro-($C_1$-$C_6$) alkyl-carbonyl ; ($C_1$-$C_6$) alkoxy-carbonyl ; halo-($C_1$-$C_6$) alkoxy-carbonyl ; –OCOR group ; –NRR′ group ; amino substituted with hydroxy, ($C_1$-$C_4$) alkoxy or ($C_1$-$C_4$) alkylthio groups ; phenylamino ; diphenylamino ; –CONRR′ group ; –OCONRR′ group ; –NRCOR′ group ; $-NRCO_2R'$ group ; –N(COR)COR′ group ; –CONRCOR′ group ; sulfhydryl ; halothio ; ($C_1$-$C_6$) alkylthio ; halo-($C_1$-$C_6$) alkylthio ; –SOR group ; $-SO_2R$ group ; phenylsulfonyl ; $-OSO_2R$ group ; halo-($C_1$-$C_6$) alkylsulfonyloxy ; $-SO_2NRR'$ group ; –NRSOR′ group ; $-NRSO_2R'$ group ; –CSR group ; $CS_2R$ group ; –NRCSR′ group ; –SCOR group ; unsubstituted or substituted phenyl having one to three of the same or different halo, cyano, nitro, hydroxy ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; phenoxy where the phenyl ring is unsubstituted or substituted with one to three of the same or different halo, cyano, nitro, hydroxy, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; phenylthio where the phenyl ring is unsubstituted or substituted with one to three of the same or different halo, cyano, nitro, hydroxy, ($C_1$-$C_4$)alkyl, ($C_1$-$C_4$) alkoxy, carboxy, ($C_1$-$C_4$) alkoxy-carbonyl, ($C_1$-$C_4$) alkanoyloxy, $NH_2$, NHZ or -NZZ′ ; or when two adjacent positions on the phenyl ring are substituted with alkoxy groups these groups may be joined to form, together with the carbon atoms to which they are both attached, a 5- or 6-membered dioxolano or dioxano heterocyclic ring ;

B is unsubstituted or substituted ($C_1$-$C_{10}$) alkyl having as the optional substituent(s) one to four of the same or different halo, cyano, nitro, hydroxy, ($C_1$-$C_4$) alkoxy, halo-($C_1$-$C_4$) alkoxy, carboxy, ($C_1$-$C_4$) alkoxycarbonyl, ($C_1$-$C_4$) alkanoyloxy, phenyl, $NH_2$, NHZ or –NZZ′.

unsubstituted or substituted ($C_3$-$C_8$) cycloalkyl or unsubstituted or substituted ($C_3$-$C_8$) cycloalkyl ($C_1$-$C_4$) alkyl, having as the optional substituent(s) one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkanoyl, ($C_1$-$C_4$) alkoxy-carbonyl, ($C_1$-$C_4$) alkanoyloxy, phenyl, $NH_2$, NHZ or –NZZ′ ;

unsubstituted or substituted ($C_2$-$C_8$) alkenyl or unsubstituted or substituted ($C_3$-$C_8$) alkadienyl having, as the optional substituent(s), a furyl, thienyl or pyridyl group or one to four of the same or different halo, cyano, nitro, hydroxy, ($C_1$-$C_4$) alkyl, ($C_3$-$C_6$) cycloalkyl, halo-($C_1$-$C_4$) alkyl, ($C_1$-$C_4$) alkoxy, halo($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ;

unsubstituted or substituted ($C_3$-$C_8$) cycloalkenyl or unsubstituted or substituted ($C_6$-$C_8$) cycloalkadienyl having as the optional substituent(s), one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$ -$C_4$) alkoxy-carbonyl, ($C_1$-$C_4$) alkanoyloxy, $NH_2$, NHZ or –NZZ′ ;

unsubstituted or substituted ($C_2$-$C_8$) alkynyl having, as optional substituent(s), one to four of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, phenyl, $NH_2$, NHZ or –NZZ′ ;

phenalkyl having one to four carbon atoms in the alkyl group and wherein the alkyl group is unsubstituted or substituted with one to three of the same or different halo, cyano, hydroxy, ($C_1$-$C_4$) alkyl, ($C_1$-$C_4$) alkoxy, ($C_1$-$C_4$) alkoxy-carbonyl, $NH_2$, NHZ or –NZZ′, and the phenyl ring is unsubstituted or substituted with one two three of the same or different halo, cyano, nitro, hydroxy, ($C_1$-$C_4$) alkyl, halo-($C_1$-$C_4$) alkyl, cyano-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy, ($C_2$-$C_6$) alkenyl, halo-($C_2$-$C_6$) alkenyl, ($C_2$-$C_6$) alkynyl, $NH_2$, NHZ or –NZZ′ ; or

phenalkenyl having two to six carbon atoms in the alkenyl group and the alkenyl group is unsubstituted or substituted with one to three of the same or different halo, cyano, hydroxy, ($C_1$-$C_4$) alkyl, halo-($C_1$-$C_4$) alkyl, ($C_1$-$C_4$) alkoxy, halo-($C_1$-$C_4$)alkoxy, ($C_1$-$C_4$) alkoxy-carbonyl, $NN_2$, NHZ or –NZZ′, and the phenyl ring is unsubstituted or substituted with one to three of the same or different halo, cyano, nitro, hydroxy, ($C_1$-$C_4$) alkyl, halo-($C_1$-$C_4$) alkyl, ($C_1$-$C_4$) alkoxy, halo-($C_1$-$C_4$) alkoxy, carboxy, ($C_1$-$C_4$) alkoxy-carbonyl, ($C_1$-$C_4$) alkanoyloxy or $NH_2$, NHZ or –NZZ′ ;

where R and R′ are hydrogen or ($C_1$-$C_6$) alkyl ; Z and Z′ are ($C_1$-$C_4$) alkyl ; "amino" means NRR′ ; or an agronomically acceptable salt thereof ; except for such a compound in which X and X′ are O, $R^1$ is t-butyl, A is unsubstituted phenyl and B is unsubstituted methyl or unsubstituted ethyl.

together with agronomically acceptable diluent or carrier to make an insecticidal composition.

2. The use according to claim 1 of the compound wherein

X and X′ are O or S ; and/or

$R^1$ is unsubstituted branched ($C_3$-$C_8$) alkyl or a straight chain ($C_1$-$C_4$)alkyl substituted with one or two of the same or different ($C_3$-$C_4$) cycloalkyl ; and/or

A is unsubstituted naphthyl ; or

unsubstituted or substituted phenyl where the substituents can be from one to three of the same or different halo ; nitro, cyano ; ($C_1$-$C_4$) alkyl ; halo-($C_1$-$C_4$) alkyl ; cyano-($C_1$-$C_4$) alkyl, ($C_1$-$C_4$) alkoxy ; ($C_1$-$C_4$) alkoxy-($C_1$-$C_4$) alkyl ; –COD ; carboxy ; ($C_1$-$C_4$) alkoxy-carbonyl ; ($C_1$-$C_4$) alkanoyloxy ; $NH_2$, NHZ or –NZZ′ ; ($C_1$-$C_4$) alkylthio ; –CSD ; –$CS_2D$ ; –SCOD ; unsubstituted or substituted phenyl having one to two of the same or different halo, nitro, ($C_1$-$C_4$) alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; or when two adjacent positions on the phenyl ring are substituted with alkoxy groups, these group are joined to form, together with the carbon atoms to which they are attached, a 5- or 6-membered dioxolano or dioxano heterocyclic ring ; and/or

B is unsubstituted or substituted ($C_1$-$C_8$) alkyl having as the optional substituent(s) one to three of the same or different halo, cyano, ($C_1$-$C_4$) alkoxy, phenyl, ($C_1$-$C_4$) alkoxy-carbonyl or halo-($C_1$-$C_4$) alkoxy ;

unsubstituted or substituted ($C_3$-$C_6$) cycloalkyl or unsubstituted or substituted ($C_3$-$C_5$) cycloalkyl-($C_1$-$C_4$) alkyl, having as the optional substituent(s) one or two of the same or different halo, ($C_1$-$C_4$) alkyl, halo-($C_1$-$C_4$) alkyl, ($C_1$-$C_4$) alkoxy, ($C_1$-$C_4$) alkanoyl or ($C_1$-$C_4$) haloalkoxy ;

unsubstituted or substituted ($C_2$-$C_6$) alkenyl having as the optional substituent(s) a furyl or one to three of the same or different ($C_1$-$C_4$) alkyl, halo-($C_1$-$C_4$) alkyl, ($C_1$-$C_4$) alkoxy or halo-($C_1$-$C_4$) alkoxy ;

unsubstituted or substituted ($C_3$-$C_8$) cycloalkenyl having as the optional substituent(s) one or two of the same or different halo, ($C_1$-$C_4$) alkyl, halo-($C_1$-$C_4$) alkyl, ($C_1$-$C_4$) alkoxy or halo-($C_1$-$C_4$) alkoxy ;

unsubstituted or phenyl substituted alkynyl ;

phenalkyl having one or two carbon atoms in the alkyl group and the alkyl group is unsubstituted or substituted with one or two of the same or different halo, ($C_1$-$C_4$) alkyl, halo-($C_1$-$C_4$) alkyl, ($C_1$-$C_4$) alkoxy or halo-($C_1$-$C_4$) alkoxy and the phenyl ring is unsubstituted or substituted with one or two of the same or different halo, ($C_1$-$C_4$) alkyl, halo-($C_1$-$C_4$) alkyl, ($C_1$-$C_4$) alkoxy or halo($C_1$-$C_4$) alkoxy ; or

phenalkenyl having two or three carbon atoms in the alkenyl group and the alkenyl group is unsubstituted or substituted with halo, ($C_1$-$C_4$) alkyl, halo-($C_1$-$C_4$) alkyl, ($C_1$-$C_4$) alkoxy or halo-($C_1$-$C_4$) alkoxy and the phenyl ring is unsubstituted or substituted with one or two of the same or different halo, ($C_1$-$C_4$) alkyl, halo-($C_1$-$C_4$) alkyl, ($C_1$-$C_4$) alkoxy or halo-($C_1$-$C_4$) alkoxy ; or an

agronomically acceptable salt thereof ; wherein D is hydrogen or ($C_1$-$C_4$) alkyl and Z and Z′ are ($C_1$-$C_4$) alkyl.

3. The use according to claim 2 of a compound wherein

X and X′ are O or S ; and/or

$R^1$ is branched ($C_3$-$C_8$) alkyl ; and/or

A is unsubstituted naphthyl ; or unsubstituted or substituted phenyl having one to three of the same or different halo, nitro, cyano, ($C_1$-$C_4$) alkyl, halo-($C_1$-$C_4$) alkyl, cyano-($C_1$-$C_4$) alkyl, ($C_1$-$C_4$) alkoxy, –COD, ($C_1$-$C_4$) alkoxy-carbonyl, ($C_1$-$C_4$) alkanoyloxy, or

unsubstituted or substituted phenyl having one or two of the same or different halo, nitro, ($C_1$-$C_4$)alkyl, ($C_1$-$C_4$) alkoxy, carboxy, ($C_1$-$C_4$) alkoxy-carbonyl, ($C_1$-$C_4$) alkanoyloxy, $NH_2$, NHZ or –NZZ′ ; and/or

B is unsubstituted or substituted ($C_1$-$C_6$) alkyl having one to three of the same or different halo, cyano, ($C_1$-$C_4$) alkoxy, phenyl, ($C_1$-$C_3$) alkoxy-carbonyl or halo-($C_1$-$C_4$) alkoxy ;

unsubstituted or substituted ($C_3$-$C_6$) cycloalkyl or unsubstituted or substituted ($C_3$-$C_6$) cycloalkyl-($C_1$-$C_4$) alkyl where the optional substituent is halo, ($C_1$-$C_4$) alkyl, ($C_1$-$C_4$) alkanoyl, ($C_1$-$C_4$) alkoxy or halo-($C_1$-$C_4$) alkoxy ;

unsubstituted or substituted ($C_2$-$C_6$) alkenyl having one or two of the same or different ($C_1$-$C_4$) alkyl, ($C_1$-$C_4$) alkoxy or halo-($C_1$-$C_4$) alkoxy ;

unsubstituted or substituted ($C_4$-$C_6$) cycloalkenyl where the substituent is halo, ($C_1$-$C_4$) alkyl, ($C_1$-$C_4$) alkoxy or halo-($C_1$-$C_4$) alkoxy ; or

unsubstituted or phenyl-substituted alkynyl ; or an

agronomically acceptable salt thereof.

4. The use according to claim 3 of a compound wherein

X and X' are O ; and/or

$R^1$ is branched ($C_4$-$C_7$) alkyl ; and/or

A is unsubstituted, monosubstituted, 2,3-disubstituted or 2,4-disubstituted phenyl where the substituents can be the same or different halo, ($C_1$-$C_4$) alkyl, ($C_1$-$C_4$) alkoxy, or halo-($C_1$-$C_4$) alkyl ; and/or

B is unsubstituted or substituted ($C_1$-$C_6$) alkyl having one to three of the same or different halo, phenyl or cyano ;

unsubstituted ($C_4$-$C_6$) cycloalkyl ;

unsubstituted or substituted ($C_2$-$C_5$) alkenyl having as the optional substituent(s) one or two of the same or different ($C_1$-$C_4$) alkyl ;

unsubstituted ($C_4$-$C_6$) cycloalkenyl ; or

phenalkyl having one or two carbon atoms in alkyl group and the phenyl ring is unsubstituted or substituted with one or two of the same or different halo, ($C_1$-$C_4$) alkyl, or ($C_1$-$C_4$) alkoxy ;

or an agronomically acceptable salt thereof,

5. The use according to claim 4 of the compound wherein

X and X' are O ; and/or

$R^1$ is t-butyl, neopentyl (2,2-dimethylpropyl) or 1,2,2-trimethylpropyl ; and/or

A is unsubstituted or monosubstituted phenyl where the substituent can be chloro, fluoro, bromo, iodo, methyl, ethyl, methoxy or trifluoromethyl ; and/or

B is unsubstituted or substituted ($C_1$-$C_4$) alkyl having as the optional substituent(s) one to three of the same or different, phenyl, chloro, fluoro or bromo ;

unsubstituted ($C_4$-$C_6$) cycloalkyl ;

unsubstituted ($C_4$-$C_8$) cycloalkenyl ; or

phenalkyl having one to two carbon atoms in the alkyl group and the phenyl ring is unsubstituted or substituted with one or two of the same or different chloro, fluoro, bromo, iodo, methyl, ethyl, methoxy or trifluoromethyl ; or an agronomically acceptable salt thereof.

6. The use according to claim 3 of the compound wherein

X and X' are O ;

$R^1$ is t-butyl ; and

A is phenyl and B is cyclohexyl, benzyl, 1-cyclohexenyl, 2-methylcyclohexadi-2,5-enyl, phenylethynyl, 1-methylpropyl, 2-methyl-1-phenylbutyl, 3-acetyl-2,2-dimethylcyclobutylmethyl, 1-butenyl, 3-butenyl, 3-carbomethoxypropyl or methoxymethyl ; or

A is 3,5-dichlorophenyl and B is 2-bromoethyl ; or

A is 4-chlorophenyl and B is cyclobutyl ; or

A is 2,3-dimethylphenyl and B is 1-methylethenyl or 1-ethylethenyl ; or

A is 4-ethylphenyl and B is 1-methylethenyl.

7. The use according to any preceding claim of the compound or salt in an amount of from 0.0001. preferably 0.001, to 99% by weight of the composition.

8. The use according to claim 6 of the compound or salt in an amount of from 0,01 to 99% by weight of the composition.

9. The use according to any of claims 6 to 8 of the compound or salt to make a composition in the form of a wettable powder, a flowable, a dust, a granule, a bait or an emulsifiable concentrate,

10. The use of a compound or salt as defined in any of claims 1 to 6, optionally in a composition also containing agronomically acceptable diluent or carrier, for controlling insects, by contacting said insects with an insecticidally effective amount of said compound or salt.

11. A mechanical process for improving the commercial value and/or profitability of vendible crops from plants whose growth is affected or likely to be affected by insects comprising (1) charging to a container, fumigation device or mechanical dissemination device an insecticidal compound or salt as defined in any of claims 1 to 6, optionally in a mixture with agronomically acceptable diluent or carrier, (2) using the container, fumigator or mechanical dissemination device to apply the insecticidal compound or salt, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to

be grown in the treated area.

12. The use or process according to claim 10 or 11 which comprises applying the compound or salt at a rate of from about 10 grams to about 10 kilograms per hectare, to growing plants or to an area where plants are to be grown.

13. The use or process according to claim 12 wherein the rate of application is 100 grams to 5 kilograms of the compound per hectaré.

14. The use or process according to claim 10 or 11 for controlling insects from the order Lepidoptera or Coleoptera.

## Ansprüche

**Patentansprüche für die benannten Vertragsstaaten BE, CH, DE, FR, GB, GR, IT, LI, LU, NL, SE.**

1. Insektizid wirkende Verbindung der Formel

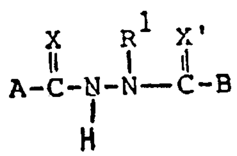

(I),

wobei

X und X′ gleich oder unterschiedlich die Bedeutung O, S oder NR haben ;

$R^1$ bedeutet :

unsubstituiertes verzweigtes ($C_3$-$C_{10}$)-Alkyl oder geradkettiges ($C_1$-$C_4$)-Alkyl, gleich oder unterschiedlich substituiert mit einem oder zwei ($C_3$-$C_6$)-Cycloalkyl ;

A bedeutet :

unsubstituiertes oder substituiertes Naphthyl, wobei die Substituenten gleich oder unterschiedlich eins bis drei aus Halo ; Cyano ; Nitro ; Hydroxy ; ($C_1$-$C_4$)-Alkoxy ; ($C_1$-$C_4$)-Alkyl ; Carboxy ; ($C_1$-$C_4$)-Alkoxy-Carbonyl ; ($C_1$-$C_4$)-Alkanoyloxy oder $NH_2$ ; NHZ oder –NZZ′ sein können ;

unsubstituiertes oder substituiertes Phenyl, wobei die Substituenten sein können gleich oder unterschiedlich eins bis fünf aus Halo ; Nitro ; Cyano ; Hydroxy ; ($C_1$-$C_6$)-Alkyl ; Halo-($C_1$-$C_6$)-alkyl ; Cyano-($C_1$-$C_6$)-alkyl ; ($C_1$-$C_6$)-Alkoxy ; Halo-($C_1$-$C_6$)-alkoxy ; ($C_1$-$C_6$)-Alkoxy-($C_1$-$C_6$)-alkyl mit unabhängig der angegebene Anzahl von Kohlenstoffatomen in jeder Alkylgruppe ; ($C_1$-$C_6$)-Alkoxy-($C_1$-$C_6$)-alkoxy mit unabhängig der angegebenen Anzahl an Kohlenstoffatomen in jeder Alkylgruppe ; –$OCO_2R$-Gruppe ; ($C_2$-$C_6$)-Alkenyl, wahlweise substituiert mit Halo, Cyano, ($C_1$-$C_4$)-Alkyl, ($C_1$-$C_4$)-Alkoxy, Halo-($C_1$-$C_4$)-alkoxy oder ($C_1$-$C_4$)-Alkylthio ; Carboxy ; –$RCO_2R'$-Gruppe ; –COR ; Halo-($C_1$-$C_6$)-alkylcarbonyl ; Cyano-($C_1$-$C_6$)-alkylcarbonyl ; Nitro-($C_1$-$C_6$)-alkyl-carbonyl ; ($C_1$-$C_6$)-Alkoxycarbonyl ; Halo-($C_1$-$C_6$)-alkoxycarbonyl ; –OCOR-Gruppe ; –NRR′-Gruppe ; Amino, substituiert mit Hydroxy-, ($C_1$-$C_4$)-Alkoxy- oder ($C_1$-$C_4$)-Alkylthio-Gruppen ; Phenylamino ; Diphenylamino ; –CONRR′-Gruppe ; –OCONRR′-Gruppe ; –NRCOR′-Gruppe ; –$NRCO_2R'$-Gruppe ; –N(COR)COR′-Gruppe ; –OCONRCOR′-Gruppe ; Sulfhydryl ; Halothio ; ($C_1$-$C_6$)-Alkylthio ; Halo-($C_1C_6$)-alkylthio ; –SOR-Gruppe ; –$SO_2R$-Gruppe ; Phenylsulfonyl ; –$OSO_2R$-Gruppe ; Halo-($C_1$-$C_6$)-alkylsulfonyloxy ; –$SO_2NRR'$-Gruppe ; –NRSOR′-Gruppe ; –$NRSO_2R'$-Gruppe, –CSR-Gruppe ; $CS_2R$-Gruppe ; –NRCSR′-Gruppe ; –SCOR-Gruppe ; unsubstituiertes oder substituiertes Phenyl mit gleich oder unterschiedlich eins bis drei aus Halo, Cyano, Nitro, Hydroxy($C_1$-$C_4$)-alkyl, ($C_1$-$C_4$)-Alkoxy, Carboxy, ($C_1$-$C_4$)-Alkoxycarbonyl, ($C_1$-$C_4$)-Alkanyloxy, $NH_2$, NHZ oder –NZZ′ ; Phenoxy, wobei der Phenylring unsubstiutiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder –NZZ′ ; Phenylthio, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder –NZZ′ ; oder, wenn zwei benachbarte Positionen am Phenylring mit Alkoxygruppen substituiert sind, diese Gruppen verbunden sein können, um zusammen mit den Kohlenstoffatomen, mit denen sie beide verbunden sind, einen 5- oder 6-gliedrigen heterocyclischen Dioxolan- oder Dioxanring zu bilden sind ;

B bedeutet :

unsubstituiertes oder substituiertes ($C_1$-$C_{10}$)-Alkyl, welches als wahlweise(n) Substituenten gleich oder unterschiedlich eins bis vier aus Halo, Cyano, Nitro, Hydroxy, ($C_1$-$C_4$)-Alkoxy, Halo-($C_1$-$C_4$)-alkoxy, Carboxy,

$(C_1-C_4)$-Alkoxycarbonyl, $(C_1-C_4)$-Alkanoyloxy, Phenyl, $NH_2$, NHZ oder –NZZ′ aufweist ;

unsubstituiertes oder substituiertes $(C_3-C_8)$-Cycloalkyl oder unsubstituiertes oder substituiertes $(C_3-C_8)$-Cycloalkyl$(C_1-C_4)$-Alkyl, welche als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkanoyl, $(C_1-C_4)$-Alkoxycarbonyl, $(C_1-C_4)$-Alkanoyloxy, Phenyl, $NH_2$, NHZ oder –NZZ′ aufweisen ;

unsubstituiertes oder substituiertes $(C_2-C_8)$-Alkenyl oder unsubstituiertes oder substituiertes $(C_3-C_8)$-Alkadienyl, welches als wahlweise(n) Substituenten eine Furyl-, Thienyl-, oder Pyridyl-Gruppe aufweist oder gleich oder unterschiedlich von eins bis vier aus Halo, Cyano, Nitro, Hydroxy, $(C_1-C_4)$-Alkyl, $(C_3-C_6)$-Cycloalkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $NH_2$, NHZ oder –NZZ′ ;

unsubstituiertes oder substituiertes $(C_3-C_8)$-Cycloalkenyl oder unsubstituiertes oder substituiertes $(C_6-C_8)$-Cycloalkadienyl, welche als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $NH_2$, NHZ oder –NZZ′ aufweisen ;

unsubstituiertes oder substituiertes $(C_2-C_8)$-Alkynyl, welches als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-Alkoxy, Carboxy, $(C_1-C_4)$-Alkoxycarbonyl, $(C_1-C_4)$-Alkanoyloxy, Phenyl, $NH_2$, NHZ oder –NZZ′ aufweisen ;

Phenalkyl mit einem bis vier Kohlenstoffatomen in der Alkylgruppe, und wobei die Alkylgruppe unsubstituiert ist oder gleich oder unterschiedlich substituiert ist mit eins bis drei aus Halo, Cyano, Hydroxy, $(C_1-C_4)$-Alkyl, $(C_1-C_4)$-Alkoxy, $(C_1-C_4)$-Alkoxycarbonyl, $NH_2$, NHZ oder NZZ′, und wobei der Phenylring unsubstituiert ist oder gleich oder unterschiedlich substituiert ist mit eins bis drei aus Halo, Cyano, Nitro, Hydroxy $(C_1-C_4)$-Alkyl, Halo-$(C_1-C_4)$-alkyl, Cyano-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $(C_2-C_6)$-Alkenyl, Halo-$(C_2-C_6)$-alkenyl, $(C_2-C_6)$-Alkynyl, $NH_2$, NHZ oder –NZZ′ ; oder

Phenalkyl mit zwei bis sechs Kohlenstoffatomen in der Alkylgruppe, wobei die Alkylgruppe unsubstituiert ist oder gleich oder unterschiedlich substituiert ist mit eins bis drei aus Halo, Cyano, Hydroxy, $(C_1-C_4)$-Alkyl, Halo-$(C_1-C_4)$-alkyl, $(C_1-C_4)$-Alkoxy, Halo-$(C_1-C_4)$-alkoxy, $(C_1-C_4)$-Alkoxycarbonyl, $NH_2$, NHZ oder –NZZ′, und wobei der Phenylring unsubstituiert ist oder gleich oder unterschiedlich substituiert ist mit einem bis drei aus Halo, Cyano, Nitro, Hydroxy $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, oder $NH_2$, NHZ oder NZZ′ ;

wobei R und R′ die Bedeutung Wasserstoff oder $(C_1-C_6)$-Alkyl haben ; Z und Z′ die Bedeutung $(C_1-C_4)$-Alkyl haben ; wobei "Amino" die Bedeutung NRR′ hat ; oder ein landwirtschaftlich vertretbares Salz davon ; mit der Ausnahme solcher Verbindungen, in denen X und X′ die Bedeutung O haben, $R^1$ die Bedeutung t-Butyl hat, A die Bedeutung unsubstituiertes Phenyl hat und B die Bedeutung unsubstituiertes Methyl oder unsubstituiertes Ethyl hat.

2. Verbindung nach Anspruch 1, wobei

X und X′ die Bedeutung O oder S haben ; und/oder

$R^1$ bedeutet :

unsubstituiertes verzweigtes $(C_3-C_8)$-Alkyl oder geradkettiges $(C_1-C_4)$-Alkyl, gleich oder unterschiedlich substituiert mit eins oder zwei aus $(C_3-C_4)$-Cycloalkyl ; und/oder

A bedeutet :

unsubstituiertes Naphthyl ; oder

unsubstituiertes oder substituiertes Phenyl, wobei die Substituenten gleich oder unterschiedlich sein können eins bis drei aus Halo ; Nitro ; Cyano ; $(C_1-C_4)$-Alkyl ; Halo-$(C_1-C_4)$-alkyl ; Cyano-$(C_1-C_4)$-alkyl ; $(C_1-C_4)$-Alkoxy, $(C_1-C_4)$-Alkoxy-$(C_1-C_4)$-alkyl ; –COD ; Carboxy ; $(C_1-C_4)$-Alkoxycarbonyl ; $(C_1-C_4)$-Alkanoyloxy ; $NH_2$ ; NHZ oder –NZZ′ ; $(C_1-C_4)$-Alkylthio ; –CSD ; –$CS_2$D ; –SCOD ; unsubstituiertes oder substituiertes Phenyl mit gleich oder unterschiedlich eins bis zwei aus Halo, Nitro, $(C_1-C_4)$-Alkyl, $(C_1-C_4)$-Alkoxy, Carboxy, $(C_1-C_4)$-Alkoxycarbonyl, $(C_1-C_4)$-Alkanoyloxy, $NH_2$, NHZ oder –NZZ′ ; oder wenn zwei benachbarte Positionen am Phenylring mit Alkoxy-Gruppen substituiert sind, sind diese Gruppen verbunden, um zusammen mit den Kohlenstoffatomen, an denen sie hängen, einen 5- oder 6-gliedrigen heterocyclischen Dioxolan- oder Dioxanring zu bilden ; und/oder

B bedeutet :

unsubstituiertes oder substituiertes $(C_1-C_8)$-Alkyl, welches als wahlweise(n) Substituenten gleich oder unterschiedlich eins bis drei aus Halo, Cyano, $(C_1-C_4)$-Alkoxy, Phenyl, $(C_1-C_4)$-Alkoxycarbonyl oder Halo-$(C_1-C_4)$-alkoxy aufweist ;

unsubstituiertes oder substituiertes $(C_3-C_6)$-Cycloalkyl oder unsubstituiertes oder substituiertes $(C_3-C_5)$-Cycloalkyl-$(C_1-C_4)$-alkyl, welche als wahlweise(n) Substituenten gleich oder unterschiedlich eins oder zwei aus

Halo, $(C_1-C_4)$-Alkyl, Halo-$(C_1-C_4)$-alkyl, $(C_1-C_4)$-Alkoxy, $(C_1-C_4)$-Alkanoyl oder $(C_1-C_4)$-Haloalkoxy aufweist ;

unsubstituiertes oder substituiertes $(C_2-C_6)$-Alkenyl, welches als wahlweise(n) Substituenten ein Furyl oder gleich oder unterschiedlich eins bis drei aus $(C_1-C_4)$-Alkyl, Halo-$(C_1-C_4)$-alkyl, $(C_1-C_4)$-Alkoxy oder Halo-$(C_1-C_4)$-alkoxy aufweist ;

unsubstituiertes oder substituiertes $(C_3-C_8)$-Cycloalkenyl, welches als wahlweise Substituenten gleich oder unterschiedlich eins oder zwei aus Halo, $(C_1-C_4)$-Alkyl, Halo-$(C_1-C_4)$-alkyl, $(C_1-C_4)$-Alkoxy oder Halo-$(C_1-C_4)$-alkoxy aufweist ;

unsubstituiertes oder substituiertes Alkynyl ;

Phenalkyl mit einem oder zwei Kohlenstoffatomen in der Alkylgruppe, wobei die Alkygruppe unsubstituiert ist oder gleich oder unterschiedlich substituiert ist mit eins oder zwei aus Halo, $(C_1-C_4)$-Alkyl, Halo-$(C_1-C_4)$-alkyl, $(C_1-C_4)$-Alkoxy oder Halo-$(C_1-C_4)$-alkoxy, und wobei der Phenylring unsubstituiert ist oder gleich oder unterschiedlich substituiert ist mit eins oder zwei aus Halo, $(C_1-C_4)$-Alkyl, Halo-$(C_1-C_4)$-alkyl, $(C_1-C_4)$-Alkoxy oder Halo-$(C_1-C_4)$-alkoxy ; oder ;

Phenalkenyl mit zwei oder drei Kohlenstoffatomen in der Alkenylgruppe, wobei die Alkenylgruppe unsubstituiert ist oder substituiert ist mit Halo, $(C_1-C_4)$-Alkyl, Halo-$(C_1-C_4)$-alkyl, $(C_1-C_4)$-Alkoxy oder Halo-$(C_1-C_4)$-alkoxy, und wobei der Phenylring unsubstituiert ist oder ist gleich oder unterschiedlich substituiert ist mit eins oder zwei aus Halo, $(C_1-C_4)$-Alkyl, Halo-$(C_1-C_4)$-alkyl, $(C_1-C_4)$-Alkoxy oder Halo-$(C_1-C_4)$-alkoxy ; oder ein

landwirtschaftlich vertretbares Salz davon ; wobei D die Bedeutung Wasserstoff oder $(C_1-C_4)$-Alkyl hat und Z und Z' die Bedeutung $(C_1-C_4)$-Alkyl hat.

3. Verbindung nach Anspruch 2, wobei

X und X' die Bedeutung O oder S haben ; und/oder

$R^1$ verzweigtes $(C_3-C_8)$-Alkyl ist ; und/oder

A bedeutet :

unsubstituiertes oder substituiertes Phenyl mit gleich oder unterschiedlich eins bis drei aus Halo, Nitro, Cyano, $(C_1-C_4)$-Alkyl, Halo-$(C_1-C_4)$-alkyl, Cyano-$(C_1-C_4)$-alkyl, $(C_1-C_4)$-Alkoxy, –COD, $(C_1-C_4)$-Alkoxycarbonyl, $(C_1-C_4)$-Alkanoyloxy oder unsubstituiertes oder substituiertes Phenyl mit gleich oder unterschiedlich eins oder zwei aus Halo, Nitro, $(C_1-C_4)$-Alkyl, $(C_1-C_4)$-Alkoxy, Carboxy, $(C_1-C_4)$-Alkoxycarbonyl, $(C_1-C_4)$-Alkanoyloxy, $NH_2$ ; NHZ oder –NZZ' ; und/oder

B bedeutet :

unsubstituiertes oder substituiertes $(C_1-C_6)$-Alkyl mit gleich oder unterschiedlich eins bis drei aus Halo, Cyano, $(C_1-C_4)$-Alkoxy, Phenyl, $(C_1-C_3)$-Alkoxycarbonyl oder Halo-$(C_1-C_4)$-alkoxy ;

unsubstituiertes oder substituiertes $(C_3-C_6)$-Cycloalkyl oder unsubstituiertes oder substituiertes $(C_3-C_6)$-Cycloalkyl-$(C_1-C_4)$-alkyl, wobei der wahlweise Substituent Halo, $(C_1-C_4)$-Alkyl, $(C_1-C_4)$-Alkanoyl, $(C_1-C_4)$-Alkoxy oder Halo-$(C_1-C_4)$-alkoxy ist ; unsubstituiertes oder substituiertes $(C_2-C_6)$-Alkenyl mit gleich oder unterschiedlich eins oder zwei aus $(C_1-C_4)$-Alkyl, $(C_1-C_4)$-Alkoxy oder Halo$(C_1-C_4)$-alkoxy ;

unsubstituiertes oder substituiertes $(C_4-C_6)$-Cycloalkenyl, wobei der Substituent Halo, $(C_1-C_4)$-Alkyl, $(C_1-C_4)$-Alkoxy oder Halo-$(C_1-C_4)$-alkoxy ist ; oder

unsubstituiertes oder phenylsubstituiertes Alkynyl ; oder ein

landwirtschaftlich vertretbares Salz davon.

4. Verbindung nach Anspruch 3, wobei

X und X' die Bedeutung O haben ; und/oder

$R^1$ die Bedeutung verzweigtes $(C_4-C_7)$-Alkyl hat ; und/oder

A bedeutet :

unsubstituiertes, monosubstituiertes, 2,3-disubstituiertes oder 2,4-disubstituiertes Phenyl, wobei die Substituenten gleich oder unterschiedlich Halo-$(C_1-C_4)$-alkyl, $(C_1-C_4)$-Alkoxy oder Halo-$(C_1-C_4)$-alkyl sein können; und/oder

B bedeutet :

unsubstituiertes oder substituiertes $(C_1-C_6)$-Alkyl mit gleich oder unterschiedlich eins bis drei aus Halo, Phenyl oder Cyano ;

unsubstituiertes $(C_4-C_6)$-Cycloalkyl ;

unsubstituiertes oder substituiertes $(C_2-C_5)$-Alkenyl, wobei die wahlweise(n) Substituenten gleich oder unterschiedlich eins oder zwei $(C_1-C_4)$-Alkyl sind ;

unsubstituiertes $(C_4-C_6)$-Cycloalkenyl ; oder

Phenalkyl mit einem oder zwei Kohlenstoffatomen in der Alkylgruppe, und wobei der Phenylring unsubstituiert ist gleich oder unterschiedlich substituiert ist mit eins oder zwei aus Halo, $(C_1-C_4)$-Alkyl oder $(C_1-C_4)$-Alkoxy ; oder ein landwirtschaftlich vertretbares Salz davon.

5. Verbindung nach Anspruch 4, wobei

X und X' die Bedeutung O haben ; und/oder

$R^1$ die Bedeutung t-Butyl, Neopentyl (2,2-Dimethylpropyl) oder 1,2,2-Trimethylpropyl hat ; und/oder
A unsubstituiertes oder monosubstituiertes Phenyl bedeutet, wobei der Substituent Chlor, Fluor, Brom, Iod, Methyl, Ethyl, Methoxy oder Trifluormethyl sein kann ; und/oder
B bedeutet :
unsubstituiertes oder substituiertes ($C_1$-$C_4$)-Alkyl, welches als wahlweisen Substituenten gleich oder unterschiedlich eins oder drei aus Phenyl, Chlor, Fluor oder Brom aufweist ;
unsubstituiertes ($C_4$-$C_6$)-Cycloalkyl ;
unsubstituiertes ($C_4$-$C_6$)-Cycloalkenyl ; oder
Phenalkyl mit einem bis zwei Kohlenstoffatomen in der Alkylgruppe, wobei der Phenylring unsubstituiert ist, oder gleich oder unterschiedlich substituiert ist mit eins oder zwei aus Chlor, Fluor, Brom, Iod, Methyl, Ethyl, Methoxy oder Trifluormethyl ; oder ein
landwirtschaftlich vertretbares Salz davon.
6. Verbindung nach Anspruch 3, wobei
X und X' die Bedeutung O haben ;
$R^1$ die Bedeutung t-Butyl hat ; und
A die Bedeutung Phenyl und B die Bedeutung Cyclohexyl, Benzyl, 1-Cyclohexenyl, 2-Methylcyclohexadi-2,5-enyl, Phenylethynyl, 1-Methylpropyl, 2-Methyl-1-phenylbutyl, 3-Acetyl-2,2-dimethylcyclobutyl-methyl, 1-Butenyl, 3-Butenyl, 3-Carbomethoxypropyl oder Methoxymethyl haben ; oder
A die Bedeutung 3,5-Dichlorphenyl und B die Bedeutung 2-Bromethyl haben ; oder
A die Bedeutung 4-Chlorphenyl und B die Bedeutung Cyclobutyl haben ; oder
A die Bedeutung 2,3-Dimethylphenyl und B die Bedeutung 1-Methylethenyl oder 1-Ethylethenyl haben ; oder
A die Bedeutung 4-Ethylphenyl und B die Bedeutung 1-Methylethenyl haben.
7. Insektizide Zusammensetzung, enthaltend einen landwirtschaftlich vertretbaren Verdünnungs- oder Trägerstoff und eine insektizide Verbindung wie in einer der Ansprüche 1 bis 6 beansprucht.
8. Zusammensetzung nach Anspruch 7, enthaltend die insektizide Verbindung in einer Menge von 0,0001, bevorzugt 0,001 bis 99 Gew.-%.
9. Zusammensetzung nach Anspruch 7, enthaltend die insektizide Verbindung in einer Menge von 0,01 bis 99 Gew.-%.
10. Zusammensetzung nach einem der Ansprüche 7 bis 9 in Form eines benetzbaren Pulvers, eines Fließfähigen, eines Staubes, eines Granulats, eines Köders oder eines emulgierbaren Konzentrats.
11. Verfahren zum Kontrollieren von Insekten, bei dem man die Insekten mit einer insektiziden Verbindung gemäß einem der Ansprüche 1 bis 6 in Kontakt bringt, wahlweise in einer Zusammensetzung nach einem der Ansprüche 7 bis 9.
12. Verfahren nach Anspruch 11, wobei man die Verbindung wachsenden Pflanzen oder einem Gebiet wo Pflanzen zu ziehen sind, in einer Menge von etwa 10 g bis 10 kg pro Hektar zusetzt.
13. Verfahren nach Anspruch 12, wobei die Applikationsrate 100 g bis 5 kg der Verbindung pro Hektar beträgt.
14. Verfahren nach Anspruch 13 zum Kontrollieren von Insekten der Ordnung Lepidoptera oder Coleoptera.

**Patentansprüche für die benannten Vertragsstaat AT**

1. Insektizid wirkende Zusammensetzung, enthaltend eine Verbindung der Formel

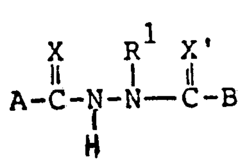

(I),

wobei
X und X' gleich oder unterschiedlich die Bedeutung O, S oder NR haben ;
$R^1$ bedeutet :
unsubstituiertes verzweigtes ($C_3$-$C_{10}$)-Alkyl oder geradkettiges ($C_1$-$C_4$)-Alkyl, gleich oder unterschiedlich substituiert mit einem oder zwei ($C_3$-$C_6$)-Cycloalkyl ;
A bedeutet :
unsubstituiertes oder substituiertes Naphthyl, wobei die Substituenten gleich oder unterschiedlich eins bis drei aus Halo ; Cyano ; Nitro ; Hydroxy ; ($C_1$-$C_4$)-Alkoxy ; ($C_1$-$C_4$)-Alkyl ; Carboxy ; ($C_1$-$C_4$)-Alkoxy-Carbonyl ;

$(C_1-C_4)$-Alkanoyloxy oder $NH_2$ ; NHZ oder –NZZ′ sein können ;

unsubstituiertes oder substituiertes Phenyl, wobei die Substituenten sein können gleich oder unterschiedlich eins bis fünf aus Halo ; Nitro ; Cyano ; Hydroxy ; $(C_1-C_6)$-Alkyl ; Halo-$(C_1-C_6)$-alkyl ; Cyano-$(C_1-C_6)$-alkyl ; $(C_1-C_6)$-Alkoxy ; Halo-$(C_1-C_6)$-alkoxy ; $(C_1-C_6)$-Alkoxy-$(C_1-C_6)$-alkyl mit unabhängig der angegebenee Anzahl von Kohlenstoffatomen in jeder Alkylgruppe ; $(C_1-C_6)$-Alkoxy-$(C_1-C_6)$-alkoxy mit unabhängig der angegebenen Anzahl an Kohlenstoffatomen in jeder Alkylgruppe ; $-OCO_2R$-Gruppe ; $(C_2-C_6)$-Alkenyl, wahlweise substituiert mit Halo, Cyano, $(C_1-C_4)$-Alkyl, $(C_1-C_4)$-Alkoxy, Halo-$(C_1-C_4)$-alkoxy oder $(C_1-C_4)$-Alkylthio ; Carboxy ; –$RCO_2R'$-Gruppe ; –COR ; Halo-$(C_1-C_6)$-alkylcarbonyl ; Cyano-$(C_1-C_6)$-alkylcarbonyl ; Nitro-$(C_1-C_6)$-alkylcarbonyl ; $(C_1-C_6)$-Alkoxycarbonyl ; Halo-$(C_1-C_6)$-alkoxycarbonyl ; –OCOR-Gruppe ; –NRR′-Gruppe ; Amino, substituiert mit Hydroxy-, $(C_1-C_4)$-Alkoxy- oder $(C_1-C_4)$-Alkylthio-Gruppen ; Phenylamino ; Diphenylamino ; –CONRR′-Gruppe ; –OCONRR′-Gruppe ; –NRCOR′Gruppe ; $-NRCO_2R'$-Gruppe ; –N(COR)COR′-Gruppe ; –OCONRCOR′-Gruppe ; Sulfhydryl ; Halothio ; $(C_1-C_6)$-Alkylthio ; Halo-$(C_1C_6)$-alkylthio ; –SOR-Gruppe ; $-SO_2R$-Gruppe ; Phenylsulfonyl ; $-OSO_2R$-Gruppe ; Halo-$(C_1-C_6)$-alkylsulfonyloxy ; $-SO_2NRR'$-Gruppe ; –NRSOR′-Gruppe ; $-NRSO_2R'$-Gruppe, –CSR-Gruppe ; $CS_2R$-Gruppe ; –NRCSR′-Gruppe ; –SCOR-Gruppe ; unsubstituiertes oder substituiertes Phenyl mit gleich oder unterschiedlich eins bis drei aus Halo, Cyano, Nitro, Hydroxy$(C_1-C_4)$-alkyl, $(C_1-C_4)$-Alkoxy, Carboxy, $(C_1-C_4)$-Alkoxycarbonyl, $(C_1-C_4)$-Alkanyloxy, $NH_2$, NHZ oder –NZZ′ ; Phenoxy, wobei der Phenylring unsubstuitiert ist oder gleich oder unterschiedlich substituiert ist mit eins bis drei aus Halo, Cyano, Nitro, Hydroxy, $(C_1-C_4)$-Alkyl, $(C_1-C_4)$-Alkoxy, Carboxy, $(C_1-C_4)$-Alkoxycarbonyl, $(C_1-C_4)$-Alkanoyloxy, $NH_2$, NHZ oder –NZZ′ ; Phenylthio, wobei der Phenylring unsubstituiert ist oder gleich oder unterschiedlich substituiert ist mit eins bis drei aus Halo, Cyano, Nitro, Hydroxy, $(C_1-C_4)$-Alkyl, $(C_1-C_4)$-Alkoxy, Carboxy, $(C_1-C_4)$-Alkoxycarbonyl, $(C_1-C_4)$-Alkanoyloxy, $NH_2$, NHZ oder –NZZ′ ; oder, wenn zwei benachbarte Positionen am Phenylring mit Alkoxygruppen substituiert sind, diese Gruppen verbunden sein können, um zusammen mit den Kohlenstoffatomen, mit denen sie beide verbunden sind, einen 5- oder 6-gliedrigen heterocyclischen Dioxolan- oder Dioxanring zu bilden sind ;

B bedeutet :

unsubstituiertes oder substituiertes $(C_1-C_{10})$-Alkyl, welches als wahlweise(n) Substituenten gleich oder unterschiedlich eins bis vier aus Halo, Cyano, Nitro, Hydroxy, $(C_1-C_4)$-Alkoxy, Halo$(C_1-C_4)$-alkoxy, Carboxy, $(C_1-C_4)$-Alkoxycarbonyl, $(C_1-C_4)$-Alkanoyloxy, Phenyl, $NH_2$, NHZ oder –NZZ′ aufweist ;

unsubstituiertes oder substituiertes $(C_3-C_8)$-Cycloalkyl oder unsubstituiertes oder substituiertes $(C_3-C_8)$-Cycloalkyl$(C_1-C_4)$-Alkyl, welche als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkanoyl, $(C_1-C_4)$-Alkoxycarbonyl, $(C_1-C_4)$-Alkanoyloxy, Phenyl, $NH_2$, NHZ oder –NZZ′ aufweisen ;

unsubstituiertes oder substituiertes $(C_2-C_8)$-Alkenyl oder unsubstituiertes oder substituiertes $(C_3-C_8)$-Alkadienyl, welches als wahlweise(n) Substituenten eine Furyl-, Thienyl-, oder Pyridyl-Gruppe aufweist oder gleich oder unterschiedlich von eins bis vier aus Halo, Cyano, Nitro, Hydroxy, $(C_1-C_4)$-Alkyl, $(C_3-C_6)$-Cycloalkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $NH_2$, NHZ oder –NZZ′ ;

unsubstituiertes oder substituiertes $(C_3-C_8)$-Cycloalkenyl oder unsubstituiertes oder substituiertes $(C_6-C_8)$-Cycloalkadienyl, welche als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $NH_2$, NHZ oder –NZZ′ aufweisen ;

unsubstituiertes oder substituiertes $(C_2-C_8)$-Alkynyl, welches als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-Alkoxy, Carboxy, $(C_1-C_4)$-Alkoxycarbonyl, $(C_1-C_4)$-Alkanoyloxy, Phenyl, $NH_2$, NHZ oder –NZZ′ aufweisen ;

Phenalkyl mit einem bis vier Kohlenstoffatomen in der Alkylgruppe, und wobei die Alkylgruppe unsubstituiert ist oder gleich oder unterschiedlich substituiert ist mit eins bis drei aus Halo, Cyano, Hydroxy, $(C_1-C_4)$-Alkyl, $(C_1-C_4)$-Alkoxy, $(C_1-C_4)$-Alkoxycarbonyl, $NH_2$, NHZ oder –NZZ′, und wobei der Phenylring unsubstituiert ist oder substituiert ist gleich oder unterschiedlich mit eins bis drei aus Halo, Cyano, Nitro, Hydroxy $(C_1-C_4)$-Alkyl, Halo-$(C_1-C_4)$-alkyl, Cyano-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $(C_2-C_6)$-Alkenyl, Halo-$(C_2-C_6)$-alkenyl, $(C_2-C_6)$-Alkynyl, $NH_2$, NHZ oder –NZZ′ ; oder

Phenalkyl mit zwei bis sechs Kohlenstoffatomen in der Alkylgruppe, wobei die Alkylgruppe unsubstituiert ist oder gleich oder unterschiedlich substituiert ist mit eins bis drei aus Halo, Cyano, Hydroxy, $(C_1-C_4)$-Alkyl, Halo-$(C_1-C_4)$-alkyl, $(C_1-C_4)$-Alkoxy, Halo-$(C_1-C_4)$-alkoxy, $(C_1-C_4)$-Alkoxycarbonyl, $NH_2$, NHZ oder –NZZ′, und wobei der Phenylring unsubstituiert ist oder gleich oder unterschiedlich substituiert ist mit einem bis drei aus Halo, Cyano, Nitro, Hydroxy $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, oder $NH_2$, NHZ oder –NZZ′ ;

wobei R und R′ die Bedeutung Wasserstoff oder ($C_1$-$C_6$)-Alkyl haben ; Z und Z′ die Bedeutung ($C_1$-$C_4$)-Alkyl haben ; wobei "Amino" die Bedeutung NRR′ hat ; oder

ein landwirtschaftlich vertretbares Salz davon ; mit der Ausnahme solcher Verbindungen, in denen X und X′ die Bedeutung O haben, $R^1$ die Bedeutung t-Butyl hat, A die Bedeutung unsubstituiertes Phenyl hat und B die Bedeutung unsubstituiertes Methyl oder unsubstituiertes Ethyl hat.
zusammen mit landwirtschaftlich vertretbaren Verdünnungs- oder Trägerstoffen.

2. Zusammensetzung nach Anspruch 1, wobei

X und X′ die Bedeutung O oder S haben ; und/oder

$R^1$ bedeutet :

unsubstituiertes verzweigtes ($C_3$-$C_8$)-Alkyl oder geradkettiges ($C_1$-$C_4$)-Alkyl, gleich oder unterschiedlich substituiert mit eins oder zwei aus ($C_3$-$C_4$)-Cycloalkyl ; und/oder

A bedeutet :

unsubstituiertes Naphthyl ; oder

unsubstituiertes oder substituiertes Phenyl, wobei die Substituenten gleich oder unterschiedlich sein können eins bis drei aus Halo ; Nitro ; Cyano ; ($C_1$-$C_4$)-Alkyl ; Halo-($C_1$-$C_4$)-alkyl ; Cyano-($C_1$-$C_4$)-alkyl ; ($C_1$-$C_4$)-Alkoxy, ($C_1$-$C_4$)-Alkoxy-($C_1$-$C_4$)-alkyl ; –COD ; Carboxy ; ($C_1$-$C_4$)-Alkoxycarbonyl ; ($C_1$-$C_4$)-Alkanoyloxy ; $NH_2$ ; NHZ oder –NZZ′ ; ($C_1$-$C_4$)-Alkylthio ; –CSD ; –$CS_2D$ ; –SCOD ; unsubstituiertes oder substituiertes Phenyl mit gleich oder unterschiedlich eins bis zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder –NZZ′ ; oder wenn zwei benachbarte Positionen am Phenylring mit Alkoxy-Gruppen substituiert sind, sind diese Gruppen verbunden, um zusammen mit den Kohlenstoffatomen, an denen sie hängen, einen 5- oder 6-gliedrigen heterocyclischen Dioxolan- oder Dioxanring zu bilden ; und/oder

B bedeutet :

unsubstituiertes oder substituiertes ($C_1$-$C_8$)-Alkyl, welches als wahlweise(n) Substituenten gleich oder unterschiedlich eins bis drei aus Halo, Cyano, ($C_1$-$C_4$)-Alkoxy, Phenyl, ($C_1$-$C_4$)-Alkoxycarbonyl oder Halo-($C_1$-$C_4$)-alkoxy aufweist ; unsubstituiertes oder substituiertes ($C_3$-$C_8$)-Cycloalkyl oder unsubstituiertes oder substituiertes ($C_3$-$C_5$)-Cycloalkyl-($C_1$-$C_4$)-alkyl, welche als wahlweise(n) Substituenten gleich oder unterschiedlich eins oder zwei aus Halo, ($C_1$-$C_4$)-Alkyl, Halo-($C_1$-$C_4$)-alkyl, ($C_1$-$C_4$)-Alkoxy, ($C_1$-$C_4$)-Alkanoyl oder ($C_1$-$C_4$)-Haloalkoxy aufweist ;

unsubstituiertes oder substituiertes ($C_2$-$C_6$)-Alkenyl, welches als wahlweise(n) Substituenten ein Furyl oder gleich oder unterschiedlich eins bis drei aus ($C_1$-$C_4$)-Alkyl, Halo-($C_1$-$C_4$)-alkyl, ($C_1$-$C_4$)-Alkoxy oder Halo-($C_1$-$C_4$)-alkoxy aufweist ;

unsubstituiertes oder substituiertes ($C_3$-$C_8$)-Cycloalkenyl, welches als wahlweise Substituenten gleich oder unterschiedlich eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy aufweist ;

unsubstituiertes oder substituiertes Alkynyl ;

Phenalkyl mit einem oder zwei Kohlenstoffatomen in der Alkylgruppe, wobei die Alkygruppe unsubstituiert ist oder gleich oder unterschiedlich substituiert ist mit eins oder zwei aus Halo, ($C_1$-$C_4$)-alkyl, Halo-($C_1$-$C_4$)-alkyl, ($C_1$-$C_4$)-Alkoxy oder Halo-($C_1$-$C_4$)-alkoxy, und wobei der Phenylring unsubstituiert ist oder gleich oder unterschiedlich substituiert ist mit eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy ; oder ;

Phenalkenyl mit zwei oder drei Kohlenstoffatomen in der Alkenylgruppe, wobei die Alkenylgruppe unsubstituiert ist oder substituiert ist mit Halo,

($C_1$-$C_4$)-Alkyl, Halo-($C_1$-$C_4$)-alkyl, ($C_1$-$C_4$)-Alkoxy oder Halo-($C_1$-$C_4$)-alkoxy, und wobei der Phenylring unsubstituiert ist oder gleich oder unterschiedlich substituiert ist mit eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy ;

D die Bedeutung Wasserstoff oder ($C_1$-$C_4$)-Alkyl hat und Z und Z′ die Bedeutung ($C_1$-$C_4$)-Alkyl haben.

3. Zusammensetzung nach Anspruch 2, wobei

X und X′ die Bedeutung O oder S haben ; und/oder

$R^1$ verzweigtes ($C_3$-$C_8$)-Alkyl ist ; und/oder

A bedeutet :

unsubstituiertes oder substituiertes Phenyl mit gleich oder unterschiedlich eins bis drei aus Halo, Nitro, Cyano, ($C_1$-$C_4$)-Alkyl, Halo-($C_1$-$C_4$)-alkyl, Cyano-($C_1$-$C_4$)-alkyl, ($C_1$-$C_4$)-Alkoxy, –COD, ($C_1$-$C_4$)-Alkoxycarbonyl, ($C_1$-$C_4$)-Alkanoyloxy oder unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$ ; NHZ oder –NZZ′ ; und/oder

B bedeutet :

unsubstituiertes oder substituiertes ($C_1$-$C_6$)-Alkyl mit gleich oder unterschiedlich eins bis drei aus Halo,

Cyano, ($C_1$-$C_4$)-Alkoxy, Phenyl, ($C_1$-$C_3$)-Alkoxycarbonyl oder Halo-($C_1$-$C_4$)-alkoxy ;

unsubstituiertes oder substituiertes ($C_3$-$C_6$)-Cycloalkyl oder unsubstituiertes oder substituiertes ($C_3$-$C_6$)-Cycloalkyl-($C_1$-$C_4$)-alkyl, wobei der wahlweise Substituent Halo, ($C_1$-$C_4$)-Alkyl, ($C_1$-$C_4$)-Alkanoyl, ($C_1$-$C_4$)-Alkoxy oder Halo-($C_1$-$C_4$)-alkoxy ist ;

unsubstituiertes oder substituiertes ($C_2$-$C_6$)-Alkenyl mit gleich oder unterschiedlich eins oder zwei aus ($C_1$-$C_4$)-Alkyl, ($C_1$-$C_4$)-Alkoxy oder Halo-($C_1$-$C_4$)-alkoxy ;

unsubstituiertes oder substituiertes ($C_4$-$C_6$)-Cycloalkenyl, wobei der Substituent Halo, ($C_1$-$C_4$)-Alkyl, ($C_1$-$C_4$)-Alkoxy oder Halo-($C_1$-$C_4$)-alkoxy ist ; oder

unsubstituiertes oder phenylsubstituiertes Alkynyl.

4. Zusammensetzung nach Anspruch 3, wobei

X und X′ die Bedeutung O haben ; und/oder

$R^1$ die Bedeutung verzweigtes ($C_4$-$C_7$)-Alkyl hat ; und/oder

A bedeutet :

unsubstituiertes, monosubstituiertes, 2,3-disubstituiertes oder 2,4-disubstituiertes Phenyl, wobei die Substituenten gleich oder unterschiedlich Halo-($C_1$-$C_4$)-alkyl, ($C_1$-$C_4$)-Alkoxy oder Halo-($C_1$-$C_4$)-alkyl sein können; und/oder

B bedeutet :

unsubstituiertes oder substituiertes ($C_1$-$C_6$)-Alkyl mit gleich oder unterschiedlich eins bis drei aus Halo, Phenyl oder Cyano ;

unsubstituiertes ($C_4$-$C_6$)-Cycloalkyl ; unsubstituiertes oder substituiertes ($C_2$-$C_5$)-Alkenyl, wobei die wahlweise(n) Substituenten gleich oder unterschiedlich eins oder zwei ($C_1$-$C_4$)-Alkyl sind ;

unsubstituiertes ($C_4$-$C_6$)-Cycloalkenyl ; oder

Phenalkyl mit einem oder zwei Kohlenstoffatomen in der Alkylgruppe, und wobei der Phenylring unsubstituiert ist gleich oder unterschiedlich substituiert ist mit eins oder zwei aus Halo, ($C_1$-$C_4$)-Alkyl oder ($C_1$-$C_4$)-Alkoxy.

5. Zusammensetzung nach Anspruch 4, wobei

X und X′ die Bedeutung O haben ; und/oder

$R^1$ die Bedeutung t-Butyl, Neopentyl (2,2-Dimethylpropyl) oder 1,2,2-Trimethylpropyl hat ; und/oder

A unsubstituiertes oder monosubstituiertes Phenyl bedeutet, wobei der Substituent Chlor, Fluor, Brom, Iod, Methyl, Ethyl, Methoxy oder Trifluormethyl sein kann ; und/oder

B bedeutet :

unsubstituiertes oder substituiertes ($C_1$-$C_4$)-Alkyl, welches als wahlweise Substituenten gleich oder unterschiedlich eins oder drei aus Phenyl, Chlor, Fluor oder Brom aufweist ;

unsubstituiertes ($C_4$-$C_6$)-Cycloalkyl ;

unsubstituiertes ($C_4$-$C_6$)-Cycloalkenyl ; oder

Phenalkyl mit einem bis zwei Kohlenstoffatomen in der Alkylgruppe, wobei der Phenylring unsubstituiert ist, oder gleich oder unterschiedlich substituiert ist mit eins oder zwei aus Chlor, Fluor, Brom, Iod, Methyl, Ethyl, Methoxy oder Trifluormethyl.

6. Zusammensetzung nach Anspruch 3, wobei

X und X′ die Bedeutung O haben ;

$R^1$ die Bedeutung t-Butyl hat ; und

A die Bedeutung Phenyl und B die Bedeutung Cyclohexyl, Benzyl, 1-Cyclohexenyl, 2-Methylcyclohexadi-2,5-enyl, Phenylethynyl, 1-Methylpropyl, 2-Methyl-1-phenylbutyl, 3-Acetyl-2,2-dimethylcyclobutylmethyl, 1-Butenyl, 3-Butenyl, 3-Carbomethoxypropyl oder Methoxymethyl haben ; oder

A die Bedeutung 3,5-Dichlorphenyl und B die Bedeutung 2-Bromethyl haben ; oder

A die Bedeutung 4-Chlorphenyl und B die Bedeutung Cyclobutyl haben ; oder

A die Bedeutung 2,3-Dimethylphenyl und B die Bedeutung 1-Methylethenyl oder 1-Ethylephenyl haben ; oder

A die Bedeutung 4-Ethylphenyl und B die Bedeutung 1-Methylethenyl haben.

7. Zusammensetzung nach einem der vorhergehenden Ansprüche, enthaltend die insektizide Verbindung in einer Menge von 0,0001, bevorzugt 0,001 bis 99 Gew.-%.

8. Zusammensetzung nach Anspruch 7, enthaltend die insektizide Verbindung in einer Menge von 0,01 bis 99 Gew.-%.

9. Zusammensetzung nach einem der Ansprüche 7 bis 8 in Form eines benetzbaren Pulvers, eines Fließfähigen, eines Staubes, eines Granulats, eines Köders oder eines emulgierbaren Konzentrats.

10. Verfahren zum Kontrollieren von Insekten, bei dem man die Insekten mit insektizider Verbindung gemäß einem der Ansprüche 1 bis 6 in Kontakt bringt, wahlweise in einer Zusammensetzung nach einem der Ansprüche 1 bis 9.

11. Verfahren nach Anspruch 10, wobei man die Verbindung wachsenden Pflanzen oder einem Gebiet wo Pflanzen zu ziehen sind, in einer Menge von etwa 10 g bis 10 kg pro Hektar zusetzt.

12. Verfahren nach Anspruch 11, wobei die Applikationsrate 100 g bis 5 kg der Verbindung pro Hektar beträgt.

13. Verfahren nach Anspruch 12 zum Kontrollieren von Insekten der Ordnung Lepidoptera oder Coleoptera.

**Patentansprüche für den benannten Vertragsstaat ES**

1. Verwendung einer insektizid wirkenden Verbindung der Formel

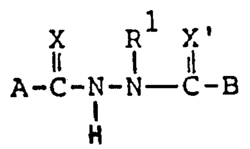

(I),

wobei

X und X′ gleich oder unterschiedlich die Bedeutung O, S oder NR haben ;

$R^1$ bedeutet :

unsubstituiertes verzweigtes $(C_3-C_{10})$-Alkyl oder geradkettiges $(C_1-C_4)$-Alkyl, gleich oder unterschiedlich substituiert mit einem oder zwei $(C_3-C_6)$-Cycloalkyl ;

A bedeutet :

unsubstituiertes oder substituiertes Naphthyl, wobei die Substituenten gleich oder unterschiedlich eins bis drei aus Halo ; Cyano ; Nitro ; Hydroxy ; $(C_1-C_4)$-Alkoxy ; $(C_1-C_4)$-Alkyl ; Carboxy ; $(C_1-C_4)$-Alkoxy-Carbonyl ; $(C_1-C_4)$-Alkanoyloxy oder $NH_2$ ; NHZ oder –NZZ′ sein können ; unsubstituiertes oder substituiertes Phenyl, wobei die Substituenten sein können gleich oder unterschiedlich eins bis fünf aus Halo ; Nitro ; Cyano ; Hydroxy ; $(C_1-C_6)$-Alkyl ; Halo-$(C_1-C_6)$-alkyl ; Cyano-$(C_1-C_6)$-alkyl ; $(C_1-C_6)$-Alkoxy ; Halo-$(C_1-C_6)$-alkoxy ; $(C_1-C_6)$-Alkoxy-$(C_1-C_6)$-alkyl mit unabhängig der angegebenen Anzahl von Kohlenstoffatomen in jeder Alkylgruppe; $(C_1-C_6)$-Alkoxy-$(C_1-C_6)$-alkoxy, unabhängig der angegebenen Anzahl an Kohlenstoffatomen in jeder Alkylgruppe ; $-OCO_2R$-Gruppe ; $(C_2-C_6)$-Alkenyl, wahlweise substituiert mit Halo, Cyano, $(C_1-C_4)$-Alkyl, $(C_1-C_4)$-Alkoxy, Halo-$(C_1-C_4)$-alkoxy oder $(C_1-C_4)$-Alkylthio ; Carboxy ; $-RCO_2R'$-Gruppe ; –COR ; Halo-$(C_1-C_6)$-alkylcarbonyl ; Cyano-$(C_1-C_6)$-alkylcarbonyl ; Nitro-$(C_1-C_6)$-alkylcarbonyl ; $(C_1-C_6)$-Alkoxycarbonyl ; Halo-$(C_1-C_6)$-alkoxycarbonyl ; –OCOR-Gruppe ; –NRR′-Gruppe ; Amino, substituiert mit Hydroxy-, $(C_1-C_4)$-Alkoxy- oder $(C_1-C_4)$-Alkylthio-Gruppen ; Phenylamino ; Diphenylamino ; –CONRR′-Gruppe ; –OCONRR′-Gruppe ; –NRCOR′-Gruppe ; $-NRCO_2R'$-Gruppe ; –N(COR)COR′-Gruppe ; –OCONRCOR′-Gruppe ; Sulfhydryl ; Halothio ; $(C_1-C_6)$-Alkylthio ; Halo-$(C_1C_6)$-alkylthio ; –SOR-Gruppe ; $-SO_2R$-Gruppe ; Phenylsulfonyl ; $-OSO_2R$-Gruppe ; Halo-$(C_1-C_6)$-alkylsulfonyloxy ; $-SO_2NRR'$-Gruppe ; –NRSOR′-Gruppe ; $-NRSO_2R'$-Gruppe, –CSR-Gruppe ; $CS_2R$-Gruppe ; –NRCSR′-Gruppe ; –SCOR-Gruppe ; unsubstituiertes oder substituiertes Phenyl mit gleich oder unterschiedlich eins bis drei aus Halo, Cyano, Nitro, Hydroxy$(C_1-C_4)$-alkyl, $(C_1-C_4)$-Alkoxy, Carboxy, $(C_1-C_4)$-Alkoxycarbonyl, $(C_1-C_4)$-Alkanyloxy, $NH_2$, NHZ oder –NZZ′ ; Phenoxy, wobei der Phenylring unsubstitutiert ist oder gleich oder unterschiedlich substituiert ist mit eins bis drei aus Halo, Cyano, Nitro, Hydroxy, $(C_1-C_4)$-Alkyl, $(C_1-C_4)$-Alkoxy, Carboxy, $(C_1-C_4)$-Alkoxycarbonyl, $(C_1-C_4)$-Alkanoyloxy, $NH_2$, NHZ oder –NZZ′ ; Phenylthio, wobei der Phenylring unsubstituiert ist oder gleich oder unterschiedlich substituiert ist mit eins bis drei aus Halo, Cyano, Nitro, Hydroxy, $(C_1-C_4)$-Alkyl, $(C_1-C_4)$-Alkoxy, Carboxy, $(C_1-C_4)$-Alkoxycarbonyl, $(C_1-C_4)$-Alkanoyloxy, $NH_2$, NHZ oder –NZZ′ ; oder, wenn zwei benachbarte Positionen am Phenylring mit Alkoxygruppen substituiert sind, diese Gruppen verbunden sein können, um zusammen mit den Kohlenstoffatomen, mit denen sie beide verbunden sind, einen 5- oder 6-gliedrigen heterocyclischen Dioxolan- oder Dioxanring zu bilden sind ;

B bedeutet :

unsubstituiertes oder substituiertes $(C_1-C_{10})$-Alkyl, welches als wahlweise(n) Substituenten gleich oder unterschiedlich eins bis vier aus Halo, Cyano, Nitro, Hydroxy, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, Phenyl, $NH_2$, NHZ oder –NZZ′ aufweist ;

unsubstituiertes oder substituiertes $(C_3-C_8)$-Cycloalkyl oder unsubstituiertes oder substituiertes $(C_3-C_8)$-Cycloalkyl$(C_1-C_4)$-Alkyl, welche als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkanoyl, $(C_1-C_4)$-Alkoxycarbonyl, $(C_1-C_4)$-Alkanoyloxy, Phenyl, $NH_2$, NHZ oder –NZZ′ aufweisen ;

unsubstituiertes oder substituiertes $(C_2-C_8)$-Alkenyl oder unsubstituiertes oder substituiertes $(C_3-C_8)$-Alkadienyl, welches als wahlweise(n) Substituenten eine Furyl-, Thienyl-, oder Pyridyl-Gruppe aufweist oder gleich

oder unterschiedlich von eins bis vier aus Halo, Cyano, Nitro, Hydroxy, $(C_1-C_4)$-Alkyl, $(C_3-C_6)$-Cycloalkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $NH_2$, NHZ oder –NZZ′ ;

unsubstituiertes oder substituiertes $(C_3-C_8)$-Cycloalkenyl oder unsubstituiertes oder substituiertes $(C_6-C_8)$-Cycloalkadienyl, welche als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $NH_2$, NHZ oder –NZZ′ aufweisen ;

unsubstituiertes oder substituiertes $(C_2-C_8)$-Alkynyl, welches als wahlweise(n) Substituenten gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-Alkoxy, Carboxy, $(C_1-C_4)$-Alkoxycarbonyl, $(C_1-C_4)$-Alkanoyloxy, Phenyl, $NH_2$, NHZ oder –NZZ′ aufweisen ;

Phenalkyl mit einem bis vier Kohlenstoffatomen in der Alkylgruppe, und wobei die Alkylgruppe unsubstituiert ist oder gleich oder unterschiedlich substituiert ist mit eins bis drei aus Halo, Cyano, Hydroxy, $(C_1-C_4)$-Alkyl, $(C_1-C_4)$-Alkoxy, $(C_1-C_4)$-Alkoxycarbonyl, $NH_2$, NHZ oder –NZZ′, und wobei der Phenylring unsubstituiert ist oder ist gleich oder unterschiedlich substituiert ist mit eins bis drei aus Halo, Cyano, Nitro, Hydroxy $(C_1-C_4)$-Alkyl, Halo-$(C_1-C_4)$-alkyl, Cyano-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $(C_2-C_6)$-Alkenyl, Halo-$(C_2-C_6)$-alkenyl, $(C_2-C_6)$-Alkynyl, $NH_2$, NHZ oder –NZZ′ ; oder

Phenalkyl mit zwei bis sechs Kohlenstoffatomen in der Alkylgruppe, wobei die Alkylgruppe unsubstituiert ist oder gleich oder unterschiedlich substituiert ist mit eins bis drei aus Halo, Cyano, Hydroxy, $(C_1-C_4)$-Alkyl, Halo-$(C_1-C_4)$-alkyl, $(C_1-C_4)$-Alkoxy, Halo-$(C_1-C_4)$-alkoxy, $(C_1-C_4)$-Alkoxycarbonyl, $NH_2$, NHZ oder –NZZ′, und wobei der Phenylring unsubstituiert ist oder gleich oder unterschiedlich substituiert ist mit einem bis drei aus Halo, Cyano, Nitro, Hydroxy $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, oder $NH_2$, NHZ oder –NZZ′ ;

wobei R und R′ die Bedeutung Wasserstoff oder $(C_1-C_6)$-Alkyl haben ; Z und Z′ die Bedeutung $(C_1-C_4)$-Alkyl haben ; wobei "Amino" die Bedeutung NRR′ hat ; oder

ein landwirtschaftlich vertretbares Salz davon ; mit der Ausnahme solcher Verbindungen, in denen X und X′ die Bedeutung O haben, $R^1$ die Bedeutung t-Butyl hat, A die Bedeutung unsubstituiertes Phenyl hat und B die Bedeutung unsubstituiertes Methyl oder unsubstituiertes Ethyl hat.

zusammen mit landwirtschaftlich vertretbaren Verdünnungs- oder Trägerstoffen zur Herstellung einer insektiziden Zusammensetzung.

2. Verwendung nach Anspruch 1 der Verbindung, wobei

X und X′ die Bedeutung O oder S haben ; und/oder

$R^1$ bedeutet :

unsubstituiertes verzweigtes $(C_3-C_8)$-Alkyl oder geradkettiges $(C_1-C_4)$-Alkyl, gleich oder unterschiedlich substituiert mit eins oder zwei aus $(C_3-C_4)$-Cycloalkyl ; und/oder

A bedeutet :

unsubstituiertes Naphthyl ; oder

unsubstituiertes oder substituiertes phenyl, wobei die Substituenten gleich oder unterschiedlich sein können eins bis drei aus Halo ; Nitro ; Cyano ; $(C_1-C_4)$-Alkyl ; Halo-$(C_1-C_4)$-alkyl ; Cyano-$(C_1-C_4)$-alkyl ; $(C_1-C_4)$-Alkoxy, $(C_1-C_4)$-Alkoxy-$(C_1-C_4)$-alkyl ; –COD ; Carboxy ; $(C_1-C_4)$-Alkoxycarbony1 ; $(C_1-C_4)$-Alkanoyloxy ; $NH_2$ ; NHZ oder –NZZ′ ; $(C_1-C_4)$-Alkylthio ; –CSD ; $-CS_2D$ ; –SCOD ; unsubstituiertes oder substituiertes phenyl mit gleich oder unterschiedlich eins bis zwei aus Halo, Nitro, $(C_1-C_4)$-Alkyl, $(C_1-C_4)$-Alkoxy, Carboxy, $(C_1-C_4)$-Alkoxycarbonyl, $(C_1-C_4)$-Alkanoyloxy, $NH_2$, NHZ oder –NZZ′ ; oder wenn zwei benachbarte positionen am phenylring mit Alkoxy-Gruppen substituiert sind, sind diese Gruppen verbunden, um zusammen mit den Kohlenstoffatomen, an denen sie hängen, einen 5- oder 6-gliedrigen heterocyclischen Dioxolan- oder Dioxanring zu bilden ; und/oder

B bedeutet :

unsubstituiertes oder substituiertes $(C_1-C_8)$-Alkyl, welches als wahlweise(n) Substituenten gleich oder unterschiedlich eins bis drei aus Halo, Cyano, $(C_1-C_4)$-Alkoxy, phenyl, $(C_1-C_4)$-Alkoxycarbonyl oder Halo-$(C_1-C_4)$-alkoxy aufweist ; unsubstituiertes oder substituiertes $(C_3-C_6)$-Cycloalkyl oder unsubstituiertes oder substituiertes $(C_3-C_5)$-Cycloalkyl-$(C_1-C_4)$-alkyl, welche als wahlweise(n) Substituenten gleich oder unterschiedlich eins oder zwei aus Halo, $(C_1-C_4)$-Alkyl, Halo-$(C_1-C_4)$-alkyl, $(C_1-C_4)$-Alkoxy, $(C_1-C_4)$-Alkanoyl oder $(C_1-C_4)$-Haloalkoxy aufweist ;

unsubstituiertes oder substituiertes $(C_2-C_6)$-Alkenyl, welches als wahlweise(n) Substituenten ein Furyl oder gleich oder unterschiedlich eins bis drei aus $(C_1-C_4)$-Alkyl, Halo-$(C_1-C_4)$-alkyl, $(C_1-C_4)$-Alkoxy oder Halo-$(C_1-C_4)$-alkoxy aufweist ;

unsubstituiertes oder substituiertes $(C_3-C_6)$-Cycloalkenyl, welches als wahlweise Substituenter gleich oder

unterschiedlich eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy aufweist;

unsubstituiertes oder substituiertes Alkynyl;

Phenalkyl mit einem oder zwei Kohlenstoffatomen in der Alkylgruppe, wobei die Alkygruppe unsubstituiert ist oder gleich oder unterschiedlich substituiert ist mit eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-amkoxy, und wobei der Phenylring unsubstituiert ist oder gleich oder unterschiedlich substituiert ist mit eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy; oder;

Phenalkenyl mit zwei oder drei Kohlenstoffatomen in der Alkenylgruppe, wobei die Alkenylgruppe unsubstituiert ist oder substituiert ist mit Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy, und wobei der Phenylring unsubstituiert ist oder gleich oder unterschiedlich substituiert ist mit eins oder zwei aus Halo, ($C_1$-$C_4$)-Alkyl, Halo-($C_1$-$C_4$)-alkyl, ($C_1$-$C_4$)-Alkoxy oder Halo-($C_1$-$C_4$)-alkoxy; oder ein

landwirtschaftlich vertretbares Salz davon; wobei D die Bedeutung Wasserstoff oder ($C_1$-$C_4$)-Alkyl hat und Z und Z′ die Bedeutung ($C_1$-$C_4$)-Alkyl haben.

3. Verwendung nach Anspruch 2 einer Verbindung, wobei

X und X′ die Bedeutung O oder S haben; und/oder

$R^1$ die Bedeutung verzweigtes ($C_3$-$C_8$)-Alkyl hat; und/oder

A bedeutet:

unsubstituiertes oder substituiertes Phenyl mit gleich oder unterschiedlich eins bis drei aus Halo, Nitro, Cyano, ($C_1$-$C_4$)-Alkyl, Halo-($C_1$-$C_4$)-alkyl, Cyano-($C_1$-$C_4$)-alkyl, ($C_1$-$C_4$)-Alkoxy, –COD, ($C_1$-$C_4$)-Alkoxycarbonyl, ($C_1$-$C_4$)-Alkanoyloxy oder unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$; NHZ oder –NZZ′; und/oder

B bedeutet:

unsubstituiertes oder substituiertes ($C_1$-$C_6$)-Alkyl mit gleich oder unterschiedlich eins bis drei aus Halo, Cyano, ($C_1$-$C_4$)-Alkoxy, Phenyl, ($C_1$-$C_3$)-Alkoxycarbonyl oder Halo-($C_1$-$C_4$)-alkoxy;

unsubstituiertes oder substituiertes ($C_3$-$C_6$)-Cycloalkyl oder unsubstituiertes oder substituiertes ($C_3$-$C_6$)-Cycloalkyl-($C_1$-$C_4$)-alkyl, wobei der wahl- ($C_3$-$C_6$)-Cycloalkyl-($C_1$-$C_4$)-alkyl, wobei der wahlweise Substituent Halo, ($C_1$-$C_4$)-Alkyl, ($C_1$-$C_4$)-Alkanoyl, ($C_1$-$C_4$)-Alkoxy oder Halo-($C_1$-$C_4$)-alkoxy ist;

unsubstituiertes oder substituiertes ($C_2$-$C_6$)-Alkenyl mit gleich oder unterschiedlich eins oder zwei aus ($C_1$-$C_4$)-Alkyl, ($C_1$-$C_4$)-Alkoxy oder Halo-($C_1$-$C_4$)-alkoxy;

unsubstituiertes oder substituiertes ($C_4$-$C_6$)-Cycloalkenyl, wobei der Substituent Halo, ($C_1$-$C_4$)-Alkyl, ($C_1$-$C_4$)-Alkoxy oder Halo-($C_1$-$C_4$)-alkoxy ist; oder

unsubstituiertes oder phenylsubstituiertes Alkynyl; oder ein

landwirtschaftlich vertretbares Salz davon.

4. Verwendung nach Anspruch 3 einer Verbindung, wobei

X und X′ die Bedeutung O haben; und/oder

$R^1$ die Bedeutung verzweigtes ($C_4$-$C_7$)-Alkyl hat; und/oder

A bedeutet:

unsubstituiertes, monosubstituiertes, 2,3-disubstituiertes oder 2,4-disubstituiertes Phenyl, wobei die Substituenten gleich oder unterschiedlich Halo-($C_1$-$C_4$)-alkyl, ($C_1$-$C_4$)-Alkoxy oder Halo-($C_1$-$C_4$)-alkyl sein können; und/oder

B bedeutet:

unsubstituiertes oder substituiertes ($C_1$-$C_6$)-Alkyl mit gleich oder unterschiedlich eins bis drei aus Halo, Phenyl oder Cyano; unsubstituiertes ($C_4$-$C_6$)-Cycloalkyl;

unsubstituiertes oder substituiertes ($C_2$-$C_5$)-Alkenyl, wobei die wahlweise(n) Substituenten gleich oder unterschiedlich eins oder zwei ($C_1$-$C_4$)-Alkyl sind;

unsubstituiertes ($C_4$-$C_6$)-Cycloalkenyl; oder

Phenalkyl mit einem oder zwei Kohlenstoffatomen in der Alkylgruppe, und wobei der Phenylring unsubstituiert ist gleich oder unterschiedlich substituiert ist mit eins oder zwei aus Halo, ($C_1$-$C_4$)-Alkyl oder ($C_1$-$C_4$)-Alkoxy; oder ein

landwirtschaftlich vertretbares Salz davon.

5. Verwendung nach Anspruch 4 der Verbindung, wobei

X und X′ die Bedeutung O haben; und/oder

$R^1$ die Bedeutung t-Butyl, Neopentyl (2,2-Dimethylpropyl) oder 1,2,2-Trimethylpropyl hat; und/oder

A unsubstituiertes oder monosubstituiertes Phenyl bedeutet, wobei der Substituent Chlor, Fluor, Brom, Iod, Methyl, Ethyl, Methoxy oder Trifluormethyl sein kann; und/oder

B bedeutet:

unsubstituiertes oder substituiertes ($C_1$-$C_4$)-Alkyl, welches als wahlweise Substituenten gleich oder unterschiedlich eins oder drei aus Phenyl, Chlor, Fluor oder Brom aufweist;

unsubstituiertes ($C_4$-$C_6$)-Cycloalkyl;

unsubstituiertes ($C_4$-$C_6$)-Cycloalkenyl; oder

Phenalkyl mit einem bis zwei Kohlenstoffatomen in der Alkylgruppe, und wobei der Phenylring unsubstituiert ist, oder gleich oder unterschiedlich substituiert ist mit eins oder zwei aus Chlor, Fluor, Brom, Iod, Methyl, Ethyl, Methoxy oder Trifluormethyl; oder ein

landwirtschaftlich vertretbares Salz davon.

6. Verwendung nach Anspruch 3 der Verbindung, wobei

X und X' die Bedeutung O haben;

$R^1$ die Bedeutung t-Butyl hat; und

A die Bedeutung Phenyl und B die Bedeutung Cyclohexyl, Benzyl, 1-Cyclohexenyl, 2-Methylcyclohexadi-2,5-enyl, Phenylethynyl, 1-Methylpropyl, 2-Methyl-1-phenylbutyl, 3-Acetyl-2,2-dimethylcyclobutylmethyl, 1-Butenyl, 3-Butenyl, 3-Carbomethoxypropyl oder Methoxymethyl haben; oder

A die Bedeutung 3,5-Dichlorphenyl und B die Bedeutung 2-Bromethyl haben; oder

A die Bedeutung 4-Chlorphenyl und B die Bedeutung Cyclobutyl haben; oder

A die Bedeutung 2,3-Dimethylphenyl und B die Bedeutung 1-Methylethenyl oder 1-Ethylethenyl haben; oder

A die Bedeutung 4-Ethylphenyl und B die Bedeutung 1-Methylethenyl haben.

7. Verwendung nach einem der vorhergehenden Ansprüche der Verbindung oder des Salzes in einer Menge von etwa 0,0001, bevorzugt 0,001 bis 99 Gew.-% der Zusammensetzung.

8. Verwendung nach Anspruch 6 der Verbindung oder des Salzes in einer Menge von 0,01 bis 99 Gew.-% der Zusammensetzung.

9. Verwendung nach einem der Ansprüche 6 bis 8 der Verbindung oder des Salzes zur Herstellung einer Zusammensetzung in Form eines benetzbaren Pulvers, eines Fließfähigen, eines Staubes, eines Granulats, eines Köders oder eines emulgierbaren Konzentrats.

10. Verwendung einer Verbindung oder eines Salzes wie in einem der Ansprüche 1 bis 6 definiert, wahlweise in einer Zusammensetzung auch enthaltend landwirtschaftlich vertretbaren Verdünnungs- oder Trägerstoff, zum Kontrollieren von Insekten durch Kontaktieren der Insekten mit einer insektizid wirksamen Menge der Verbindung oder des Salzes.

11. Mechanisches Verfahren zum Verbessern des Handelswertes und/oder der Rentabilität verkaufbarer Ernten aus Pflanzen, deren Wachstum von Insekten beeinflußt wird oder möglicherweise beeinflußt wird, bei dem man

(1) einen Behälter, einer Vernebelungsvorrichtung oder einem mechanischen Ausbreiter eine insektizide Verbindung oder ein Salz davon gemäß einem der Ansprüche 1 bis 6 zuführt, wahlweise in einer Mischung mit landwirtschaftlich vertretbarem Verdünnungs- oder Trägerstoff,

(2) den Behälter, Vernebeler oder mechanischen Ausbreiter dazu verwendet, die insektizide Verbindung oder das Salz in Form von Granulat, Staub, Rauch, Dampf oder einer tensidhaltigen Flüssigkeitszubereitung wachsenden Pflanzen oder einem Wachstumsmedium zu applizieren, wo die Pflanzen wachsen oder gezogen werden, oder den Insekten selbst,

(3) die Dosis des Wirkstoffs während des Applizierungsschrittes so steuert, daß die Applizierungsrate der aktiven insektiziden Verbindung zur Bekämpfung der Insekte ausreicht, jedoch nicht ausreicht zur Erzeugung einer nicht hinnehmbaren nachteiligen Auswirkung auf die in dem behandelten Gebiet wachsenden oder von den behandelten Samen zu ziehenden Erntepflanzen.

12. Verwendung oder Verfahren nach Anspruch 10 oder 11, wobei man die Menge oder das Salz mit einer Rate von etwa 10 g bis 10 kg pro Hektar zusetzt.

13. Verwendung oder Verfahren nach Anspruch 12, wobei die Applikationsrate 100 g bis 5 kg der Verbindung pro Hektar beträgt.

14. Verwendung oder Verfahren nach Anspruch 10 oder 11 zum Kontrollieren von Insekten der Ordnung Lepidoptera oder Coleoptera.

**Revendications**

**REVENDICATIONS Pour les Etats Contractants BE, CH, DE, FR, GB, GR, IT, LI, LU, NL, SE**

1. Composé à action insecticide, qui est un composé de formule

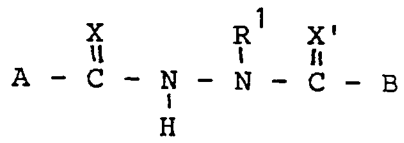

(I)

dans laquelle

X et X′ sont identiques ou différents, et représentent O, S ou NR ;

$R^1$ est un radical alkyle en $C_3$-$C_{10}$ ramifié non substitué ou un radical alkyle en $C_1$-$C_4$ à chaîne droite substitué par un ou deux groupes cycloalkyle en $C_3$-$C_6$, identiques ou différents ;

A est un radical naphtyle non substitué ou substitué comportant de un à trois substituants,identiques ou différents, choisis parmi des atomes d'halogène et des groupes cyano, nitro, hydroxy, alcoxy en $C_1$-$C_4$, alkyle en $C_1$-$C_4$, carboxy, alcoxy($C_1$-$C_4$)-carbonyle, alcanoyloxy en $C_1$-$C_4$ ou $NH_2$, NHZ ou –NZZ′ ;

un radical phényle non substitué ou substitué comportant de un à cinq substituants, identiques ou différents, choisis parmi des atomes d'halogène et des groupes nitro ; cyano ; hydroxy ; alkyle en $C_1$-$C_6$ ; halogénoalkyle en $C_1$-$C_6$ ; cyanoalkyle en $C_1$-$C_6$ ; alcoxy en $C_1$-$C_6$ ; halogénoalcoxy en $C_1$-$C_6$ ; alcoxy($C_1$-$C_6$)-alkyle($C_1$-$C_6$) ayant indépendamment le nombre indiqué d'atomes de carbone dans chaque groupe alkyle ; alcoxy($C_1$-$C_6$)-alcoxy($C_1$-$C_6$) ayant indépendamment le nombre indiqué d'atomes de carbone dans chaque groupe alkyle ; $-OCO_2R$ ; alcényle en $C_2$-$C_6$ éventuellement substitué par un atome d'halogène ou par un groupe cyano, alkyle en $C_1$-$C_4$, alcoxy en $C_1$-$C_4$, halogénoalcoxy en $C_1$-$C_4$ ou alkylthio en $C_1$-$C_4$ ; carboxy ; $-RCO_2R'$ ; –COR ; halogénoalkyl($C_1$-$C_6$)-carbonyle ; cyanoalkyl($C_1$-$C_6$)-carbonyle ; nitroalkyl($C_1$-$C_6$)-carbonyle ; alcoxy($C_1$-$C_6$)-carbonyle ; halogénoalcoxy($C_1$-$C_6$)-carbonyle ; –OCOR ; –NRR′ ; amino substitué par des groupes hydroxy, alcoxy en $C_1$-$C_4$ ou alkylthio en $C_1$-$C_4$ ; phénylamino ; diphénylamino ; –CONRR′ ; –OCONRR′ ; –NRCOR′ ; $-NRCO_2R'$ ; –N(COR)COR′ ; –OCONRCOR′ ; sulfhydryle ; halogénothio ; alkylthio en $C_1$-$C_6$ ; halogénoalkyl($C_1$-$C_6$)-thio ; –SOR ; $-SO_2R$ ; phénylsulfonyle ; $-OSO_2R$ ; halogénoalkyl($C_1$-$C_6$)-sulfonyloxy ; $-SO_2NRR'$ ; –NRSOR′ ; $-NRSO_2R'$ ; –CSR ; $-CS_2R$ ; –NRCSR′ ; –SCOR ; phényle non substitué ou substitué comportant de un à trois substituants, identiques ou différents, choisis parmi des atomes d'halogène et des groupes cyano, nitro, hydroxyalkyle en $C_1$-$C_4$, alcoxy en $C_1$-$C_4$, carboxy, alcoxy ($C_1$-$C_4$)-carbonyle, alcanoyloxy en $C_1$-$C_4$, $NH_2$, NHZ ou –NZZ′ ; un groupe phénoxy dans lequel le noyau phényle est non substitué ou substitué par un à trois substituants, identiques ou différents, choisis parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ′ ; un groupe phénylthio dans lequel le noyau phényle est non substitué ou substitué par un à trois substituants, identiques ou différents, choisis parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ′ ; ou, lorsque deux positions adjacentes sur le noyau phényle sont substituées par des groupes alcoxy, ces groupes peuvent être liés pour former, conjointement avec les atomes de carbone sur lesquels ils sont fixés, un noyau hétérocyclique dioxolanno ou dioxanno à 5 ou 6 éléments ;

B est un radical alkyle en $C_1$-$C_{10}$ non substitué ou substitué comportant, en tant que substituant(s) éventuel(s) de un à quatre substituants, identiques ou différents, choisis parmi des atomes d'halogène et des groupes cyano, nitro, hydroxy, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$ ; phényle, $NH_2$, NHZ ou –NZZ′ ;

un radical cycloalkyle en $C_3$-$C_8$ non substitué ou substitué ou cycloalkyl($C_3$-$C_8$)-alkyle($C_1$-$C_4$) non substitué ou substitué, comportant, en tant que substituant(s) éventuel(s), de un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcanoyle en $C_1$-$C_4$, alcoxy($C_1$-$C_4$)-carbonyle, alcanoyloxy en $C_1$-$C_4$, phényle, $NH_2$, NHZ ou –NZZ′ ;

un radical alcényle en $C_2$-$C_8$ non substitué ou substitué ou alcadiényle en $C_3$-$C_8$ non substitué ou substitué, comportant, en tant que substituant(s) éventuel(s) un groupe furyle, thiényle ou pyridyle ou de un à quatre substituants, identiques ou différents, choisis parmi des atomes d'halogène et des groupes cyano, nitro, hydroxy, alkyle en $C_1$-$C_4$, cycloalkyle en $C_3$-$C_6$, halogénoalkyle en $C_1$-$C_4$, alcoxy en $C_1$-$C_4$, halogénoalcoxy en $C_1$-$C_4$, carboxy, alcoxy-($C_1$-$C_4$)-carbonyle, alcanoyloxy en $C_1$-$C_4$, $NH_2$, NHZ ou –NZZ′ ;

un radical cycloalcényle en $C_3$-$C_8$ non substitué ou substitué ou cycloalcadiényle en $C_6$-$C_8$ substitué ou non substitué, comportant, en tant que substituant(s) éventuel(s),de un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, $NH_2$, NHZ ou –NZZ′ ;

un radical alcynyle en $C_2$-$C_8$ non substitué ou substitué comportant, en tant que substituant(s) éventuel(s), de un à quatre substituants, identiques ou différents, choisis parmi des atomes d'halogène et des groupes

cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, phényle, $NH_2$, NHZ ou –NZZ′ ;

un radical phénalkyle ayant de 1 à 4 atomes dans le fragment alkyle et dans lequel le fragment alkyle est non substitué ou substitué par un à trois substituants, identiques ou différents, choisis parmi des atomes d'halogène et des groupes cyano, hydroxy, alkyle en $C_1$-$C_4$, alcoxy en $C_1$-$C_4$, alcoxy($C_1$-$C_4$)-carbonyle, $NH_2$, NHZ ou –NZZ′, et le noyau phényle est non substitué ou substitué par un à trois substituants, identiques ou différents, choisis parmi des atomes d'halogène et des groupes cyano, nitro, hydroxy, alkyle en $C_1$-$C_4$, halogénoalkyle en $C_1$-$C_4$, cyanoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, alcényle en $C_2$-$C_6$, halogénoalcényle en $C_2$-$C_6$, alcynyle en $C_2$-$C_6$, $NH_2$, NHZ ou –NZZ′; ou

un radical phénalcényle ayant de 2 à 6 atomes de carbone dans le fragment alcényle et dont le fragment alcényle est non substitué ou substitué par un à trois substituants, identiques ou différents, choisis parmi des atomes d'halogène et des groupes cyano, hydroxy, alkyle en $C_1$-$C_4$, halogénoalkyle en $C_1$-$C_4$, alcoxy en $C_1$-$C_4$, halogénoalcoxy en $C_1$-$C_4$, alcoxy($C_1$-$C_4$)-carbonyle, $NH_2$, NHZ ou –NZZ′, et le noyau phényle est non substitué ou substitué par un à trois substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$ ou $NH_2$, NHZ ou –NZZ′ ;

R et R′ étant des atomes d'hydrogène ou des groupes alkyle en $C_1$-$C_6$ ; Z et Z′ étant des groupes alkyle en $C_1$-$C_4$ ; "amino" signifiant NRR′ ;

ou un sel agronomiquement acceptable de celui-ci ; à l'exception d'un composé dans lequel X et X′ sont O, $R^1$ est le groupe tert-butyle, A est un radical phényle non substitué et B est un radical méthyle non substitué ou éthyle non substitué.

2. Composé selon la revendication 1, qui est un composé dans lequel

X et X′ sont O ou S ; et/ou

$R^1$ est un radical alkyle en $C_3$-$C_8$ ramifié non substitué ou un radical alkyle en $C_1$-$C_4$ à chaîne droite substitué par un ou deux groupes cycloalkyle en $C_3$-$C_4$ identiques ou différents ; et/ou

A est un radical naphtyle non substitué ; ou

un radical phényle non substitué ou substitué comportant de un à trois substituants, identiques ou différents, choisis parmi des atomes d'halogène et des groupes nitro ; cyano ; alkyle en $C_1$-$C_4$ ; halogénoalkyle en $C_1$-$C_4$ ; cyanoalkyle en $C_1$-$C_4$ ; alcoxy en $C_1$-$C_4$ ; alcoxy ($C_1$-$C_4$)alkyle($C_1$-$C_4$) –COD ; carboxy ; alcoxy($C_1$-$C_4$)-carbonyle ; alcanoyloxy en $C_1$-$C_4$ ; $NH_2$, NHZ ou –NZZ′ ; alkylthio en $C_1$-$C_4$ ; –CSD ; –$CS_2$D ; –SCOD ; un groupe phényle substitué ou non substitué comportant un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ′ ; ou, lorsque deux positions adjacentes sur le noyau phényle sont substituées par des groupes alcoxy, ces groupes sont liés pour former, conjointement avec les atomes de carbone auxquels ils sont fixés, un noyau hétérocyclique dioxolanno ou dioxanno à 5 ou 6 éléments ; et/ou

B est un radical alkyle en $C_1$-$C_8$ non substitué ou substitué comportant, en tant que substituant(s) éventuel(s), de un à trois substituants, identiques ou différents, choisis parmi des atomes d'halogène et des groupes cyano, alcoxy en $C_1$-$C_4$, phényle, alcoxy($C_1$-$C_4$)-carbonyle et halogénoalcoxy en $C_1$-$C_4$ ;

un radical cycloalkyle en $C_3$-$C_6$ non substitué ou substitué ou cycloalkyl($C_3$-$C_5$)-alkyle($C_1$-$C_4$) non substitué ou substitué, comportant, en tant que substituant(s) éventuel(s), un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$, alcanoyle en $C_1$-$C_4$ et halogénoalcoxy en $C_1$-$C_4$ ;

un radical alcényle en $C_2$-$C_6$ non substitué ou substitué comportant, en tant que substituant(s) éventuel(s) un groupe furyle ou de un à trois substituants, identiques ou différents, choisis parmi des groupes alkyle en $C_1$-$C_4$, halogénoalkyle en $C_1$-$C_4$, alcoxy en $C_1$-$C_4$ et halogénoalcoxy en $C_1$-$C_4$ ;

un radical cycloalcényle en $C_3$-$C_6$ non substitué ou substitué comportant,en tant que substituant(s) éventuels(s) un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ;

un radical alcynyle non substitué ou substitué par le groupe phényle ;

un radical phénalkyle ayant 1 ou 2 atomes de carbone dans le fragment alkyle et dont le fragment alkyle est non substitué ou substitué par un ou deux substituants identiques ou différents, choisis parmi des atomes d'halogène et des groupes alkyle en $C_1$-$C_4$, halogénoalkyle en $C_1$-$C_4$, alcoxy en $C_1$-$C_4$ ou halogénoalcoxy en $C_1$-$C_4$, et le noyau phényle est non substitué ou substitué par un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ; ou

un radical phénalcényle ayant 2 ou 3 atomes de carbone dans le fragment alcényle et dont le fragment alcényle est non substitué ou substitué par des substituants choisis parmi des atomes d'halogène et des grou-

pes alkyle en $C_1$-$C_4$, halogénoalkyle en $C_1$-$C_4$, alcoxy $C_1$-$C_4$ ou halogénoalkyle en $C_1$-$C_4$, et le noyau phényle est non substitué ou substitué par un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ;
ou un sel agronomiquement acceptable de celui-ci ; D étant un atome d'hydrogène ou un groupe alkyle en $C_1$-$C_4$, et Z et Z' étant des groupes alkyle en $C_1$-$C_4$.

3. Composé selon la revendication 2, qui est un composé dans lequel
X et X' sont O ou S ; et/ou
$R^1$ est un radical alkyle en $C_3$-$C_8$ ramifié ; et/ou
A est un radical naphtyle non substitué ; ou
un radical phényle non substitué ou substitué comportant de un à trois substituants, identiques ou différents, choisis parmi des atomes d'halogène et des groupes nitro, cyano, alkyle en $C_1$-$C_4$, halogénoalkyle en $C_1$-$C_4$, cyanoalkyle en $C_1$-$C_4$, alcoxy en $C_1$-$C_4$, –COD, alcoxy($C_1$-$C_4$)-carbonyle, alcanoyloxy en $C_1$-$C_4$ et un groupe phényle non substitué ou substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; et/ou
B est un radical alkyle en $C_1$-$C_6$ non substitué ou substitué comportant de un à trois substituants, identiques ou différents, choisis parmi des atomes d'halogène et des groupes cyano, alcoxy en $C_1$-$C_4$, phényle, alcoxy($C_1$-$C_3$)-carbonyle et halogénoalcoxy en $C_1$-$C_4$ ;
un radical cycloalkyle en $C_3$-$C_6$ non substitué ou substitué ou cycloalkyl($C_3$-$C_6$)-alkyle($C_1$-$C_4$) non substitué ou substitué, dans lesquels le substituant éventuel est un atome d'halogène ou un groupe alkyle en $C_1$-$C_4$, alcanoyle en $C_1$-$C_4$, alcoxy en $C_1$-$C_4$ ou halogénoalcoxy en $C_1$-$C_4$ ;
un radical alcényle non substitué ou substitué comportant un ou deux substituants, identiques ou différents, alkyle en $C_1$-$C_4$, alcoxy en $C_1$-$C_4$ ou halogénoalcoxy en $C_1$-$C_4$ ;
un radical cycloalcényle en $C_4$-$C_6$ non substitué ou substitué dans lequel le substituant est un atome d'halogène ou
un groupe alkyle en $C_1$-$C_4$, alcoxy en $C_1$-$C_4$ ou halogénoalcoxy en $C_1$-$C_4$ ; ou
un radical alcynyle non substitué ou substitué par le groupe phényle ;
ou un sel agronomiquement acceptable de celui-ci.

4. Composé selon la revendication 3, qui est un composé dans lequel
X et X' sont O ; et/ou
$R^1$ est un radical alkyle en $C_4$-$C_7$ ramifié ; et/ou
A est un radical phényle non substitué, monosubstitué, 2,3-disubstitué ou 2,4-disubstitué, dans lequel les substituants peuvent être des substituants, identiques ou différents, choisis parmi des atomes d'halogène et des groupes alkyle en $C_1$-$C_4$, alcoxy en $C_1$-$C_4$ et halogénoalkyle en $C_1$-$C_4$ ; et/ou
B est un radical alkyle en $C_1$-$C_6$ non substitué ou substitué comportant de un à trois substituants, identiques ou différent, halogéno, phényle ou cyano ;
un radical cycloalkyle en $C_4$-$C_6$ non substitué ;
un radical alcényle en $C_2$-$C_5$ non substitué ou substitué comportant, en tant que substituant(s) éventuel(s), un ou deux groupes alkyle en $C_1$-$C_4$ identiques ou différents ;
un radical cycloalcényle en $C_4$-$C_6$ non substitué ; ou
un radical phénalkyle ayant 1 ou 2 atomes de carbone dans le fragment alkyle et dont le noyau phényle est non substitué ou substitué par un ou deux substituants, identiques ou différents, choisis parmi des atomes d'halogène et des groupes alkyle en $C_1$-$C_4$ et alcoxy en $C_1$-$C_4$ ;
ou un sel agronomiquement acceptable de celui-ci.

5. Composé selon la revendication 4, qui est un composé dans lequel
X et X' sont O ; et/ou
$R^1$ est le groupe tert-butyle, néopentyle (2,2-diméthylpropyle) ou 1,2,2-triméthylpropyle ; et/ou
A est un radical phényle non substitué ou monosubstitué dans lequel le substituant peut être chloro, fluoro, bromo, iodo, méthyle, éthyle, méthoxy ou trifluorométhyle ; et/ou
B est un radical alkyle en $C_1$-$C_4$ non substitué ou substitué comportant, en tant que substituant(s) éventuel(s), un à trois des substituants, identiques ou différents, phényle, chloro, fluoro ou bromo ;
un radical cycloalkyle en $C_4$-$C_6$ non substitué ;
un radical cycloalcényle en $C_4$-$C_6$ non substitué ; ou
un radical phénalkyle ayant 1 ou 2 atomes de carbone dans le fragment alkyle et dont le noyau phényle est non substitué ou substitué par un ou deux des substituants, identiques ou différents, chloro, fluoro, bromo, iodo, méthyle, éthyle, méthoxy ou trifluorométhyle ;
ou un sel agronomiquement acceptable de celui-ci.

6. Composé selon la revendication 3, qui est un composé dans lequel

X et X′ sont O ;

$R^1$ est le groupe tert-butyle ; et

A est le groupe phényle et B est le groupe cyclohexyle, benzyle, 1-cyclohexényle, 2-méthylcyclohexadi-2,5-ényle, phényléthynyle, 1-méthylpropyle, 2-méthyl-1-phénylbutyle, 3-acétyl-2,2-diméthylcyclobutylméthyle, 1-butényle, 3-butényle, 3-carbométhoxypropyle ou méthoxyméthyle ; ou

A est le groupe 3,5-dichlorophényle et B est le groupe 2-bromoéthyle ; ou

A est le groupe 4-chlorophényle et B est le groupe cyclobutyle ; ou

A est le groupe 2,3-diméthylphényle et B est le groupe 1-méthyléthényle ou 1-éthyléthényle ; ou

A est le groupe 4-éthylphényle et B est le groupe 1-méthyléthényle.

7. Composition insecticide comprenant un diluant ou support agronomiquement acceptable et un composé insecticide selon l'une quelconque des revendications 1 à 6.

8. Composition selon la revendication 7, contenant le composé insecticide en une quantité de 0,0001, de préférence 0,001, à 99%, en poids.

9. Composition selon la revendication 7, contenant le composé insecticide en une quantité de 0,01 à 99% en poids.

10. Composition selon l'une quelconque des revendications 7 à 9, sous la forme d'une poudre mouillable, d'une composition fluide, d'une poudre, d'un granulé, d'un appât ou d'un concentré émulsifiable.

11. Procédé de lutte contre les insectes, lequel comprend la mise en contact desdits insectes avec un composé insecticide selon l'une quelconque des revendications 1 à 6, éventuellement dans une composition selon l'une quelconque des revendications 7 à 9.

12. Procédé selon la revendication 11, lequel comprend l'application du composé, à un taux d'environ 10 g à environ 10 kg par hectare, sur des plantes en croissance ou sur une zone où des plantes sont destinées à croître.

13. Procédé selon la revendication 12, dans lequel le taux d'application va de 100 g à 5 kg du composé par hectare.

14. Procédé selon la revendication 13, pour la lutte contre des insectes appartenant à l'ordre des lépidoptères ou des coléoptères.

**REVENDICATIONS Pour l'Etat Contractant AT**

1. Composition à action insecticide, comprenant un composé de formule

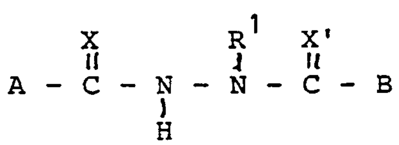

(I)

dans laquelle

X et X′ sont identiques ou différents, et représentent O, S ou NR ;

$R^1$ est un radical alkyle en $C_3$-$C_{10}$ ramifié non substitué ou un radical alkyle en $C_1$-$C_4$ à chaîne droite substitué par un ou deux groupes cycloalkyle en $C_3$-$C_6$, identiques ou différents ;

A est un radical naphtyle non substitué ou substitué comportant de un à trois substituants, identiques ou différents, choisis parmi des atomes d'halogène et des groupes cyano, nitro, hydroxy, alcoxy en $C_1$-$C_4$, alkyle en $C_1$-$C_4$, carboxy, alcoxy($C_1$-$C_4$)-carbonyle, alcanoyloxy en $C_1$-$C_4$ ou $NH_2$, NHZ ou –NZZ′ ;

un radical phényle non substitué ou substitué comportant de un à cinq substituants, identiques ou différents, choisis parmi des atomes d'halogène et des groupes nitro ; cyano ; hydroxy ; alkyle en $C_1$-$C_6$ ; halogénoalkyle en $C_1$-$C_6$ ; cyanoalkyle en $C_1$-$C_6$ ; alcoxy en $C_1$-$C_6$ ; halogénoalcoxy en $C_1$-$C_6$ ; alcoxy($C_1$-$C_6$)-alkyle($C_1$-$C_6$) ayant indépendamment le nombre indiqué d'atomes de carbone dans chaque groupe alkyle ; alcoxy($C_1$-$C_6$)-alcoxy($C_1$-$C_6$) ayant indépendamment le nombre indiqué d'atomes de carbone dans chaque groupe alkyle ; $-OCO_2R$ ; alcényle en $C_2$-$C_6$ éventuellement substitué par un atome d'halogène ou par un groupe cyano, alkyle en $C_1$-$C_4$, alcoxy en $C_1$-$C_4$, halogénoalcoxy en $C_1$-$C_4$ ou alkylthio en $C_1$-$C_4$ ; carboxy ; $-RCO_2R'$ ; –COR ; halogénoalkyl($C_1$-$C_6$)-carbonyle ; cyanoalkyl($C_1$-$C_6$)-carbonyle ; nitroalkyl($C_1$-$C_6$)-carbonyle ; alcoxy($C_1$-$C_6$)-carbonyle ; halogénoalcoxy($C_1$-$C_6$)-carbonyle ; –OCOR ; –NRR′ ; amino substitué par des groupes hydroxy, alcoxy en $C_1$-$C_4$ ou alkylthio en $C_1$-$C_4$ ; phénylamino ; diphénylamino ; –CONRR′ ; –OCONRR′ ; –NRCOR′ ; $-NRCO_2R'$ ; –N(COR)COR′ ; –OCONRCOR′ ; sulfhydryle ; halogénothio ; alkylthio en $C_1$-$C_6$ ; halogénoalkyl($C_1$-$C_6$)-thio ; –SOR ; $-SO_2R$ ; phénylsulfonyle ; $-OSO_2R$ ; halogénoalkyl($C_1$-$C_6$)-sul-

fonyloxy ; $-SO_2NRR'$ ; $-NRSOR'$ ; $-NRSO_2R'$ ; $-CSR$ ; $-CS_2R$ ; $-NRCSR'$ ; $-SCOR$ ;

un radical phényle non substitué ou substitué comportant de un à trois substituants, identiques ou différents, choisis parmi des atomes d'halogène et des groupes cyano, nitro, hydroxyalkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; un groupe phénoxy dans lequel le noyau phényle est non substitué ou substitué par un à trois substituants, identiques ou différents, choisis parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; un groupe phénylthio dans lequel le noyau phényle est non substitué ou substitué par un à trois substituants, identiques ou différents, choisis, parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; ou, lorsque deux positions adjacentes sur le noyau phényle sont substituées par des groupes alcoxy, ces groupes peuvent être liés pour former, conjointement avec les atomes de carbone sur lesquels ils sont fixés, un noyau hétérocyclique dioxolanno ou dioxanno à 5 ou 6 éléments ;

B est un radical alkyle en $C_1$-$C_{10}$ non substitué ou substitué comportant, en tant que substituant(s) éventuel(s), de un à quatre substituants, identiques ou différents,choisis parmi des atomes d'halogène et des groupes cyano, nitro, hydroxy, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$ ; phényle, $NH_2$, NHZ ou –NZZ' ;

un radical cycloalkyle en $C_3$-$C_8$ non substitué ou substitué ou cycloalkyl($C_3$-$C_8$)-alkyle($C_1$-$C_4$) non substitué ou substitué comportant en tant que substituant(s) éventuel(s) de un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcanoyle en $C_1$-$C_4$, alcoxy($C_1$-$C_4$)-carbonyle, alcanoyloxy en $C_1$-$C_4$, phényle, $NH_2$, NHZ ou –NZZ' ;

un radical alcényle en $C_2$-$C_8$ non substitué ou substitué ou alcadiényle en $C_3$-$C_8$ non substitué ou substitué, comportant, en tant que substituant(s) éventuel(s), un groupe furyle, thiényle ou pyridyle ou de un à quatre substituants, identiques ou différents, choisis parmi des atomes d'halogène et des groupes cyano, nitro, hydroxy, alkyle en $C_1$-$C_4$, cycloalkyle en $C_3$-$C_6$, halogénoalkyle en $C_1$-$C_4$, alcoxy en $C_1$-$C_4$, halogénoalcoxy en $C_1$-$C_4$, carboxy, alcoxy-($C_1$-$C_4$)-carbonyle, alcanoyloxy en $C_1$-$C_4$, $NH_2$, NHZ ou –NZZ' ;

un radical cycloalcényle en $C_3$-$C_8$ non substitué ou substitué ou cycloalcadiényle en $C_6$-$C_8$ substitué ou non substitué, comportant, en tant que substituant(s) éventuel(s), de un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, $NH_2$, NHZ ou –NZZ' ;

un radical alcynyle en $C_2$-$C_8$ non substitué ou substitué comportant en tant que substituant(s) éventuel(s) de un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, phényle, $NH_2$, NHZ ou –NZZ' ;

un radical phénalkyle ayant de 1 à 4 atomes dans le fragment alkyle et dans lequel le fragment alkyle est non substitué ou substitué par un à trois substituants, identiques ou différents, choisis parmi des atomes d'halogène et des groupes cyano, hydroxy, alkyle en $C_1$-$C_4$, alcoxy en $C_1$-$C_4$, alcoxy($C_1$-$C_4$)-carbonyle, $NH_2$, NHZ ou –NZZ', et le noyau phényle est non substitué ou substitué par un à trois substituants, identiques ou différents, choisis parmi des atomes d'halogène et des groupes cyano, nitro, hydroxy, alkyle en $C_1$-$C_4$, halogénoalkyle en $C_1$-$C_4$, cyanoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, alcényle en $C_2$-$C_6$, halogénoalcényle en $C_2$-$C_6$, alcynyle en $C_2$-$C_6$, $NH_2$, NHZ ou –NZZ'; ou

un radical phénalcényle ayant de 2 à 6 atomes de carbone dans le fragment alcényle et dont le fragment alcényle est non substitué ou substitué par un à trois substituants, identiques ou différents, choisis parmi des atomes d'halogène et des groupes cyano, hydroxy, alkyle en $C_1$-$C_4$, halogénoalkyle en $C_1$-$C_4$, alcoxy en $C_1$-$C_4$, halogénoalcoxy en $C_1$-$C_4$, alcoxy($C_1$-$C_4$)-carbonyle, $NH_2$, NHZ ou –NZZ', et le noyau phényle est non substitué ou substitué par un à trois substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$ ou $NH_2$, NHZ ou –NZZ' ;

R et R' étant des atomes d'hydrogène ou des groupes alkyle en $C_1$-$C_6$ ; Z et Z' étant des groupes alkyle en $C_1$-$C_4$ ; "amino" signifiant NRR' ;

ou un sel agronomiquement acceptable de celui-ci ; à l'exception d'un composé dans lequel X et X' sont O, $R^1$ est le groupe tert-butyle, A est un radical phényle non substitué et B est un radical méthyle non substitué ou éthyle non substitué ; conjointement avec un diluant ou support agronomiquement acceptable.

2. Composition selon la revendication 1, dans laquelle

X et X' sont O ou S ; et/ou

$R^1$ est un radical alkyle en $C_3$-$C_8$ ramifié non substitué ou un radical alkyle en $C_1$-$C_4$ à chaîne droite substitué par un ou deux groupes cycloalkyle en $C_3$-$C_4$, identiques ou différents ; et/ou

A est un radical naphtyle non substitué ; ou

un radical phényle non substitué ou substitué comportant de un à trois substituants identiques ou différents choisis parmi des atomes d'halogène et des groupes nitro ; cyano ; alkyle en $C_1$-$C_4$ ; halogénoalkyle en $C_1$-$C_4$; cyanoalkyle en $C_1$-$C_4$ ; alcoxy en $C_1$-$C_4$ ; alcoxy($C_1$-$C_4$)alkyle($C_1$-$C_4$) ; –COD ; carboxy ; alcoxy($C_1$-$C_4$)-carbonyle ; alcanoyloxy en $C_1$-$C_4$ ; $NH_2$, NHZ ou –NZZ' ; alkylthio en $C_1$-$C_4$ ; –CSD ; –$CS_2D$ ; –SCOD ; phényle substitué ou non substitué comportant un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; ou, lorsque deux positions adjacentes sur le noyau phényle sont substituées par des groupes alcoxy, ces groupes sont liés pour former, conjointement avec les atomes de carbone auxquels ils sont fixés, un noyau hétérocyclique dioxolanno ou dioxanno à 5 ou 6 éléments ; et/ou

B est un radical alkyle en $C_1$-$C_8$ non substitué ou substitué comportant, en tant que substituant(s) éventuel(s), de un à trois substituants, identiques ou différents, choisis parmi des atomes d'halogène et des groupes cyano, alcoxy en $C_1$-$C_4$, phényle, alcoxy($C_1$-$C_4$)-carbonyle et halogénoalcoxy en $C_1$-$C_4$ ;

un radical cycloalkyle en $C_3$-$C_6$ non substitué ou substitué ou cycloalkyl($C_3$-$C_5$)-alkyle($C_1$-$C_4$) non substitué ou substitué, comportant, en tant que substituant(s) éventuel(s), un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$, alcanoyle en $C_1$-$C_4$ et halogénoalcoxy en $C_1$-$C_4$ ;

un radical alcényle en $C_2$-$C_6$ non substitué ou substitué comportant en tant que substituant(s) éventuel(s) un groupe furyle ou de un à trois substituants, identiques ou différents, choisis parmi des groupes alkyle en $C_1$-$C_4$, halogénoalkyle en $C_1$-$C_4$, alcoxy en $C_1$-$C_4$ et halogénoalcoxy en $C_1$-$C_4$ ;

un radical cycloalcényle en $C_3$-$C_6$ non substitué ou substitué comportant,en tant que substituant(s) éventuels(s),un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ;

un radical alcynyle non substitué ou substitué par le groupe phényle ;

un radical phénalkyle ayant 1 ou 2 atomes de carbone dans le fragment alkyle et dont le fragment alkyle est non substitué ou substitué par un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ ou halogénoalcoxy en $C_1$-$C_4$, et le noyau phényle est non substitué ou substitué par un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ; ou

un radical phénalcényle ayant 2 ou 3 atomes de carbone dans le fragment alcényle et dont le fragment alcényle est non substitué ou substitué par des substituants choisis parmi des atomes d'halogène et des groupes alkyle en $C_1$-$C_4$, halogénoalkyle en $C_1$-$C_4$, alcoxy en $C_1$-$C_4$ ou halogénoalkyle en $C_1$-$C_4$, et le noyau phényle est non substitué ou substitué par un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ;

D étant un atome d'hydrogène ou un groupe alkyle en $C_1$-$C_4$, et

Z et Z' étant des groupes alkyle en $C_1$-$C_4$.

3. Composition selon la revendication 2, dans laquelle

X et X' sont O ou S ; et/ou

$R^1$ est un radical alkyle en $C_3$-$C_8$ ramifié ; et/ou

A est un radical naphtyle non substitué ; ou

un radical phényle non substitué ou substitué comportant de un à trois substituants, identiques ou différents, choisis parmi des atomes d'halogène et des groupes nitro, cyano, alkyle en $C_1$-$C_4$, halogénoalkyle en $C_1$-$C_4$, cyanoalkyle en $C_1$-$C_4$, alcoxy en $C_1$-$C_4$, –COD, alcoxy($C_1$-$C_4$)-carbonyle, alcanoyloxy en $C_1$-$C_4$ et un groupe phényle non substitué ou substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; et/ou

B est un radical alkyle en $C_1$-$C_6$ non substitué ou substitué comportant de un à trois substituants, identiques ou différents, choisis parmi des atomes d'halogène et des groupes cyano, alcoxy en $C_1$-$C_4$, phényle, alcoxy($C_1$-$C_3$)-carbonyle et halogénoalcoxy en $C_1$-$C_4$ ;

un radical cycloalkyle en $C_3$-$C_6$ non substitué ou substitué ou cycloalkyl($C_3$-$C_6$)-alkyle($C_1$-$C_4$) non substitué ou substitué, dans lesquels le substituant éventuel est un atome d'halogène ou un groupe alkyle en $C_1$-$C_4$, alcanoyle en $C_1$-$C_4$, alcoxy en $C_1$-$C_4$ ou halogénoalcoxy en $C_1$-$C_4$ ;

un radical alcényle non substitué ou substitué comportant un ou deux substituants, identiques ou différents, alkyle en $C_1$-$C_4$, alcoxy en $C_1$-$C_4$ ou halogénoalcoxy en $C_1$-$C_4$ ;

un radical cycloalcényle en $C_4$-$C_6$ non substitué ou substitué dans lequel le substituant est un atome d'halogène ou un groupe alkyle en $C_1$-$C_4$, alcoxy en $C_1$-$C_4$ ou halogénoalcoxy alcoxy en $C_1$-$C_4$ ; ou

un radical alcynyle non substitué ou substitué par le groupe phényle.

4. Composition selon la revendication 3, dans laquelle

X et X′ sont O ; et/ou

$R^1$ est un radical alkyle en $C_4$-$C_7$ ramifié ; et/ou

A est un radical phényle non substitué, monosubstitué, 2,3-disubstitué ou 2,4-disubstitué, dans lequel les substituants peuvent être des substituants, identiques ou différents, choisis parmi des atomes d'halogène et des groupes alkyle en $C_1$-$C_4$, alcoxy en $C_1$-$C_4$ et halogénoalkyle en $C_1$-$C_4$ ; et/ou

B est un radical alkyle en $C_1$-$C_6$ non substitué ou substitué comportant de un à trois substituants, identiques ou différents, halogéno, phényle ou cyano ;

un radical cycloalkyle en $C_4$-$C_6$ non substitué ;

un radical alcényle en $C_2$-$C_5$ non substitué ou substitué comportant, en tant que substituant(s) éventuel(s), un ou deux groupes alkyle en $C_1$-$C_4$, identiques ou différents ;

un radical cycloalcényle en $C_4$-$C_6$ non substitué ; ou

un radical phénalkyle ayant 1 ou 2 atomes de carbone dans le fragment alkyle et dont le noyau phényle est non substitué ou substitué par un ou deux substituants, identiques ou différents, choisis par des atomes d'halogène et des groupes alkyle en $C_1$-$C_4$ et alcoxy en $C_1$-$C_4$.

5. Composition selon la revendication 4, dans laquelle

X et X′ sont O ; et/ou

$R^1$ est le groupe tert-butyle, néopentyle (2,2-diméthylpropyle) ou 1,2,2-triméthylpropyle ; et/ou

A est un radical phényle non substitué ou monosubstitué dans lequel le substituant peut être chloro, fluoro, bromo, iodo, méthyle, éthyle, méthoxy ou trifluorométhyle ; et/ou

B est un radical alkyle en $C_1$-$C_4$ non substitué ou substitué comportant,en tant que substituant(s) éventuel(s), un à trois des substituants, identiques ou différents, phényle, chloro, fluoro ou bromo ;

un radical cycloalkyle en $C_4$-$C_6$ non substitué ;

un radical cycloalcényle en $C_4$-$C_6$ non substitué ; ou

un radical phénalkyle ayant 1 ou 2 atomes de carbone dans le fragment alkyle et dont le noyau phényle est non substitué ou substitué par un ou deux des substituants, identiques ou différents, chloro, fluoro, bromo, iodo, méthyle, éthyle, méthoxy ou trifluorométhyle.

6. Composition selon la revendication 3, dans laquelle

X et X′ sont O ;

$R^1$ est le groupe tert-butyle ; et

A est le groupe phényle et B est le groupe cyclohexyle, benzyle, 1-cyclohexényle, 2-méthylcyclohexadi-2,5-ényle, phényléthynyle, 1-méthylpropyle, 2-méthyl-1-phénylbutyle, 3-acétyl-2,2-diméthylcyclobutylméthyle, 1-butényle, 3-butényle, 3-carbométhoxypropyle ou méthoxyméthyle ; ou

A est le groupe 3,5-dichlorophényle et B est le groupe 2-bromoéthyle ; ou

A est le groupe 4-chlorophényle et B est le groupe cyclobutyle ; ou

A est le groupe 2,3-diméthylphényle et B est le groupe 1-méthyléthényle ou 1-éthyléthényle ; ou

A est le groupe 4-éthylphényle et B est le groupe 1-méthyléthényle.

7. Composition selon l'une quelconque des revendications précédentes, contenant le composé insecticide en une quantité de 0,0001, de préférence 0,001, à 99%, en poids.

8. Composition selon la revendication 7, contenant le composé insecticide en une quantité de 0,01 à 99%, en poids.

9. Composition selon la revendication 7 ou 8, sous la forme d'une poudre mouillable, d'une composition fluide, d'une poudre, d'un granulé, d'un appât ou d'un concentré émulsifiable.

10. Procédé de lutte contre les insectes, lequel comprend la mise en contact desdits insectes avec un composé insecticide tel que défini dans l'une quelconque des revendications 1 à 6, éventuellement dans une composition selon l'une quelconque des revendications 1 à 9.

11. Procédé selon la revendication 10, lequel comprend l'application du composé, à un taux d'environ 10 g à environ 10 kg par hectare, sur des plantes en croissance ou sur une zone où des plantes sont destinées à croître.

12. Procédé selon la revendication 11, dans lequel le taux d'application va de 100 g à 5 kg du composé par hectare.

13. Procédé selon la revendication 12, pour la lutte contre des insectes appartenant à l'ordre des lépidoptères ou des coléoptères.

**REVENDICATIONS Pour l'Etat Contractant ES**

1. Utilisation d'un composé à action insecticide, qui est un composé de formule

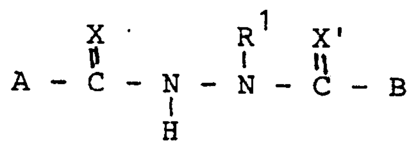

(I)

dans laquelle

X et X' sont identiques ou différents, et représentent O, S ou NR ;

$R^1$ est un radical alkyle en $C_3$-$C_{10}$ ramifié non substitué ou un radical alkyle en $C_1$-$C_4$ à chaîne droite substitué par un ou deux groupes cycloalkyle en $C_3$-$C_6$,identiques ou différents ;

A est un radical naphtyle non substitué ou substitué comportant de un à trois substituants, identiques ou différents, choisis parmi des atomes d'halogène et des groupes cyano, nitro, hydroxy, alcoxy en $C_1$-$C_4$, alkyle en $C_1$-$C_4$, carboxy, alcoxy($C_1$-$C_4$)-carbonyle, alcanoyloxy en $C_1$-$C_4$ ou $NH_2$, NHZ ou –NZZ' ;

un radical phényle non substitué ou substitué comportant de un à cinq substituants, identiques ou différents, choisis parmi des atomes d'halogène et des groupes nitro ; cyano ; hydroxy ; alkyle en $C_1$-$C_6$ ; halogénoalkyle en $C_1$-$C_6$ ; cyanoalkyle en $C_1$-$C_6$ ; alcoxy en $C_1$-$C_6$ ; halogénoalcoxy en $C_1$-$C_6$ ; alcoxy($C_1$-$C_6$)-alkyle($C_1$-$C_6$) ayant indépendamment le nombre indiqué d'atomes de carbone dans chaque groupe alkyle ; alcoxy($C_1$-$C_6$)-alcoxy($C_1$-$C_6$) ayant indépendamment le nombre indiqué d'atomes de carbone dans chaque groupe alkyle ; $-OCO_2R$ ; alcényle en $C_2$-$C_6$ éventuellement substitué par un atome d'halogène ou par un groupe cyano, alkyle en $C_1$-$C_4$, alcoxy en $C_1$-$C_4$, halogénoalcoxy en $C_1$-$C_4$ ou alkylthio en $C_1$-$C_4$ ; carboxy ; $-RCO_2R'$ ; –COR ; halogénoalkyl-($C_1$-$C_6$)-carbonyle ; cyanoalkyl($C_1$-$C_6$)-carbonyle ; nitroalkyl($C_1$-$C_6$)-carbonyle ; alcoxy($C_1$-$C_6$)-carbonyle ; halogénoalkoxy ($C_1$-$C_6$)-carbonyle ; –OCOR ; –NRR' ; amino substitué par des groupes hydroxy, alcoxy en $C_1$-$C_4$ ou alkylthio en $C_1$-$C_4$ ; phénylamino ; diphénylamino ; –CONRR' ; –OCONRR' ; –NRCOR' ; $-NRCO_2R'$ ; –N(COR)COR' ; –OCONRCOR' ; sulfhydryle ; halogénothio ; alkylthio en $C_1$-$C_6$ ; halogénoalkyl($C_1$-$C_6$)-thio ; –SOR ; $-SO_2R$ ; phénylsulfonyle ; $-OSO_2R$ ; halogénoalkyl-($C_1$-$C_6$)-sulfonyloxy ; $-SO_2NRR'$ ; –NRSOR' ; $-NRSO_2R'$ ; –CSR ; $-CS_2R$ ; –NRCSR' ; –SCOR ; phényle non substitué ou substitué comportant de un à trois substituants, identiques ou différents, choisis parmi des atomes d'halogène et des groupes cyano, nitro, hydroxyalkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; un groupe phénoxy dans lequel le noyau phényle est non substitué ou substitué par un à trois substituants, identiques ou différents, choisis parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; un groupe phénylthio dans lequel le noyau phényle est non substitué ou substitué par un à trois substituants, identiques ou différents choisis parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; ou, lorsque deux positions adjacentes sur le noyau phényle sont substituées par des groupes alcoxy, ces groupes peuvent être liés pour former, conjointement avec les atomes de carbone sur lesquels ils sont fixés, un noyau hétérocyclique dioxolanno ou dioxanno à 5 ou 6 éléments ;

B est un radical alkyle en $C_1$-$C_{10}$ non substitué ou substitué comportant, en tant que substituant(s) éventuel(s) de un à quatre substituants, identiques ou différents, choisis parmi des atomes d'halogène et des groupes cyano, nitro, hydroxy, alcoxy en $C_1$-$C_4$, halogénoalcoxy en ($C_1$-$C_4$), carboxy, alcoxy($C_1$-$C_4$)-carbonyle, alcanoyloxy en $C_1$-$C_4$ ; phényle, $NH_2$, NHZ ou –NZZ' ;

un radical cycloalkyle en $C_3$-$C_8$ non substitué ou substitué ou cycloalkyl($C_3$-$C_8$)-alkyle($C_1$-$C_4$) non substitué ou substitué, comportant, en tant que substituant(s) éventuel(s), de un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcanoyle en $C_1$-$C_4$, alcoxy($C_1$-$C_4$)-carbonyle, alcanoyloxy en $C_1$-$C_4$, phényle, $NH_2$, NHZ ou –NZZ' ;

un radical alcényle en $C_2$-$C_8$ non substitué ou substitué ou alcadiényle en $C_3$-$C_8$ non substitué ou substitué, comportant en tant que substituant(s) éventuel(s), un groupe furyle, thiényle ou pyridyle ou de un à quatre substituants, identiques ou différents,choisis parmi des atomes d'halogène et des groupes cyano, nitro, hydroxy, alkyle en $C_1$-$C_4$, cycloalkyle en $C_3$-$C_6$, halogénoalkyle en $C_1$-$C_4$, alcoxy en $C_1$-$C_4$, halogénoalcoxy en $C_1$-$C_4$, carboxy, alcoxy-($C_1$-$C_4$)-carbonyle, alcanoyloxy en $C_1$-$C_4$, $NH_2$, NHZ ou –NZZ' ;

un radical cycloalcényle en $C_3$-$C_8$ non substitué ou substitué ou cycloalcadiényle en $C_6$-$C_8$ substitué ou non substitué, comportant, en tant que substituant(s) éventuel(s), de un à quatre substituants, identiques ou

différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, $NH_2$, NHZ ou –NZZ' ; un radical alcynyle en $C_2$-$C_8$ non substitué ou substitué comportant, en tant que substituant(s) éventuel(s), de un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, phényle, $NH_2$, NHZ ou –NZZ' ;

un radical phénalkyle ayant de 1 à 4 atomes dans le fragment alkyle et dans lequel le fragment alkyle est non substitué ou substitué par un à trois substituants, identiques ou différents, choisis parmi des atomes d'halogène et des groupes cyano, hydroxy, alkyle en $C_1$-$C_4$, alcoxy en $C_1$-$C_4$, alcoxy($C_1$-$C_4$)-carbonyle, $NH_2$, NHZ ou –NZZ', et le noyau phényle est non substitué ou substitué par un à trois substituants, identiques ou différents, choisis parmi des atomes d'halogène et des groupes cyano, nitro, hydroxy, alkyle en $C_1$-$C_4$, halogénoalkyle en $C_1$-$C_4$, cyanoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$, alcényle en $C_2$-$C_6$, halogénoalcényle en $C_2$-$C_6$, alcynyle en $C_2$-$C_6$, $NH_2$, NHZ ou –NZZ'; ou

un radical phénalcényle ayant de 2 à 6 atomes de carbone dans le fragment alcényle et dont le fragment alcényle est non substitué ou substitué par un à trois substituants, identiques ou différents, choisis parmi des atomes d'halogène et des groupes cyano, hydroxy, alkyle en $C_1$-$C_4$, halogénoalkyle en $C_1$-$C_4$, alcoxy en $C_1$-$C_4$, halogénoalcoxy en $C_1$-$C_4$, alcoxy($C_1$-$C_4$)-carbonyle, $NH_2$, NHZ ou –NZZ', et le noyau phényle est non substitué ou substitué par un à trois substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcoxy($C_1$-$C_4$)-carbonyle, alcanoyloxy en $C_1$-$C_4$ ou $NH_2$, NHZ ou –NZZ' ;

R et R' étant des atomes d'hydrogène ou des groupes alkyle en $C_1$-$C_6$ ; Z et Z' étant des groupes alkyle en $C_1$-$C_4$ ; "amino" signifiant NRR' ;

ou d'un sel agronomiquement acceptable de celui-ci ; à l'exception d'un composé dans lequel X et X' sont O, $R^1$ est le groupe tert-butyle, A est un radical phényle non substitué et B est un radical méthyle non substitué ou éthyle non substitué, conjointement avec un diluant ou support agronomiquement acceptable, pour l'obtention d'une composition insecticide.

2. Utilisation selon la revendication 1 du composé dans lequel

X et X' sont O ou S ; et/ou

$R^1$ est un radical alkyle en $C_3$-$C_8$ ramifié non substitué ou un radical alkyle en $C_1$-$C_4$ à chaîne droite substitué par un ou deux groupes cycloalkyle en $C_3$-$C_4$ identiques ou différents ; et/ou

A est un radical naphtyle non substitué ; ou un radical phényle non substitué ou substitué comportant de un à trois substituants, identiques ou différents, choisis parmi des atomes d'halogène et des groupes nitro ; cyano ; alkyle en $C_1$-$C_4$ ; halogénoalkyle en $C_1$-$C_4$ ; cyanoalkyle en $C_1$-$C_4$ ; alcoxy en $C_1$-$C_4$ ; alcoxy($C_1$-$C_4$)alkyle($C_1$-$C_4$) ; –COD ; carboxy ; alcoxy($C_1$-$C_4$)-carbonyle ; alcanoyloxy en $C_1$-$C_4$ ; $NH_2$, NHZ ou –NZZ' ; alkylthio en $C_1$-$C_4$ ; –CSD ; –$CS_2$D ; –SCOD ; un groupe phényle substitué ou non substitué comportant un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; ou lorsque deux positions adjacentes sur le noyau phényle sont substituées par des groupes alcoxy, ces groupes sont liés pour former, conjointement avec les atomes de carbone auxquels ils sont fixés, un noyau hétérocyclique dioxolanno ou dioxanno à 5 ou 6 éléments ; et/ou

B est un radical alkyle en $C_1$-$C_8$ non substitué ou substitué comportant, en tant que substituant(s) éventuel(s), de un à trois substituants, identiques ou différents, choisis parmi des atomes d'halogène et des groupes cyano, alcoxy en $C_1$-$C_4$, phényle, alcoxy($C_1$-$C_4$)-carbonyle et halogénoalcoxy en $C_1$-$C_4$ ;

un radical cycloalkyle en $C_3$-$C_6$ non substitué ou substitué ou cycloalkyl($C_3$-$C_5$)-alkyle($C_1$-$C_4$) non substitué ou substitué, comportant, en tant que substituant(s) éventuel(s), un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$, alcanoyle en $C_1$-$C_4$ et halogénoalcoxy en $C_1$-$C_4$ ;

un radical alcényle en $C_2$-$C_6$ non substitué ou substitué comportant,en tant que substituant(s) éventuel(s), un groupe furyle ou de un à trois substituants, identiques ou différents, choisis parmi des groupes alkyle en $C_1$-$C_4$, halogénoalkyle en $C_1$-$C_4$, alcoxy en $C_1$-$C_4$ et halogénoalcoxy en $C_1$-$C_4$ ;

un radical cycloalcényle en $C_3$-$C_6$ non substitué ou substitué comportant, en tant que substituant(s) éventuels(s), un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ;

un radical alcynyle non substitué ou substitué par le groupe phényle ;

un radical phénalkyle ayant 1 ou 2 atomes de carbone dans le fragment alkyle et dont le fragment alkyle est non substitué ou substitué par un ou deux substituants identiques ou différents, choisis parmi des atomes

d'halogène et des groupes alkyle en $C_1$-$C_4$, halogénoalkyle en $C_1$-$C_4$, alcoxy en $C_1$-$C_4$ ou halogénoalcoxy en $C_1$-$C_4$, et le noyau phényle est non substitué ou substitué par un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ; ou

un radical phénalcényle ayant 2 ou 3 atomes de carbone dans le fragment alcényle et dont le fragment alcényle est non substitué ou substitué par des substituants choisis parmi des atomes d'halogène et des groupes alkyle en $C_1$-$C_4$ ou halogénoalkyle en $C_1$-$C_4$, alcoxy en $C_1$-$C_4$, ou halogénoalkyle en $C_1$-$C_4$, et le noyau phényle est non substitué ou substitué par un ou deux substituants, identiques ou différents,choisis parmi des atomes d'halogène et des groupes alkyle en $C_1$-$C_4$, halogénoalkyle en $C_1$-$C_4$, alcoxy en $C_1$-$C_4$ et halogénoalcoxy en $C_1$-$C_4$ ;

ou d'un sel agronomiquement acceptable de celui-ci ; D étant un atome d'hydrogène ou un groupe alkyle en $C_1$-$C_4$, et Z et Z' étant des groupes alkyle en $C_1$-$C_4$.

3. Utilisation selon la revendication 2 d'un composé dans lequel

X et X' sont O ou S ; et/ou

$R^1$ est un radical alkyle en $C_3$-$C_8$ ramifié ; et/ou

A est un radical naphtyle non substitué ; ou

un radical phényle non substitué ou substitué comportant de un à trois substituants, identiques ou différents, choisis parmi des atomes d'halogène et des groupes nitro, cyano, alkyle en $C_1$-$C_4$, halogénoalkyle en $C_1$-$C_4$, cyanoalkyle en $C_1$-$C_4$, alcoxy en $C_1$-$C_4$, –COD, alcoxy($C_1$-$C_4$)-carbonyle, alcanoyloxy en $C_1$-$C_4$ et un groupe phényle non substitué ou substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, $NH_2$, NHZ ou –NZZ' ; et/ou

B est un radical alkyle en $C_1$-$C_6$ non substitué ou substitué comportant de un à trois substituants, identiques ou différents, choisis parmi des atomes d'halogène et des groupes cyano, alcoxy en $C_1$-$C_4$, phényle, alcoxy($C_1$-$C_3$)-carbonyle et halogénoalcoxy en $C_1$-$C_4$ ;

un radical cycloalkyle en $C_3$-$C_6$ non substitué ou substitué ou cycloalkyl($C_3$-$C_6$)-alkyle($C_1$-$C_4$) non substitué ou substitué, dans lesquels le substituant éventuel est un atome d'halogène ou un groupe alkyle en $C_1$-$C_4$, alcanoyle en $C_1$-$C_4$, alcoxy en $C_1$-$C_4$ ou halogénoalcoxy en $C_1$-$C_4$ ;

un radical alcényle non substitué ou substitué comportant un ou deux substituants, identiques ou différents, alkyle en $C_1$-$C_4$, alcoxy en $C_1$-$C_4$ ou halogénoalcoxy en $C_1$-$C_4$ ;

un radical cycloalcényle en $C_4$-$C_8$ non substitué ou substitué dans lequel le substituant est un atome d'halogène ou un groupe alkyle en $C_1$-$C_4$, alcoxy en $C_1$-$C_4$ ou halogénoalcoxy en $C_1$-$C_4$ ; ou

un radical alcynyle non substitué ou substitué par le groupe phényle ;

ou d'un sel agronomiquement acceptable de celui-ci.

4. Utilisation selon la revendication 3 d'un composé dans lequel

X et X' sont O ; et/ou

$R^1$ est un radical alkyle en $C_4$-$C_7$ ramifié ; et/ou

A est un radical phényle non substitué, monosubstitué, 2,3-disubstitué ou 2,4-disubstitué, dans lequel les substituants peuvent être des substituants, identiques ou différents, choisis parmi des atomes d'halogène et des groupes alkyle en $C_1$-$C_4$, alcoxy en $C_1$-$C_4$ et halogénoalkyle en $C_1$-$C_4$ ; et/ou

B est un radical alkyle en $C_1$-$C_6$ non substitué ou substitué comportant de un à trois substituants, identiques ou différents, halogéno, phényle ou cyano ;

un radical cycloalkyle en $C_4$-$C_6$ non substitué ;

un radical alcényle en $C_2$-$C_5$ non substitué ou substitué comportant, en tant que substituant(s) éventuel(s), un ou deux groupes alkyle en $C_1$-$C_4$ identiques ou différents ;

un radical cycloalcényle en $C_4$-$C_6$ non substitué ; ou

un radical phénalkyle ayant 1 ou 2 atomes de carbone dans le fragment alkyle et dont le noyau phényle est non substitué ou substitué par un ou deux substituants identiques ou différents,choisis parmi des atomes d'halogène et des groupes alkyle en $C_1$-$C_4$ et alcoxy en $C_1$-$C_4$ ;

ou d'un sel agronomiquement acceptable de celui-ci.

5. Utilisation selon la revendication 4 d'un composé dans lequel

X et X' sont O ; et/ou

$R^1$ est le groupe tert-butyle, néopentyle (2,2-diméthylpropyle) ou 1,2,2-triméthylpropyle ; et/ou

A est un radical phényle non substitué ou monosubstitué dans lequel le substituant peut être chloro, fluoro, bromo, iodo, méthyle, éthyle, méthoxy ou trifluorométhyle ; et/ou

B est un radical alkyle en $C_1$-$C_4$ non substitué ou substitué comportant, en tant que substituant(s) éventuel(s), un à trois des substituants, identiques ou différents, phényle, chloro, fluoro ou bromo ;

un radical cycloalkyle en $C_4$-$C_6$ non substitué ;

un radical cycloalcényle en $C_4$-$C_6$ non substitué ; ou un radical phénalkyle ayant 1 ou 2 atomes de carbone dans le fragment alkyle et dont le noyau phényle est non substitué ou substitué par un ou deux des substituants, identiques ou différents, chloro, fluoro, bromo, iodo, méthyle, éthyle, méthoxy ou trifluorométhyle ;
ou d'un sel agronomiquement acceptable de celui-ci.

6. Utilisation selon la revendication 3 du composé dans lequel
X et X' sont O ;
$R^1$ est le groupe tert-butyle ; et
A est le groupe phényle et B est le groupe cyclohexyle, benzyle, 1-cyclohexényle, 2-méthylcyclohexadi-2,5-ényle, phényléthynyle, 1-méthylpropyle, 2-méthyl-1-phénylbutyle, 3-acétyl-2,2-diméthylcyclobutylméthyle, 1-buténylé, 3-buténylé, 3-carbométhoxypropyle ou méthoxyméthyle ; ou
A est le groupe 3,5-dichlorophényle et B est le groupe 2-bromoéthyle ; ou
A est le groupe 4-chlorophényle et B est le groupe cyclobutyle ; ou
A est le groupe 2,3-diméthylphényle et B est le groupe 1-méthyléthényle ou 1-éthyléthényle ; ou
A est le groupe 4-éthylphényle et B est le groupe 1-méthyléthényle.

7. Utilisation selon l'une quelconque des revendications précédentes du composé ou sel en une quantité de 0,0001, de préférence 0,001, à 99%, en poids.

8. Utilisation selon la revendication 6 du composé ou sel en une quantité de 0,01 à 99% en poids.

9. Utilisation selon l'une quelconque des revendications 6 à 8 du composé ou sel pour la préparation d'une composition sous la forme d'une poudre mouillable, d'une composition fluide, d'une poudre, d'un granulé, d'un appât ou d'un concentré émulsifiable.

10. Utilisation d'un composé ou sel tel que défini dans l'une quelconque des revendications 1 à 6, éventuellement dans une composition contenant également un diluant ou support agronomiquement acceptable, pour la lutte contre des insectes, par mise en contact desdits insectes avec une quantité à action insecticide dudit composé ou sel.

11. Procédé mécanique pour l'amélioration de la valeur commerciale et/ou de la rentabilité de récoltes vendables de plantes dont la croissance est affectée ou susceptible d'être affectée par des insectes, comprenant (1) la charge, dans un récipient, dispositif de fumigation ou dispositif de dissémination mécanique, d'un composé ou sel insecticide tel que défini dans l'une quelconque des revendications 1 à 6, éventuellement en mélange avec un diluant ou support agronomiquement acceptable, (2) l'utilisation du récipient, fumigateur ou dispositif de dissémination mécanique pour l'application du composé ou sel insecticide, sous la forme de granules, poudre, fumée, vapeur ou préparation liquide contenant un agent tensioactif, sur des plantes en croissance ou sur un milieu de croissance sur lequel les plantes sont en croissance ou sont destinées à croître, ou sur les insectes eux-mêmes, (3) l'ajustement de la dose du composant actif au cours de cette étape d'application de sorte que le taux d'application du composé insecticide actif soit suffisant pour combattre les insectes, mais insuffisant pour avoir un effet nuisible sur les plantes cultivées en croissance ou destinées à croître dans la zone traitée.

12. Utilisation ou procédé selon la revendication 10 ou 11, comprenant l'application du composé ou sel à un taux d'environ 10 g à environ 10 kg par hectare, sur des plantes en croissance ou sur une zone où des plantes sont destinées à croître.

13. Utilisation ou procédé selon la revendication 12, dans lesquels le taux d'application va de 100 g à 5 kg du composé par hectare.

14. Utilisation ou procédé selon la revendication 10 ou 11, pour la lutte contre des insectes appartenant à l'ordre des lépidoptères ou des coléoptères.